# EUROPEAN PATENT APPLICATION

(11) **EP 3 992 211 A1**
(43) Date of publication of application: **04.05.2022**
(21) Application number: 20831939.2
(22) Date of filing: 24.06.2020
(51) Int. Cl.: C07K 16/30, C07K 19/00, C12N 15/13, C12N 15/63, A61K 39/395, A61K 47/68, A61P 35/00, G01N 33/50

(54) **ANTI-CEA ANTIBODY AND APPLICATION THEREOF**

(30) Priority: 26.06.2019 CN 201910560329
(71) Applicant: Jiangsu Hengrui Medicine Co., Ltd., Lianyungang, Jiangsu 222047 (CN); Shanghai Hengrui Pharmaceutical Co., Ltd., Shanghai 200245 (CN)
(72) Inventor: YING, Hua, Shanghai 200245 (CN); MAO, Langyong, Shanghai 200245 (CN); ZHANG, Ling, Shanghai 200245 (CN); GE, Hu, Shanghai 200245 (CN); TAO, Weikang, Shanghai 200245 (CN)
(74) Representative: Potter Clarkson
(86) International application number: PCT/CN2020/097984
(87) International publication number: WO 2020/259550

(57) **Abstract**

Provided is an anti-CEA antibody comprising a heavy chain variable region and a light chain variable region, a drug conjugate thereof, and a composition containing the anti-CEA antibody or the drug conjugate thereof, and an application thereof as a drug.

## Description

The present disclosure claims the priority of Chinese patent application "Anti-CEA antibody and application thereof" (Application No. 201910560329.6; Application date of June 26, 2019).

### FIELD OF THE INVENTION

The present disclosure relates to the field of antibody drugs. Specifically, it relates to anti-CEA antibody drugs and applications thereof.

### BACKGROUND OF THE INVENTION

The descriptions herein only provide background information about the present disclosure, and do not necessarily constitute prior art.

Carcinoembryonic antigen (CEA, also known as CEACAM-5 or CD66e), one of the earliest found tumor-associated antigens, is a glycoprotein with a molecular weight of about 180kDa. CEA is a member of the immunoglobulin superfamily and contains 7 domains that are connected to the cell membrane via Glycosyl Phosphatidyl Inositol (GPI) anchor (Thompson JA, J Clin Lab Anal. 5:344-366, 1991). CEA was first discovered and reported in colon cancer tissue extracts by Gold P and Freedman SO (Gold and Freedman 1965; Gold and Freedman 1965), and Thomson, Krupey et al. 1969, subsequently reported that CEA was detected in the serum from colon cancer patients and other tumor patients using sensitive radio-immunoassay method, while the level of CEA in the serum from healthy people or patients with other diseases was extremely low. The expression of CEA is increased in cancer cells, and the increased CEA promotes cell adhesion, which in turn promotes cell metastasis (Marshall J., Semin Oncol., 30 (Supplement 8): 30-6, 2003). CEA is commonly expressed in epithelial tissues, including cells in the gastrointestinal, respiratory and genitourinary tracts, and cells in the colon, cervix, sweat glands and prostate (Nap et al., Tumour Biol., 9(2-3): 145-53, 1988; Nap et al., Cancer Res., 52(8): 2329-2339, 1992).

At present, a variety of anti-CEA antibodies have been disclosed in WO1999043817A1, WO2004032962A1, WO2005086875A3, WO2012117002A1 and other patents.

### SUMMARY OF THE INVENTION

The present disclosure provides a novel anti-CEA antibody. The antibodies of the present disclosure include full-length antibodies and antigen-binding fragments thereof.

In some embodiments, the anti-CEA antibody of the present disclosure comprises an antibody heavy chain variable region and light chain variable region, wherein:
i) a heavy chain variable region, wherein the HCDR1 and HCDR3 thereof are the same as the HCDR1 and HCDR3 of the heavy chain variable region as shown in SEQ ID NO: 7; and the HCDR2 thereof is the same as the HCDR2 of the heavy chain variable region as shown in SEQ ID NO: 7, or has one amino acid difference thereto;
   a light chain variable region, wherein the LCDR1, LCDR2 and LCDR3 thereof are the same as the LCDR1, LCDR2 and LCDR3 of the light chain variable region as shown in SEQ ID NO: 8; or
ii) a heavy chain variable region, wherein the HCDR1 and HCDR3 thereof are the same as the HCDR1 and HCDR3 of the heavy chain variable region as shown in SEQ ID NO: 9; and the HCDR2 thereof is the same as the HCDR2 of the heavy chain variable region as shown in SEQ ID NO: 9, or has one amino acid difference thereto;
   a light chain variable region, wherein the LCDR1, LCDR2 and LCDR3 thereof are the same as the LCDR1, LCDR2 and LCDR3 of the light chain variable region as shown in SEQ ID NO: 10; or
iii) a heavy chain variable region, wherein the HCDR1, HCDR2 and HCDR3 thereof are the same as the HCDR1, HCDR2 and HCDR3 of the heavy chain variable region as shown in SEQ ID NO: 11;
   a light chain variable region, wherein the LCDR1, LCDR2 and LCDR3 thereof are the same as the LCDR1, LCDR2 and LCDR3 of the light chain variable region as shown in SEQ ID NO: 12; or
iv) a heavy chain variable region, wherein the HCDR1 and HCDR3 thereof are the same as the HCDR1 and HCDR3 of the heavy chain variable region as shown in SEQ ID NO: 13; and the HCDR2 thereof is the same as the HCDR2 of the heavy chain variable region as shown in SEQ ID NO: 13, or has one amino acid difference thereto;
   a light chain variable region, wherein the LCDR1 and LCDR3 thereof are the same as the LCDR1 and LCDR3 of the light chain variable region as shown in SEQ ID NO: 14; and the LCDR2 thereof is the same as the LCDR2 of the light chain variable region as shown in SEQ ID NO: 14, or has one amino acid difference thereto.

In some embodiments, the anti-CEA antibody according to any one as described above comprises a heavy chain variable region and a light chain variable region, wherein:
v) the heavy chain variable region comprises HCDR1, HCDR2 and HCDR3 as shown in SEQ ID NO: 15, SEQ ID NO: 16 and SEQ ID NO: 17, respectively,
the light chain variable region comprises LCDR1, LCDR2 and LCDR3 as shown in SEQ ID NO: 18, SEQ ID NO: 19 and SEQ ID NO: 20, respectively; or
the heavy chain variable region comprises HCDR1, HCDR2 and HCDR3 as shown in SEQ ID NO: 15, SEQ ID NO: 38 and SEQ ID NO: 17, respectively,
the light chain variable region comprises LCDR1, LCDR2 and LCDR3 as shown in SEQ ID NO: 18, SEQ ID NO: 19 and SEQ ID NO: 20, respectively;
vi) the heavy chain variable region comprises HCDR1, HCDR2 and HCDR3 as shown in SEQ ID NO: 21, SEQ ID NO: 22 and SEQ ID NO: 23, respectively,
the light chain variable region comprises LCDR1, LCDR2 and LCDR3 as shown in SEQ ID NO: 24, SEQ ID NO: 25 and SEQ ID NO: 26, respectively; or
the heavy chain variable region comprises HCDR1, HCDR2 and HCDR3 as shown in SEQ ID NO: 21, SEQ ID NO: 47 and SEQ ID NO: 23, respectively,
the light chain variable region comprises LCDR1, LCDR2 and LCDR3 as shown in SEQ ID NO: 24, SEQ ID NO: 25 and SEQ ID NO: 26, respectively; or
vii) the heavy chain variable region comprises HCDR1, HCDR2 and HCDR3 as shown in SEQ ID NO: 27, SEQ ID NO: 28 and SEQ ID NO: 29, respectively,
the light chain variable region comprises LCDR1, LCDR2 and LCDR3 as shown in SEQ ID NO: 30, SEQ ID NO: 31 and SEQ ID NO: 32, respectively; or
viii) the heavy chain variable region comprises HCDR1 and HCDR3 as shown in SEQ ID NO: 33 and SEQ ID NO: 34 respectively, and HCDR2 as shown in SEQ ID NO: 16 or SEQ ID NO: 38;
the light chain variable region comprises LCDR1 and LCDR3 as shown in SEQ ID NO: 35 and SEQ ID NO: 37 respectively, and LCDR2 as shown in SEQ ID NO: 36 or SEQ ID NO: 64.

In some embodiments, the heavy chain variable region comprises HCDR1, HCDR2 and HCDR3 as shown in SEQ ID NO: 33, SEQ ID NO: 16 and SEQ ID NO: 34, respectively,
the light chain variable region comprises LCDR1, LCDR2 and LCDR3 as shown in SEQ ID NO: 35, SEQ ID NO: 36 and SEQ ID NO: 37, respectively; or
the heavy chain variable region comprises HCDR1, HCDR2 and HCDR3 as shown in SEQ ID NO: 33, SEQ ID NO: 38 and SEQ ID NO: 34, respectively,
the light chain variable region comprises LCDR1, LCDR2 and LCDR3 as shown in SEQ ID NO: 35, SEQ ID NO: 36 and SEQ ID NO: 37, respectively; or
the heavy chain variable region comprises HCDR1, HCDR2 and HCDR3 as shown in SEQ ID NO: 33, SEQ ID NO: 16 and SEQ ID NO: 34, respectively,
the light chain variable region comprises LCDR1, LCDR2 and LCDR3 as shown in SEQ ID NO: 35, SEQ ID NO: 64 and SEQ ID NO: 37, respectively; or
the heavy chain variable region comprises HCDR1, HCDR2 and HCDR3 as shown in SEQ ID NO: 33, SEQ ID NO: 38 and SEQ ID NO: 34, respectively,
the light chain variable region comprises LCDR1, LCDR2 and LCDR3 as shown in SEQ ID NO: 35, SEQ ID NO: 64 and SEQ ID NO: 37, respectively.

In some embodiments of the anti-CEA antibody according to any one as described above, wherein the antibody is a murine antibody, a chimeric antibody, or a humanized antibody.

In some embodiments of the anti-CEA antibody according to any one as described above, wherein the antibody is a murine antibody or a chimeric antibody, which comprises a heavy chain variable region and a light chain variable region, wherein:
(a) the amino acid sequence of the heavy chain variable region is as shown in SEQ ID NO: 7 or has at least 90% sequence identity to SEQ ID NO: 7; and/or
   the amino acid sequence of the light chain variable region is as shown in SEQ ID NO:8 or has at least 90% sequence identity to SEQ ID NO: 8; or
(b) the amino acid sequence of the heavy chain variable region is as shown in SEQ ID NO: 9 or has at least 90% sequence identity to SEQ ID NO: 9; and/or
   the amino acid sequence of the light chain variable region is as shown in SEQ ID NO:10 or has at least 90% sequence identity to SEQ ID NO: 10;
(c) the amino acid sequence of the heavy chain variable region is as shown in SEQ ID NO: 11 or has at least 90% sequence identity to SEQ ID NO: 11; and/or
   the amino acid sequence of the light chain variable region is as shown in SEQ ID NO:12 or has at least 90% sequence identity to SEQ ID NO: 12; or
(d) the amino acid sequence of the heavy chain variable region is as shown in SEQ ID NO: 13 or has at least 90% sequence identity to SEQ ID NO: 13; and/or
   the amino acid sequence of the light chain variable region is as shown in SEQ ID NO:14 or has at least 90% sequence identity to SEQ ID NO: 14.

In some embodiments, "has at least 90% sequence identity" as described in any one of the above (a) to (d) refers to having at least 90%, 91%, 92%, 93%, 94%, 95%, 96%, 97%, 98%, 99% or 100% sequence identity.

In some embodiments of the anti-CEA antibody according to any one as described above, wherein the antibody is a humanized antibody, which comprises a heavy chain variable region and a light chain variable region, wherein:
(e) the amino acid sequence of the heavy chain variable region is as shown in SEQ ID NO: 39, 40, 41 or 42, or has at least 90% sequence identity to any amino acid sequence as shown in SEQ ID NO: 39, 40, 41 or 42; and/or
   the amino acid sequence of the light chain variable region is as shown in SEQ ID NO:43, 44, 45 or 46, or has at least 90% sequence identity to any amino acid sequence as shown in SEQ ID NO: 43, 44, 45 or 46;
(f) the amino acid sequence of the heavy chain variable region is as shown in SEQ ID NO: 48, 49, 50, 51 or 52, or has at least 90% sequence identity to any amino acid sequence as shown in SEQ ID NO: 48, 49, 50, 51 or 52; and/or
   the amino acid sequence of the light chain variable region is as shown in SEQ ID NO:53, 54 or 55, or has at least 90% sequence identity to any amino acid sequence as shown in SEQ ID NO: 53, 54 or 55;
(g) the amino acid sequence of the heavy chain variable region is as shown in SEQ ID NO: 56, 57 or 58, or has at least 90% sequence identity to any amino acid sequence as shown in SEQ ID NO: 56, 57 or 58; and/or
   the amino acid sequence of the light chain variable region is as shown in SEQ ID NO:59, 60, 61, 62 or 63, or has at least 90% sequence identity to any amino acid sequence as shown in SEQ ID NO: 59, 60, 61, 62 or 63; or
(h) the amino acid sequence of the heavy chain variable region is as shown in SEQ ID NO: 65, 66, 67 or 68, or has at least 90% sequence identity to any amino acid sequence as shown in SEQ ID NO: 65, 66, 67 or 68; and/or
   the amino acid sequence of the light chain variable region is as shown in SEQ ID NO:69, 70, 71, 72, 73, 74, 75 or 76, or has at least 90% sequence identity to any amino acid sequence as shown in SEQ ID NO: 69, 70, 71, 72, 73, 74, 75 or 76.

In some embodiments, "has at least 90% sequence identity" as described in any one of the above (e) to (h) refers to having at least 90%, 91%, 92%, 93%, 94%, 95%, 96%, 97%, 98%, 99% or 100% sequence identity.

In some embodiments, the anti-CEA antibody as described above comprises the combination of a heavy chain variable region and a light chain variable as shown in the following Table a-1, Table a-2, Table a-3 or Table a-4:

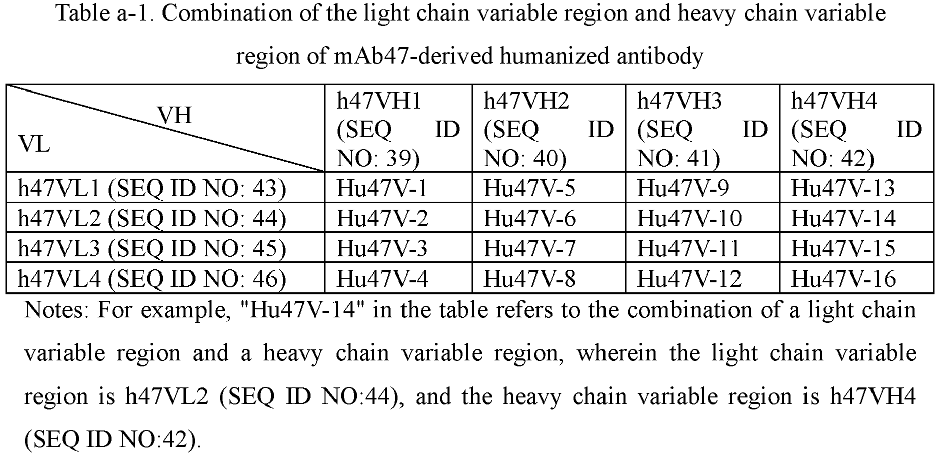

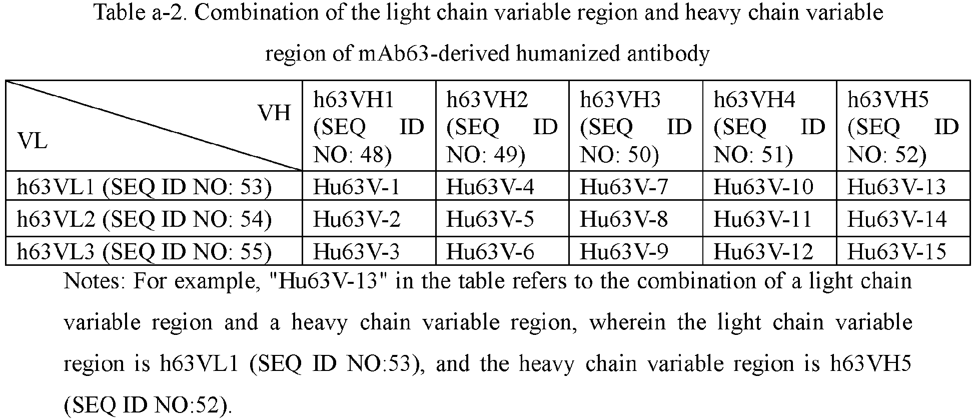

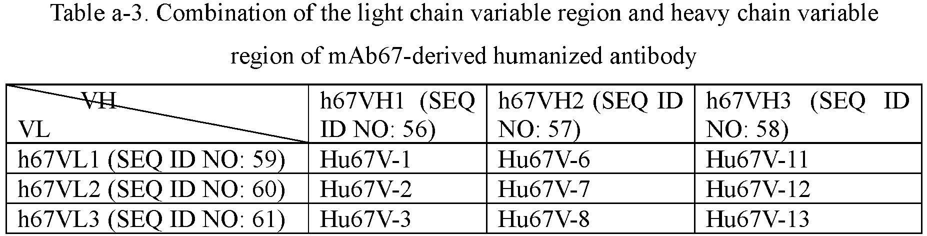

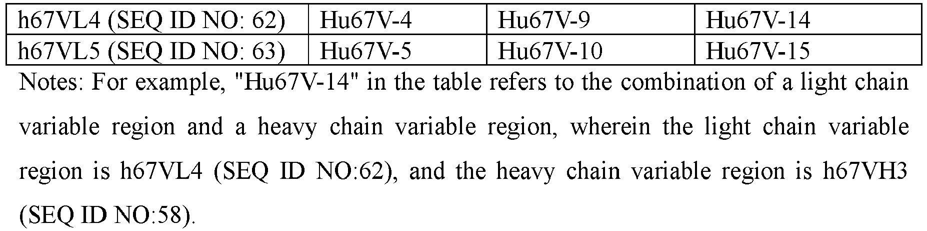

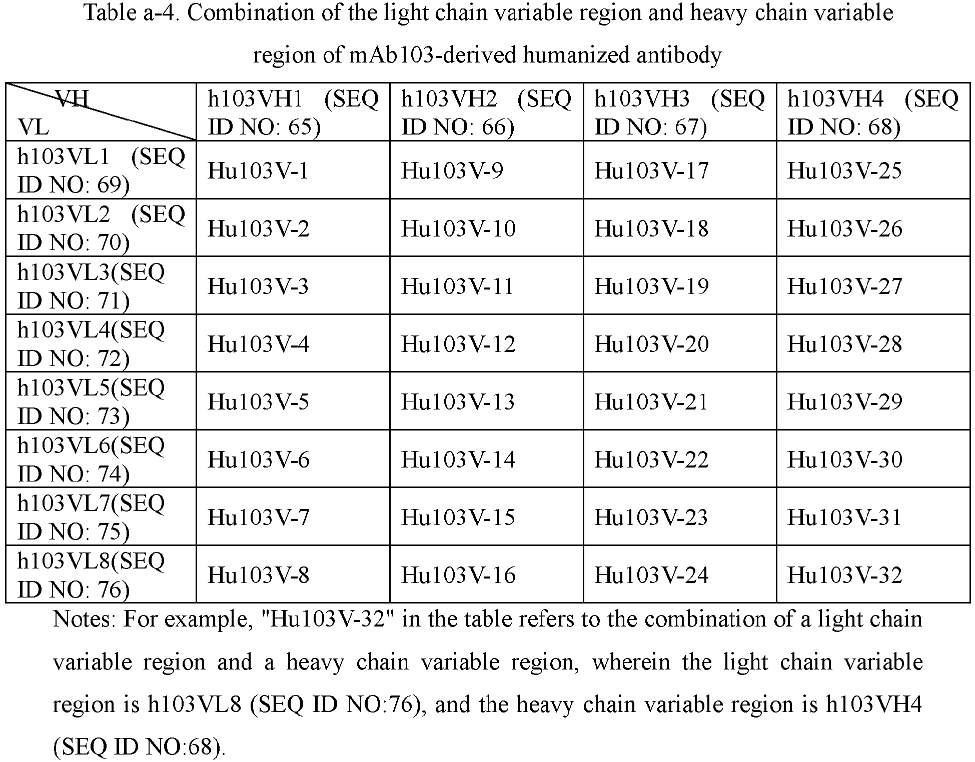

In some embodiments, the humanized anti-CEA antibody as described above comprises a heavy chain variable region and a light chain variable region, wherein the amino acid sequence of the light chain variable region is as shown in SEQ ID NO:44, and the amino acid sequence of the heavy chain variable region is as shown in SEQ ID NO: 42; or
the amino acid sequence of the light chain variable region is as shown in SEQ ID NO:53, and the amino acid sequence of the heavy chain variable region is as shown in SEQ ID NO: 52; or
the amino acid sequence of the light chain variable region is as shown in SEQ ID NO:62, and the amino acid sequence of the heavy chain variable region is as shown in SEQ ID NO: 58; or
the amino acid sequence of the light chain variable region is as shown in SEQ ID NO:76, and the amino acid sequence of the heavy chain variable region is as shown in SEQ ID NO: 68.

In some embodiments of the anti-CEA antibody according to any one as described above, wherein the antibody comprises framework region(s) derived from a human antibody or framework region variant(s) thereof, and the framework region variant has 1 to 10 back mutation(s) on each of the human antibody light chain framework region and/or heavy chain framework region;
preferably, the framework region variant comprises amino acid mutation(s) selected from the following (i) or (1):
(i) when the light chain variable region comprises LCDR1, LCDR2, and LCDR3 as shown in SEQ ID NO: 18, SEQ ID NO: 19 and SEQ ID NO: 20, respectively, the framework region(s) of the light chain variable region comprise(s) one or more amino acid back mutation(s) selected from the group consisting of 46P, 47W, 49Y, 70S and 71Y, and/or
when the heavy chain variable region comprises HCDR1 as shown in SEQ ID NO:15, HCDR2 as shown in SEQ ID NO:16 or SEQ ID NO:38, and HCDR3 as shown in SEQ ID NO:17, the framework region(s) of the heavy chain variable region comprise(s) one or more amino acid back mutation(s) selected from 38K and 46K;
(j) when the light chain variable region comprises LCDR1, LCDR2, and LCDR3 as shown in SEQ ID NO: 24, SEQ ID NO: 25 and SEQ ID NO: 26, respectively, the framework region(s) of the light chain variable region comprise(s) one or more amino acid back mutation(s) selected from the group consisting of 2V, 42G, 44V and 71Y, and/or
when the heavy chain variable region comprises HCDR1 as shown in SEQ ID NO:21, HCDR2 as shown in SEQ ID NO:22 or SEQ ID NO:47, and HCDR3 as shown in SEQ ID NO:23, the framework region(s) of the heavy chain variable region comprise(s) one or more amino acid back mutation(s) selected from the group consisting of 66K, 67A, 69L, 71V, 73K, 82F, 82A R;
(k) when the light chain variable region comprises LCDR1, LCDR2, and LCDR3 as shown in SEQ ID NO: 30, SEQ ID NO: 31 and SEQ ID NO: 32, respectively, the framework region(s) of the light chain variable region comprise(s) one or more amino acid back mutation(s) selected from the group consisting of 3V, 43P and 58V, and/or
when the heavy chain variable region comprises HCDR1, HCDR2, and HCDR3 as shown in SEQ ID NO: 27, SEQ ID NO: 28 and SEQ ID NO: 29, respectively, the framework region(s) of the heavy chain variable region comprise(s) one or more amino acid back mutation(s) selected from the group consisting of 38K, 66K and 71V, and
(l) when the light chain variable region comprises LCDR1 as shown in SEQ ID NO:35, LCDR2 as shown in SEQ ID NO:36 or SEQ ID NO:64, and LCDR3 as shown in SEQ ID NO:37, the framework region(s) of the light chain variable region comprise(s) one or more amino acid back mutation(s) selected from the group consisting of 4V, 36Y, 43P, 47V, 49E, 70D and 871; and/or
when the heavy chain variable region comprises HCDR1 as shown in SEQ ID NO:33, HCDR2 as shown in SEQ ID NO:16 or SEQ ID NO:38, and HCDR3 as shown in SEQ ID NO:34, the framework region(s) of the heavy chain variable region comprise(s) one or more amino acid back mutation(s) selected from the group consisting of 21, 38K and 46K;
wherein, the mutation sites are numbered according to Kabat numbering criteria. For example, "46P" indicates that the amino acid corresponding to position 46 according to the Kabat numbering criteria is back-mutated to "P".

Those skilled person should understand that when numbering criteria other than Kabat is used, different numbers can be assigned to amino acid residues that are functionally and/or structurally equivalent, but these residues are still corresponding to the positions defined in the present disclosure.

In some embodiments, the anti-CEA antibody comprises:
(i-1) a light chain variable region comprising LCDR1, LCDR2 and LCDR3 as shown in SEQ ID NO: 18, SEQ ID NO: 19 and SEQ ID NO: 20, respectively; and
a heavy chain variable region comprising HCDR1 as shown in SEQ ID NO: 15, HCDR2 as shown in SEQ ID NO: 16 or SEQ ID NO: 38, and HCDR3 as shown in SEQ ID NO: 17; and
wherein the framework region(s) of the light chain variable region comprise(s) one or more amino acid back mutation(s) selected from the group consisting of 46P, 47W, 49Y, 70S and 71Y, and/or the framework region(s) of the heavy chain variable region comprise(s) one or more amino acid back mutation(s) selected from 38K and 46K;
(j-1) a light chain variable region comprising LCDR1, LCDR2 and LCDR3 as shown in SEQ ID NO: 24, SEQ ID NO: 25 and SEQ ID NO: 26, respectively; and
a heavy chain variable region comprising HCDR1 as shown in SEQ ID NO: 21, HCDR2 as shown in SEQ ID NO: 22 or SEQ ID NO: 47, and HCDR3 as shown in SEQ ID NO: 23; and
wherein the framework region(s) of the light chain variable region comprise(s) one or more amino acid back mutation(s) selected from the group consisting of 2V, 42G, 44V and 71Y, and/or the framework region(s) of the heavy chain variable region comprise(s) one or more amino acid back mutation(s) selected from the group consisting of 481, 66K, 67A, 69L, 71V, 73K, 82F and 82A R;
(k-1) a light chain variable region comprising LCDR1, LCDR2 and LCDR3 as shown in SEQ ID NO: 30, SEQ ID NO: 31 and SEQ ID NO: 32, respectively; and
a heavy chain variable region comprising HCDR1, HCDR2 and HCDR3 as shown in SEQ ID NO: 27, SEQ ID NO: 28 and SEQ ID NO: 29, respectively, and
wherein the framework region(s) of the light chain variable region comprise(s) one or more amino acid back mutation(s) selected from the group consisting of 3V, 43P and 58V, and/or the framework region(s) of the heavy chain variable region comprise(s) one or more amino acid back mutation(s) selected from the group consisting of 38K, 66K and 71V;
(l-1) a light chain variable region comprising LCDR1 as shown in SEQ ID NO:35, LCDR2 as shown in SEQ ID NO:36 or SEQ ID NO:64, and LCDR3 as shown in SEQ ID NO:37; and
a heavy chain variable region comprising HCDR1 as shown in SEQ ID NO: 33, HCDR2 as shown in SEQ ID NO: 16 or SEQ ID NO: 38, and HCDR3 as shown in SEQ ID NO: 34; and
wherein the framework region(s) of the light chain variable region comprise(s) one or more amino acid back mutation(s) selected from the group consisting of 4V, 36Y, 43P, 47V, 49E, 70D and 871, and/or the framework region(s) of the heavy chain variable region comprise(s) one or more amino acid back mutation(s) selected from the group consisting of 21, 38K and 46K;
wherein, the back mutation sites are numbered according to Kabat numbering criteria.

In some embodiments of the anti-CEA antibody according to any one of the above, wherein the antibody further comprises an antibody heavy chain constant region and a light chain constant region; preferably, the heavy chain constant region is selected from the group consisting of human IgG1, IgG2, IgG3 and IgG4 constant regions and conventional variant(s) thereof, and the light chain constant region is selected from the group consisting of human antibody κ and λ chain constant regions and conventional variant(s) thereof; more preferably, the antibody comprises a heavy chain constant region as shown in SEQ ID NO: 77 and a light chain constant region as shown in SEQ ID NO: 78 or SEQ ID NO: 79;
most preferably, the antibody comprises:
(m) a heavy chain as shown in SEQ ID NO: 80 or having at least 85% sequence identity thereto, and/or a light chain as shown in SEQ ID NO: 81 or having at least 85% sequence identity thereto; or
(n) a heavy chain as shown in SEQ ID NO: 82 or having at least 85% sequence identity thereto, and/or a light chain as shown in SEQ ID NO: 83 or having at least 85% sequence identity thereto; or
(o) a heavy chain as shown in SEQ ID NO: 84 or having at least 85% sequence identity thereto, and/or a light chain as shown in SEQ ID NO: 85 or having at least 85% sequence identity thereto; or
(p) a heavy chain as shown in SEQ ID NO: 86 or having at least 85% sequence identity thereto, and/or a light chain as shown in SEQ ID NO: 87 or having at least 85% sequence identity thereto.

In some embodiments, "having at least 85% sequence identity" as described in any one of the above (m) to (p) refers to having at least 85%, 86%, 87%, 88%, 89%, 90%, 91%, 92%, 93%, 94%, 95%, 96%, 97%, 98%, 99% or 100% sequence identity.

In some embodiments, the anti-CEA antibody according to any one as described above, wherein the antibody is a full-length antibody or an antigen-binding fragment.

In some embodiments, the anti-CEA antibody of any one as described above, wherein the anti-CEA antibody comprises:
(q) a heavy chain as shown in SEQ ID NO: 80 and a light chain as shown in SEQ ID NO: 81;
(r) a heavy chain as shown in SEQ ID NO: 82 and a light chain as shown in SEQ ID NO: 83;
(s) a heavy chain as shown in SEQ ID NO: 84 and a light chain as shown in SEQ ID NO: 85; or
(t) a heavy chain as shown in SEQ ID NO: 86 and a light chain as shown in SEQ ID NO: 87.

In some embodiments, the present disclosure also provides an isolated anti-CEA antibody, which competes with the anti-CEA antibody of any one as described above, for the binding to human CEA or epitope thereof or fragment thereof.

In some embodiments, the anti-CEA antibody as described above not only binds to human CEA on the surface of cell membrane (membrane-bound or trans-membrane type), (for example, with high affinity), but also binds to monkey CEA on the surface of cell membrane (for example, with high affinity). Furthermore, the ratio of the EC50 value of the anti-CEA antibody binding to monkey CEA on the cell membrane surface to the EC50 value for binding to human CEA on the cell membrane surface is ≤3.5, preferably ≤3.4, ≤3.3, ≤3.2, ≤3.1, ≤3.0, ≤2.9, ≤2.8, ≤2.7, ≤2.6, ≤2.5, ≤2.4, ≤2.3, ≤2.2, ≤2.1, ≤2.0, ≤ 1.9, ≤1.8, ≤1.7, ≤1.6, ≤1.5, ≤1.0, ≤0.9, ≤0.8, ≤0.7, ≤0.6, ≤0.5, ≤0.4, ≤0.3, ≤0.2 or ≤0.1, more preferably ≤0.7, ≤0.6, ≤0.5, ≤0.4, ≤0.3, ≤0.2, the EC50 value (nM) of the anti-CEA antibody binding to CEA is determined by FACS method, and an illustrative example is shown in Test Example 1 of the present disclosure.

In some embodiments, the anti-CEA antibody as described above competitively binds to human CEA (CEA) on the surface of cell membrane when compared to soluble human CEA (sCEA). In some embodiments, in cells expressing human CEA (such as MKN45) without sCEA *v.s*. with sCEA, the maximum value of the fluorescence signal ratio showing the anti-CEA antibody binding to the antigen is less than 2.0, more preferably ≤ 1.9, ≤1.8, ≤1.7, ≤1.6, ≤1.5, ≤1.4, ≤1.3, ≤1.2, ≤1.1 or ≤1.0, the fluorescence signal value is detected by the FACS method, such as Test Example 2 of the present disclosure.

In some embodiments, the anti-CEA antibody binds to soluble human CEA with a KD value greater than or equal to 10⁻⁷M (for example, greater than or equal to 10⁻⁷M, greater than or equal to 1× 10⁻⁶M, greater than or equal to 1× 10⁻⁵M, preferably greater than or equal to 1×10⁻⁷M and less than or equal to 5×10⁻⁶M); meanwhile, the anti-CEA antibody binds to human or monkey CEA on the surface of cell membrane with a EC50 value less than or equal to 10⁻⁹M (for example, less than or equal to 9×10⁻⁹M, less than or equal to 8×10⁻⁹M, less than or equal to 7×10⁻⁹M, less than or equal to 6×10⁻⁹M, less than or equal to 5×10⁻⁹M, less than or equal to 4×10⁻⁹M, less than or equal to 3 × 10⁻⁹M, less than or equal to 2×10⁻⁹M, less than or equal to 1×10⁻⁹M, less than or equal to 1×10⁻¹⁰); the EC50 value is determined using the FACS method, such as described in Test Example 1 of the present disclosure; the KD value is determined using the Biacore method, such as Test Example 3 of the present disclosure.

In some embodiments, the anti-CEA antibody as described above has the following features:
a. The anti-CEA antibody competitively binds to human CEA on the surface of cell membrane when compared to soluble human CEA (sCEA); and in cells expressing human CEA (such as MKN45) without sCEA *v.s*. with sCEA, the maximum value of the fluorescence signal ratio showing the anti-CEA antibody binding to the antigen is less than 1.7, and the fluorescent signal value is detected by the FACS method;
b. The anti-CEA antibody not only binds to the human CEA on the surface of cell membrane with high affinity, but also binds to monkey CEA on the surface of cell membrane with high affinity, and the ratio of the EC50 value of the anti-CEA antibody binding to monkey CEA on the cell membrane surface to the EC50 value for binding to human CEA on the cell membrane surface is ≤0.7, the EC50 value is determined by the FACS method; and/or
c. The anti-CEA antibody binds to soluble human CEA with a KD value greater than or equal to 1×10⁻⁷M and less than or equal to 5×10⁻⁶; meanwhile, the anti-CEA antibody binds to human or monkey CEA on the surface of cell membrane with an EC50 value less than or equal to 8×10⁻⁹; the EC50 value is determined by the FACS method, and the KD value is determined by the Biacore method.

In some embodiments, the present disclosure also provides a nucleic acid molecule that encodes the anti-CEA antibody of any one as described above.

In some embodiments, the present disclosure also provides a host cell comprising the nucleic acid molecule as described above.

In some embodiments, the present disclosure also provides an antibody-drug conjugate, which is formed by conjugating the anti-CEA antibody of any one as described above to a cytotoxic drug.

In other embodiments, the present disclosure also provides an antibody-drug conjugate comprising or consisting of:
- the anti-CEA antibody as described above, and
- a cytotoxic drug;
   wherein, the cytotoxic drug is covalently conjugated to the anti-CEA antibody.

In some embodiments, the present disclosure also provides a pharmaceutical composition, comprising a prophylactically or therapeutically effective amount of the anti-CEA antibody, nucleic acid molecule, or antibody-drug conjugate of any one as described above, and one or more pharmaceutically acceptable carriers, diluents, buffers or excipients.

In some embodiments, the present disclosure provides a method for the immunoassay or detection of CEA, including a step of contacting the above-mentioned anti-CEA antibody with a sample.

In some embodiments, the present disclosure also provides a kit comprising the anti-CEA antibody of any one as described above.

In some embodiments, the present disclosure also provides a method for preventing or treating a disease, the method comprising administering to a subject a prophylactically or therapeutically effective amount of the anti-CEA antibody, nucleic acid molecule, or antibody-drug conjugate or pharmaceutical composition of any one as described above.

In some embodiments, the present disclosure also provides a method of treating CEA-related diseases, the method comprising administering to a subject a therapeutically effective amount of the anti-CEA antibody, nucleic acid molecule, or antibody-drug conjugate or pharmaceutical composition of any one as described above.

In some embodiments, wherein the disease as described above is tumor; in other embodiments, the disease is selected from the group consisting of: head and neck squamous cell cancer, head and neck cancer, brain cancer, glioma, glioblastoma multiforme, neuroblastoma, central nervous system cancer, neuroendocrine tumor, throat cancer, nasopharyngeal cancer, esophageal cancer, thyroid cancer, malignant pleural mesothelioma, lung cancer, breast cancer, liver cancer, hepatocellular tumor, hepatocellular cancer, liver and gallbladder cancer, pancreatic cancer, gastric cancer, gastrointestinal cancer, intestinal cancer, colon cancer, colorectal cancer, kidney cancer, clear cell renal cell carcinoma, ovarian cancer, endometrial cancer, cervical cancer, bladder cancer, prostate cancer, testicular cancer, skin cancer, melanoma, leukemia, lymphoma, bone cancer, chondrosarcoma, myeloma, multiple myeloma, myelodysplastic syndrome, Krukenberg tumor, myeloproliferative tumor, squamous cell carcer, Ewing sarcoma, systemic light chain amyloidosis and Merkel cell carcer; In other embodiments, the lymphoma is selected from the group consisting of: Hodgkin's lymphoma, non-Hodgkin's lymphoma, diffuse large B-cell lymphoma, follicular lymphoma, primary mediastinal large B-cell lymphoma, mantle cell lymphoma, small lymphocytic lymphoma, large B-cell lymphoma rich in T-cells/histiocytes, and lymphoplasmacytic lymphoma; the lung cancer is selected from the group consisting of: non-small cell lung cancer and small cell lung cancer; and the leukemia is selected from the group consisting of: chronic myeloid leukemia, acute myeloid leukemia, lymphocytic leukemia, lymphoblastic leukemia, acute lymphoblastic leukemia, chronic lymphocytic leukemia, and myeloid leukemia.

In some embodiments, the prophylactically or therapeutically effective amount as described above refers to a unit dose of the composition comprising 0.1 mg to 3000 mg (preferably 1 mg to 1000 mg) of the anti-CEA antibody, nucleic acid molecule, antibody-drug conjugate or pharmaceutical composition as described above.

In some embodiments, the present disclosure also provides use of the anti-CEA antibody, nucleic acid molecule, antibody-drug conjugate or pharmaceutical composition of any one as described above in the preparation of a medicament for preventing or treating a disease.

In some embodiments, the present disclosure also provides use of the anti-CEA antibody, nucleic acid molecule, antibody-drug conjugate or pharmaceutical composition of any one as described above in the preparation of a medicament for treating a disease or disorder.

In some embodiments, the disease as described above is preferably CEA-related disease. In some embodiments, wherein the disease is a tumor.

In other embodiments, the disease is selected from the group consisting of: head and neck squamous cell cancer, head and neck cancer, brain cancer, glioma, glioblastoma multiforme, neuroblastoma, central nervous system cancer, neuroendocrine tumor, throat cancer, nasopharyngeal cancer, esophageal cancer, thyroid cancer, malignant pleural mesothelioma, lung cancer, breast cancer, liver cancer, hepatocellular tumor, hepatocellular cancer, liver and gallbladder cancer, pancreatic cancer, gastric cancer, gastrointestinal cancer, intestinal cancer, colon cancer, colorectal cancer, kidney cancer, clear cell renal cell carcinoma, ovarian cancer, endometrial cancer, cervical cancer, bladder cancer, prostate cancer, testicular cancer, skin cancer, melanoma, leukemia, lymphoma, bone cancer, chondrosarcoma, myeloma, multiple myeloma, myelodysplastic syndrome, Krukenberg tumor, myeloproliferative tumor, squamous cell carcer, Ewing sarcoma, systemic light chain amyloidosis and Merkel cell carcer; In other embodiments, the lymphoma is selected from the group consisting of: Hodgkin's lymphoma, non-Hodgkin's lymphoma, diffuse large B-cell lymphoma, follicular lymphoma, primary mediastinal large B-cell lymphoma, mantle cell lymphoma, small lymphocytic lymphoma, large B-cell lymphoma rich in T-cells/histiocytes, and lymphoplasmacytic lymphoma; the lung cancer is selected from the group consisting of: non-small cell lung cancer and small cell lung cancer; and the leukemia is selected from the group consisting of: chronic myeloid leukemia, acute myeloid leukemia, lymphocytic leukemia, lymphoblastic leukemia, acute lymphoblastic leukemia, chronic lymphocytic leukemia, and myeloid leukemia.

In some embodiments, the present disclosure also provides the anti-CEA antibody, or the nucleic acid molecule, or the antibody-drug conjugate or the pharmaceutical composition of any one as described above, for use in the prevention or treatment of disease. In some embodiments, the disease as described above is preferably CEA-related disease. In some embodiments, wherein the disease is a tumor. In other embodiments, the disease is selected from the group consisting of: head and neck squamous cell cancer, head and neck cancer, brain cancer, glioma, glioblastoma multiforme, neuroblastoma, central nervous system cancer, neuroendocrine tumor, throat cancer, nasopharyngeal cancer, esophageal cancer, thyroid cancer, malignant pleural mesothelioma, lung cancer, breast cancer, liver cancer, hepatocellular tumor, hepatocellular cancer, liver and gallbladder cancer, pancreatic cancer, gastric cancer, gastrointestinal cancer, intestinal cancer, colon cancer, colorectal cancer, kidney cancer, clear cell renal cell carcinoma, ovarian cancer, endometrial cancer, cervical cancer, bladder cancer, prostate cancer, testicular cancer, skin cancer, melanoma, leukemia, lymphoma, bone cancer, chondrosarcoma, myeloma, multiple myeloma, myelodysplastic syndrome, Krukenberg tumor, myeloproliferative tumor, squamous cell carcer, Ewing sarcoma, systemic light chain amyloidosis and Merkel cell carcer; In other embodiments, the lymphoma is selected from the group consisting of: Hodgkin's lymphoma, non-Hodgkin's lymphoma, diffuse large B-cell lymphoma, follicular lymphoma, primary mediastinal large B-cell lymphoma, mantle cell lymphoma, small lymphocytic lymphoma, large B-cell lymphoma rich in T-cells/histiocytes, and lymphoplasmacytic lymphoma; the lung cancer is selected from the group consisting of: non-small cell lung cancer and small cell lung cancer; and the leukemia is selected from the group consisting of: chronic myeloid leukemia, acute myeloid leukemia, lymphocytic leukemia, lymphoblastic leukemia, acute lymphoblastic leukemia, chronic lymphocytic leukemia, and myeloid leukemia.

In some embodiments, the anti-CEA antibody, the nucleic acid molecule or the antibody-drug conjugate according to the present disclosure is the only active ingredient in the prevention or treatment.

In other embodiments, the anti-CEA antibody, the nucleic acid molecule, and the antibody-drug conjugate according to the present disclosure can be administered in combination with other active ingredient(s).

### DESCRIPTION OF THE DRAWINGS

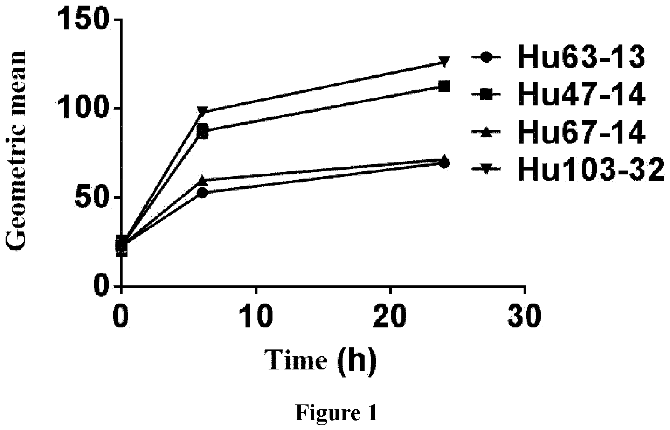

Figure 1: Endocytosis activity of the humanized antibodies in MKN45 cells.

### DETAILED DESCRIPTION OF THE INVENTION

### Terminology

In order to make the present disclosure be more easily understood, certain technical and scientific terms are specifically defined below. Unless otherwise defined explicitly herein, all other technical and scientific terms used herein have the meaning commonly understood by those skilled in the art to which this disclosure belongs.

Three-letter codes and one-letter codes for amino acids used in the present disclosure are as described in J. biol. chem, 243, p3558 (1968).

The term "cytotoxic drug" refers to a substance that inhibits or prevents the function of cells and/or results in cell death or destruction. Cytotoxic drugs comprise toxins, chemotherapeutic drugs and other compounds that can be used to kill tumor cells.

The term "toxin" refers to any substance capable of generating harmful effects on the growth or proliferation of cells, and such substance can be a small molecule toxin and derivative thereof from bacteria, fungi, plants or animals, including, but not limited to, camptothecin derivatives such as exatecan, maytansinoids and derivatives thereof (CN101573384) such as DM1, DM3, DM4, auristatin F (AF) and derivatives thereof, such as MMAF, MMAE, 3024 (WO 2016/127790 A1, compound 7), diphtheria toxin, exotoxin, ricin A chain, abrin A chain, modeccin, α-sarcin, *Aleutites fordii* toxic protein, *dianthin* toxic protein, *Phytolaca americana* toxic protein (PAPI, PAPII and PAP-S), *Momordica charantia* inhibitor, curcin, crotin, *Sapaonaria officinalis* inhibitor, gelonin, mitogellin, restrictocin, phenomycin, enomycin, and trichothecenes.

The term "chemotherapeutic drug" is a chemical compound that can be used for the treatment of tumors. Such definition also includes antihormone agents capable of modulating, reducing, blocking, or inhibiting the hormonal effects that can promote cancer growth, and are generally in the form of systemic therapy. They can be hormones themselves. Examples of chemotherapeutic drugs include alkylating agents, such as thiotepa; cyclosphamide (CYTOXAN^{™}); Alkyl sulfonate such as busulfan, improsulfan and piposulfan; aziridine such as benaodopa, carboquone, meturedopa and uredopa; aziridine and methylamelamine including altretamine, triethylenemelamine, triethylene phosphoramide, triethylene thiophosphoramide and trimethylolomelamine; nitrogen mustards such as chlorambucil, chlornaphazine, cholophosphamide, estramustine, ifosfamide, mechlorethamine, mechlorethaminoxide; melphalan, novembichin, phenesterine, prednimustine, trofosfamide, uramustine; nitrosureas such as carmustine, chlorozotocin, fotemustine, lomustine, nimustine, ranimustine; antibiotics such as clarithromycin, actinomycin, authramycin, azaserine, bleomycin, cactinomycin, calicheamicin, carabicin, chromomycin, carzinophilin, chromomycin, actinomycin D, daunorubicin, detorubicin, 6-diazo-5-oxy-L-norleucine, doxorubicin, epirubicin, esorubicin, idarubicin, marcellomycin, mitomycin, mycophenolic acid, nogalamycin, olivomycin, peplomycin, potfiromycin, puromycin, quelamycin, rodorubicin, streptonigrin; streptozocin, tubercidin, ubenimex, zinostatin, zorubicin; antimetabolites such as methotrexate, 5-FU; folic acid analogs such as denopterin, methotrexate, pteropterin, trimetrexate; methotrexate analogs such as fludarabine, 6-mercaptopterin, thiometherin, thioguanopterin; pyrimidine analogs such as ancitabine, azacitidine, 6-azuridine, carmofur, cytarabine, dideoxyuridine, doxitluridine, enocitabine, fluorouridine, 5-FU; androgens such as calusterone, dromostanolong propionate, epitiostanol, mepitiostane, testolactone; anti-adrenergic agents such as aminoglutethimide, mitotane, trilostane; folic acid supplements such as frolinic acid; acetoglucone; aldophosphamideglycoside; aminolevulinic acid; amsacrine; bestrabucil; biasntrene; edatraxate; defofamine; colchicine; diaziquone; elfomithine; elliptinium acetate; etoglucid; gallium nitrate; hydroxyurea; lentinan; lonidamine; mitoguazone; mitoxantrone; mopidamol; nitracrine; pintostatin; phenamet; pirarubicin; podophyllinic acid; 2-ethylhydrazide; procarbazine; PSK^{®}; razoxane; sizofiran; spirogermanium; Alternaria tenuazonic acid; triimine quinone; 2,2',2"- trichlorrotriethylamine; urethan; vinblastine amide; dacarbazine; mannitol mustard; dibromomannitol (mitobronitol); dibromo euonymus alcohol; pipobroman; gacytosine; arabinoside ("Ara-C"); cyclophosphamide; thiotepa; taxanes such as paclitaxel (TAXOL^{®}, Bristol-Myers Squibb Oncology, Princeton, NJ) and docetaxel (TAXOTERE^{®}, Rhone-Poulenc Rorer, Antony, France); chlorambucil; gemcitabine; 6-thioguanine; mercaptopurine; methotrexate; platinum analogs such as cisplatin and carboplatin; vinblastine; platinum; etoposide (VP-16); ifosfamide; mitomycin C; mitoxantrone; vincristine; vinorelbine; navelbine; novantrone; teniposide; daunorubicin; aminopterin; xeloda; ibandronate; CPT-11; Topoisomerase inhibitor RFS2000; Difluoromethylornithine (DMFO); retinoic acid, esperamicins; capecitabine; and pharmaceutically acceptable salts, acids or derivatives of any of the above substances. This definition also includes anti-hormonal agents that can modulate or inhibit the effect of hormones on tumors. For example, anti-estrogens include tamoxifen, raloxifene, aromatase inhibitor 4(5)-imidazole, 4-hydroxyl tamoxifen, trioxifene, keoxifene, LY117018, onapristone, and toremifene (Fareston); and anti-androgens such as flutamide, nilutamide, bicalutamide, leuprolide and goserelin; and pharmaceutically acceptable salts, acids or derivatives of any of the above substances.

As used herein, "antibody" refers to an immunoglobulin, and a complete antibody is generally a four-peptide chain structure formed by two identical heavy chains and two identical light chains connected together by interchain disulfide bond(s). Immunoglobulin heavy chain constant regions exhibit different amino acid compositions and orders, hence present different antigenicity. Accordingly, immunoglobulins can be divided into five types, or named as immunoglobulin isotypes, namely IgM, IgD, IgG, IgA and IgE, and the corresponding heavy chains are µ, δ, γ, α and ε, respectively. According to its amino acid composition of hinge region and the number and location of heavy chain disulfide bonds, the same type of Ig can further be divided into different sub-types, for example, IgG can be divided into IgG1, IgG2, IgG3 and IgG4. Light chains can be divided into κ or λ chain based on different constant regions. Each of the five types of Ig has a kappa chain or a lambda chain.

About 110 amino acid sequences adjacent to the N-terminus of the antibody heavy and light chains are highly variable, known as variable regions (Fv regions); the rest of amino acid sequences close to the C-terminus are relatively stable, known as constant regions. The variable region comprises 3 hypervariable regions (HVRs) and 4 framework regions (FRs) with relatively conservative sequences. The three hypervariable regions which determine the specificity of the antibody are also known as complementarity determining regions (CDRs). Each light chain variable region (VL) and each heavy chain variable region (VH) consists of three CDR regions and four FR regions, arranged from the amino terminus to carboxyl terminus in the following order: FR1, CDR1, FR2, CDR2, FR3, CDR3, and FR4. The three CDR regions of the light chain refer to LCDR1, LCDR2, and LCDR3, and the three CDR regions of the heavy chain refer to HCDR1, HCDR2, and HCDR3.

The antibodies of the present disclosure include murine antibodies, chimeric antibodies, and humanized antibodies. In addition to full-length antibodies, the antibodies of the present disclosure also include antigen-binding fragments capable of binding to the antigen.

As used herein, the term "murine antibody" refers to monoclonal antibodies against human CEA prepared according to the knowledge and skills in the art. During the preparation, test subject is injected with CEA antigen, and then a hybridoma expressing the antibody which possesses desired sequence or functional characteristics is isolated. In a preferred embodiment of the present disclosure, the murine anti-CEA antibody or antigen-binding fragments thereof could further comprise a light chain constant region of murine kappa, lambda chain or variants thereof, or further comprise a heavy chain constant region of murine IgG1, IgG2, IgG3, or variants thereof.

The term "chimeric antibody" is an antibody formed by fusing the variable region of a murine antibody with the constant region of a human antibody, which can alleviate the immune response induced by the murine antibody. To establish a chimeric antibody, it is necessary to first establish a hybridoma secreting murine-derived specific monoclonal antibodies, then clone the variable region genes from the mouse hybridoma cells, and then clone the constant region genes of the human antibody as needed. The murine variable region gene is connected with the human constant region gene to form a chimeric gene which is inserted into an expression vector, and finally the chimeric antibody molecule is expressed in a eukaryotic system or a prokaryotic system. In a preferred embodiment of the present disclosure, the antibody light chain of the chimeric antibody further comprises a light chain constant region of human kappa, lambda chain or a variant thereof. The antibody heavy chain of the CEA chimeric antibody further comprises a heavy chain constant region of human IgG1, IgG2, IgG3, IgG4 or variants thereof, preferably comprises a heavy chain constant region of human IgG1, IgG2 or IgG4, or of IgG1, IgG2 or IgG4 variant with amino acid mutation(s) (such as L234A and/or L235A mutation, and/or S228P mutation).

The term "humanized antibody", also known as CDR-grafted antibody, refers to an antibody generated by grafting the murine CDR sequences into human antibody variable region frameworks, i.e., an antibody produced in different types of human germline antibody framework sequences. Humanized antibody can avoid heterologous responses induced by chimeric antibody which carries a large number of murine protein components. Such framework sequences can be obtained from public DNA database or from published references covering germline antibody gene sequences. For example, germline DNA sequences of human heavy and light chain variable region genes can be found in "VBase" human germline sequence database (available on www.mrccpe.com.ac.uk/vbase), as well as in Kabat, EA, et al. 1991 Sequences of Proteins of Immunological Interest, 5th Ed. To avoid a decrease in activity caused by the decreased immunogenicity, the framework sequences in human antibody variable region can be subjected to minimal reverse mutation or back mutation to maintain the activity. The humanized antibodies of the present disclosure also comprise humanized antibodies on which CDR affinity maturation is performed by yeast display.

Because of the presence of residues that are in contacted with an antigen, the grafting of CDRs can result in a decreased affinity of an antibody or antigen binding fragment thereof to the antigen, due to the framework residues in contacted with the antigen. Such interactions can be resulted from highly somatic mutations. Therefore, it is necessary to graft such donor framework amino acids onto the humanized antibody frameworks. The amino acid residues involved in antigen binding and derived from non-human antibody or antigen binding fragment thereof can be identified by checking the sequence and structure of animal monoclonal antibody variable region. The donor CDR framework amino acid residues which are different from the germ lines can be considered as being related. If it is not possible to determine the most closely related germ line, the sequence can be compared to the consensus sequence shared by subtypes or the animal antibody sequence with high similarity percentage. Rare framework residues are thought to be the result of a high mutation in somatic cells, and play an important role in binding.

In an embodiment of the present disclosure, the antibody or antigen binding fragment thereof further comprises a light chain constant region derived from human or muine κ, λ chain or variant thereof, or further comprises a heave chain constant region derived from human or murien IgG1, IgG2, IgG3, IgG4 or variant thereof; preferably comprises a heavy chain constant region derived from human IgG1, IgG2 or IgG4, or IgG1, IgG2 or IgG4 variant with amino acid mutation(s) (such as L234A and/or L235A mutation, and/or S228P mutation).

As used in the present disclosure, the "conventional variants" of the human antibody heavy chain constant region and the human antibody light chain constant region refer to the human heavy or chain constant region variants disclosed in the prior art which do not change the structure and function of the antibody variable regions. Exemplary variants include IgG1, IgG2, IgG3 or IgG4 heavy chain constant region variants by site-directed modification and amino acid substitutions on the heavy chain constant region. The particular substitutions are, for example, YTE mutation, L234A and/or L235A mutations, S228P mutation, or mutations resulting in a knob-into-hole structure (the antibody heavy chain is allowed to have a combination of knob-Fc and hole-Fc), etc. These mutations have been demonstrated to confer new properties on the antibody, without changing the function of the antibody variable region.

"Human antibody (HuMAb)", "antibody derived from human ", "fully human antibody" and "completely human antibody" are used interchangeably, and can be antibodies derived from human or antibodies obtained from a genetically modified organism which has been "engineered" by any method known in the art to produce human antibodies in response to antigen stimulation. In some technologies, elements of human heavy and light chain loci are introduced into cell lines of organisms derived from embryonic stem cell lines, and the endogenous heavy and light chain loci in these cell lines are targeted and disrupted. Transgenic organisms can synthesize human antibodies specific for human antigens, and the organisms can be used to produce hybridomas that secrete human antibodies. A human antibody can also be such antibody in which the heavy and light chains are encoded by nucleotide sequences derived from one or more human DNA sources. Fully human antibodies can also be constructed by gene or chromosome transfection methods and phage display technology, or constructed from B cells activated *in vitro,* all of which are known in the art.

The terms "full-length antibody", "full antibody", " intact antibody" and "complete antibody" are used interchangeably herein and refer to an antibody in a substantially complete form, which is distinguished from antigen-binding fragments defined below. The term specifically refers to antibodies that contain constant regions in the light and heavy chains. The "antibody" of the present disclosure includes "full-length antibodies" and antigen-binding fragments thereof.

In some embodiments, the full-length antibody of the present disclosure includes full-length antibodies formed by a light chain (a light chain variable region linked to a light chain constant region) and a heavy chain (a heavy chain variable region linked to a heavy chain constant region). Those skilled in the art can select the light chain constant region and heavy chain constant region from various antibody sources according to actual needs, such as human antibody-derived light chain constant region and heavy chain constant region.

The term "antigen-binding fragment" or "functional fragment" of an antibody refers to one or more fragments of the antibody that retain the ability to specifically bind to an antigen (e.g., CEA). It has been shown that fragments of a full-length antibody can be used to achieve function of binding to an antigen. Examples of the binding fragments contained in the term "antigen-binding fragment" of an antibody include (i) Fab fragment, a monovalent fragment composed of VL, VH, CL and CH1 domains; (ii) F(ab')₂ fragment, a bivalent fragment comprising two Fab fragments connected by a disulfide bridge in the hinge region, (iii) Fd fragment composed of VH and CH1 domains; (iv) Fv fragment composed of the VH and VL domains of one arm of the antibody; (v) dsFv, a stable antigen-binding fragment formed by VH and VL via interchain disulfide bonds; and (vi) diabody, bispecific antibody and multispecific antibody containing fragments such as scFv, dsFv, or Fab. In addition, the two domains (VL and VH domain) of the Fv fragment are encoded by two separate genes, however they can be linked by a synthetic linker by using recombinant methods, to generate a single protein chain in which a monovalent molecular is formed by pairing the VL with VH domain (referred to as single chain Fv (scFv); see, e.g., Bird et al. (1988): 423-426; Science 242 and Huston et al (1988) Proc. Natl. Acad. Sci USA85:5879-5883). Such single chain antibodies are also included in the term of "antigen binding fragment" of an antibody. Such antigen binding fragments are obtained using conventional techniques known in the field, and can be screened by using the same method as that for an intact antibody. Antigen binding portions can be produced by recombinant DNA technology or by enzymatic or chemical disruption of an intact immunoglobulin. Antibodies can be in the form of different isotypes, e.g., IgG (e.g., IgG1, IgG2, IgG3 or IgG4 subtype), IgA1, IgA2, IgD, IgE or IgM antibody.

Fab is an antigen binding fragment obtained by treating an IgG antibody molecule with a papain (which cleaves the amino acid residue of H chain), and the antibody fragment has a molecular weight of about 50,000 and has antigen binding activity, in which about a half of the N-terminal side of H chain and the entire L chain are bound together through disulfide bond(s).

F(ab')2 is an antigen binding fragment having molecular weight of about 100,000 and having antigen binding activity and comprising two Fab regions which are bound in the hinge position, it can be produced by digesting the part downstream the two disulfide bonds in the hinge region of IgG with pepsin.

Fab' is an antigen binding fragment having a molecular weight of about 50,000 and having antigen binding activity, which is obtained by cleaving the disulfide bonds at the hinge region of the above-mentioned F (ab')2. Fab' of the present disclosure can be produced by treating F(ab')2 that specifically recognizes and binds to CEA with a reducing agent such as dithiothreitol.

Further, the Fab' can be produced by inserting DNA encoding Fab' fragment of the antibody into a prokaryotic expression vector or eukaryotic expression vector and introducing the vector into a prokaryote or eukaryote to express the Fab'.

The term "single chain antibody", "single chain Fv" or "scFv" refers to a molecule comprising antibody heavy chain variable domain (or region; VH) connected to antibody light chain variable domain (or region; VL) by a linker. Such scFv molecules have general structure of NH₂-VL-linker-VH-COOH or NH₂-VH-linker-VL-COOH. A suitable linker in the prior art consists of repeated GGGGS amino acid sequence or variant thereof, for example, variant with 1-4 repeats (Holliger et al. (1993), Proc. Natl. Acad. Sci. USA 90:6444-6448). Other linkers that can be used in the present disclosure are described by Alfthan et al. (1995), Protein Eng. 8:725-731, Choi et al. (2001), Eur. J. Immunol. 31:94-106, Hu et al. (1996), Cancer Res. 56:3055-3061, Kipriyanov et al. (1999), J. Mol. Biol. 293:41-56 and Roovers et al. (2001), Cancer Immunol.

Diabody is an antibody fragment where scFv or Fab is dimerized, and it is an antigen binding fragment having divalent antigen binding activity. In the bivalent antigen binding activity, the two antigens can be the same or different.

Bispecific and multispecific antibody refer to an antibody that can simultaneously bind to two or more antigens or antigenic determinants, including scFv or Fab fragments that can bind CEA.

The diabody of the present disclosure can be produced by the following steps: obtaining cDNAs encoding VH and VL of the monoclonal antibody specifically recognizing and binding to human CEA of the present disclosure, constructing a DNA encoding scFv to make the length of a linker peptide of 8 or less amino acid residues, inserting the DNA into a prokaryotic or eukaryotic expression vector, and then introducing the expression vector into a prokaryote or eukaryote to express the diabody.

dsFv is obtained by substituting one amino acid residue in each of VH and VL with a cysteine residue, and then connecting the substituted polypeptides via a disulfide bond between the two cysteine residues. The amino acid residues to be substituted with a cysteine residue can be selected based on three-dimensional structure prediction of the antibody in accordance with known methods (Protein Engineering, 7, 697 (1994)).

The full-length antibody or antigen-binding fragment of the present disclosure can be produced by the following steps: obtaining the cDNA encoding the antibody of the present disclosure that specifically recognizes and binds to human CEA, constructing DNA encoding dsFv, inserting the DNA into a prokaryotic expression vector or a eukaryotic expression vector, and then introducing the expression vector into a prokaryote or eukaryote to express the dsFv.

The term "amino acid difference" or "amino acid mutation" refers to amino acid changes or mutations in a protein or polypeptide variant compared to the original protein or polypeptide, including 1, 2, 3 or more amino acid insertions, deletions or substitutions on the basis of the original protein or polypeptide.

The term "antibody framework" or "FR region" refers to a part of the variable domain, either VL or VH, which serves as a scaffold for the antigen binding loops (CDRs) of this variable domain. Essentially, it is a variable domain without CDRs.

The term "complementarity determining region", "CDR" or "hypervariable region" refers to one of the six hypervariable regions present in the antibody variable domain that mainly contribute to antigen binding. Generally, there are three CDRs (HCDR1, HCDR2, HCDR3) in each heavy chain variable region, and three CDRs (LCDR1, LCDR2, LCDR3) in each light chain variable region. The amino acid sequence boundaries of CDRs can be determined by any of a variety of well-known schemes, including the "Kabat" numbering criteria (see Kabat et al. (1991), "Sequences of Proteins of Immunological Interest", 5th edition, Public Health Service, National Institutes of Health, Bethesda, MD), "Chothia" numbering criteria (see Al-Lazikani et al., (1997) JMB 273:927-948) and ImmunoGenTics (IMGT) numbering criteria (Lefranc MP, Immunologist, 7, 132- 136 (1999); Lefranc, MP, etc., Dev. Comp. Immunol., 27, 55-77 (2003), and the like. For example, for the classical format, following the Kabat criteria, the CDR amino acid residues in the heavy chain variable domain (VH) are numbered as 31-35 (HCDR1), 50-65 (HCDR2) and 95-102 (HCDR3); and the CDR amino acid residues in the light chain variable domain (VL) are numbered as 24-34 (LCDR1), 50-56 (LCDR2), and 89-97 (LCDR3). Following the Chothia criteria, the CDR amino acid residues in VH are numbered as 26-32 (HCDR1), 52-56 (HCDR2) and 95-102 (HCDR3); and the amino acid residues in VL are numbered as 26-32 (LCDR1), 50- 52 (LCDR2) and 91-96 (LCDR3). By combining both Kabat and Chothia to define CDRs, the CDRs are composed of amino acid residues 26-35 (HCDR1), 50-65 (HCDR2) and 95-102 (HCDR3) in the human VH and amino acid residues 24-34 (LCDR1), 50-56 (LCDR2) and 89-97 (LCDR3) in the human VL. Following IMGT criteria, the CDR amino acid residues in VH are roughly numbered as 26-35 (CDR1), 51-57 (CDR2) and 93-102 (CDR3), and the CDR amino acid residues in VL are roughly numbered as 27-32 (CDR1), 50-52 (CDR2) and 89-97 (CDR3). Following IMGT criteria, the CDR regions of an antibody can be determined by using IMGT/DomainGap Align Program. Unless otherwise specified, the antibody variable regions and CDR sequences involved in the specific embodiments of the present disclosure are applicable for the "Kabat" numbering criteria.

The term "epitope" or "antigenic determinant" refers to a site on an antigen to which an immunoglobulin or antibody specifically binds (e.g., a specific site on CEA molecule). Epitopes include linear epitopes and conformational epitopes. For example, conformational epitopes usually have a unique spatial conformation and include at least 3, 4, 5, 6, 7, 8, 9, 10, 11, 12, 13, 14 or 15 contiguous or non-contiguous amino acids. See, for example, Epitope Mapping Protocols in Methods in Molecular Biology, Vol. 66, G.E. Morris, Ed. (1996).

The term "specifically bind to" or "selectively bind to" refers to the binding of an antibody to a predetermined epitope on an antigen. Typically, the antibody binds with an affinity (KD) of less than about 10⁻⁷M, for example, less than about 10⁻⁸ M, 10⁻⁹ M, 10⁻¹⁰ M, 10⁻¹¹ M or 10⁻¹² M or even less.

The term "KD" refers to the dissociation equilibrium constant for particular antibody-antigen interaction. Generally, the antibody of the present disclosure binds to CEA with a dissociation equilibrium constant (KD) of less than about 10⁻⁷ M, for example, less than about 10⁻⁸ M or 10⁻⁹ M, for example, the affinity of the antibody in the present disclosure to the cell surface antigen is determined by measuring KD value with Biacore method.

When the term "competition" is used in the context of antigen binding proteins (e.g., neutralizing antigen binding proteins or neutralizing antibodies) that compete for the same epitope, it means that competition occurs between the antigen binding proteins, which is determined by the assays in which an antigen binding protein to be tested (e.g., an antibody or immunologically functional fragment thereof) prevents or inhibits (e.g., reduces) the specific binding of a reference antigen binding protein (e.g., a ligand or reference antibody) to a common antigen (e.g., a CEA antigen or fragments thereof). Numerous types of competitive binding assays are available to determine whether an antigen binding protein competes with another. These assays are, for example, solid phase direct or indirect radioimmunoassay (RIA), solid phase direct or indirect enzyme immunoassay (EIA), Sandwich competition assay (see, e.g., Stahli et al., 1983, Methods in Enzymology 9: 242-253); solid phase direct biotin-avidin EIA (see, e.g., Kirkland et al., 1986, J. Immunol. 137: 3614-3619), solid phase direct labeling assay, solid phase direct labeling sandwich assay (see, e.g., Harlow and Lane, 1988, Antibodies, A Laboratory Manual, Cold Spring Harbor Press); solid phase direct labeling RIA with 1-125 label (see, e.g., Morel et al., 1988, Molec. Immunol. 25: 7-15); solid phase direct biotin-avidin EIA (see, e.g., Cheung, et al., 1990, Virology 176: 546-552); and direct labeling RIA (Moldenhauer et al., 1990, Scand. J. Immunol. 32: 77-82). Typically, the assay involves the use of a purified antigen capable of binding to a solid surface or cell loaded with both an unlabeled test antigen binding protein and a labeled reference antigen binding protein. Competitive inhibition is determined by measuring the amount of label bound to the solid surface or to the cell in the presence of the test antigen binding protein. Usually, the test antigen binding protein is present in excess. Antigen binding proteins identified by competitive assay (competing with the antigen binding protein) includes: antigen binding proteins that bind to the same epitope as that of the reference antigen binding protein; and antigen binding proteins that bind to an epitope that is sufficiently close to the epitope to which the reference antigen binding protein binds, where the two epitopes spatially interfere with each other to hinder the binding. Additional details regarding methods for determining competitive binding are provided in the Examples herein. Typically, when a competitive antigen binding protein is present in excess, it will inhibit (e.g., reduce) the specific binding of the reference antigen binding protein to the common antigen by at least 40-45%, 45-50%, 50-55%, 55-60%, 60-65%, 65-70%, 70-75% or 75% or even more. In some cases, the binding is inhibited by at least 80-85%, 85-90%, 90-95%, 95-97%, or 97% or even more.

As used herein, the term "nucleic acid molecule" refers to DNA molecules and RNA molecules. The nucleic acid molecule can be single-stranded or double-stranded, and is preferably double-stranded DNA or single-stranded mRNA or modified mRNA. A nucleic acid is "operably linked" when it is placed into a functional relationship with another nucleic acid sequence. For instance, a promoter or enhancer is operably linked to a coding sequence if it affects the transcription of the sequence.

Amino acid sequence "identity" refers to the percentage of the amino acid residues that are identical between the first and the second sequence when the amino acid sequences are aligned (introducing gaps when necessary) to achieve the maximum percentage of sequence identity, and any conservative substitution is not considered as part of the sequence identity. For the purpose of determining the percentage of amino acid sequence identity, the alignment can be achieved in a variety of ways within the scope of the art, for example, using publicly available computer software, such as BLAST, BLAST-2, ALIGN, ALIGN-2 or Megalign (DNASTAR) software. Those skilled in the art can determine the parameters suitable for measuring the alignment, including any algorithm required to achieve the maximum alignment over the entire length of the sequences being compared.

The term "expression vector" refers to a nucleic acid molecule capable of transporting another nucleic acid to which it has been linked. In one embodiment, the vector is a "plasmid," which refers to a circular double stranded DNA loop into which additional DNA segments can be ligated. In another embodiment, the vector is a viral vector, wherein additional DNA segments can be ligated into the viral genome. The vectors disclosed herein are capable of self-replicating in the host cell into which they are introduced (e.g., bacterial vectors having a bacterial replication origin and episomal mammalian vectors), or can be integrated into the genome of a host cell upon introduction into the host cell, and thereby are replicated along with the host genome (e.g., non-episomal mammalian vectors).

Methods for producing and purifying antibodies and antigen-binding fragments are well known in the art, for example, Antibodies, A Laboratory Manual, Cold Spring Harbor, chapters 5-8 and 15. For example, mice can be immunized with human CEA or fragment thereof, and the resulting antibodies can then be renatured, purified, and sequenced for amino acid sequences by using conventional methods well known in the art. Antigen-binding fragments can also be prepared by conventional methods. The antibodies or antigen binding fragments of the present disclosure are engineered to incorporate one or more human framework regions onto the CDR regions derived from non-human antibody. Human FR germline sequences can be obtained from ImMunoGeneTics (IMGT) via their website http://imgt.cines.fr, or from The Immunoglobulin Facts Book, 2001, ISBN 012441351, by aligning against IMGT database of human antibody variable region germline gene using MOE software.

The term "host cell" refers to a cell into which an expression vector has been introduced. Host cells can include bacterial, microbial, plant or animal cells. Bacteria susceptible to be transformed include members of the *Enterobacteriaceae,* such as *Escherichia coli* or *Salmonella*; the family *Bacillaceae* such as *Bacillus subtilis*; *Pneumococcus; Streptococcus* and *Haemophilus influenzae.* Suitable microorganisms include *Saccharomyces cerevisiae* and *Pichia pastoris.* Suitable animal host cell lines include CHO (Chinese Hamster Ovary Cell Line), 293 cells and NS0 cells.

The engineered antibodies or antigen-binding fragments of the present disclosure can be prepared and purified by conventional methods. For example, the cDNA sequences encoding the heavy and light chains can be cloned and recombined into a GS expression vector. The vectors expressing recombinant immunoglobulin can then be stably transfected into CHO cells. As a more recommended method well known in the art, mammalian expression systems will result in glycosylation, typically at highly conserved N-terminal sites in the Fc region. Stable clones can be obtained by expressing an antibody specifically binding to human CEA. Positive clones can be expanded in serum-free culture medium in bioreactors for antibody production. Culture medium, into which an antibody has been secreted, can be purified by conventional techniques. For example, purification can be performed on Protein A or G Sepharose FF column that has been modified with buffer. The nonspecific binding components are removed by washing. The bound antibody is eluted by pH gradient and antibody fragments are detected by SDS-PAGE, and then pooled. The antibodies can be filtered and concentrated using common techniques. Soluble mixtures and aggregates can be effectively removed by common techniques, such as size exclusion or ion exchange. The resulting product is then immediately frozen, for example at -70°C, or can be lyophilized.

"administration", "dosing" or "treatment," as it applies to an animal, human, experimental subject, cell, tissue, organ, or biological fluid, refers to contacting an exogenous pharmaceutical, therapeutic, diagnostic agent, or composition with the animal, human, subject, cell, tissue, organ, or biological fluid. "Administration", "dosing" or "treatment" can refer, e.g., to therapeutic, pharmacokinetic, diagnostic, research, and experimental methods. The treatment of a cell encompasses contacting a reagent with the cell, as well as contacting a reagent with a fluid, where the fluid is in contact with the cell. "Administering", "dosing" or "treating" also means *in vitro* or *ex vivo* treatments, e.g., of a cell, with a reagent, diagnostic, binding compound, or with another cell. "Treatment", as it applies to a human, veterinary, or research subject, refers to therapeutic treatment, prophylactic or preventative measures, research and diagnostic applications.

"Treat" means to administer a therapeutic agent, such as a composition containing any of binding compounds of the present disclosure, internally or externally to a patient having one or more disease symptoms for which the agent has known therapeutic activity. Typically, the agent is administered in an amount effectively to alleviate one or more disease symptoms in the patient or population to be treated, to induce the regression of or inhibit the progression of such symptom(s) by any clinically measurable degree. The amount of a therapeutic agent that is effective to alleviate any particular disease symptom (also referred to as the "therapeutically effective amount") can vary according to various factors such as the disease state, age, and body weight of the patient, and the ability of the drug to elicit a desired response in the patient. Whether a disease symptom has been alleviated can be assessed by any clinical measurement typically used by physicians or other skilled healthcare providers to assess the severity or progression status of that symptom. While an embodiment of the present disclosure (e.g., a treatment method or article of manufacture) may not be effective in alleviating the target disease symptom(s) in every patient, it should alleviate the target disease symptom(s) in a statistically significant number of patients as determined by any statistical test known in the art such as Student's t-test, chi-square test, U-test according to Mann and Whitney, Kruskal-Wallis test (H-test), Jonckheere-Terpstra-test and Wilcoxon-test.

"Conservative modification" or "conservative substitution or replacement" refers to substitutions of amino acids in a protein with other amino acids having similar characteristics (e.g. charge, side-chain size, hydrophobicity/hydrophilicity, backbone conformation and rigidity, etc.), such that the changes can frequently be made without altering the biological activity of the protein. Those skilled in the art understand that, generally, a single amino acid substitution in a non-essential region of a polypeptide does not substantially change the biological activity (see, for example, Watson et al. (1987) Molecular Biology of the Gene, The Benjamin/Cummings Pub. Co., Page 224, (4th edition)). In addition, substitutions with structurally or functionally similar amino acids are less likely to disrupt biological activity. Exemplary conservative substitutions are set forth below.

| Original residue | Conservative substitution |
|---|---|
| Ala (A) | Gly; Ser |
| Arg(R) | Lys; His |
| Asn (N) | Gln; His; Asp |
| Asp (D) | Glu; Asn |
| Cys (C) | Ser; Ala; Val |
| Gln(Q) | Asn; Glu |
| Glu (E) | Asp; Gln |
| Gly (G) | Ala |
| His (H) | Asn; Gln |
| Ile (I) | Leu; Val |
| Leu (L) | Ile; Val |
| Lys (K) | Arg; His |
| Met (M) | Leu; Ile; Tyr |
| Phe (F) | Tyr; Met; Leu |
| Pro(P) | Ala |
| Ser(S) | Thr |
| Thr(T) | Ser |
| Trp (W) | Tyr; Phe |
| Tyr (Y) | Trp; Phe |
| Val (V) | Ile; Leu. |

"Effective amount" or "effective dose" refers to the amount of a medicament, compound, or pharmaceutical composition necessary to obtain any one or more beneficial or desired prophylactic or therapeutic outcomes. For prophylactic applications, beneficial or desired outcomes include elimination or reduction of risk, reduction of severity, or delay of the onset of the disease, including the biochemical, histological, and behavioral manifestations of the condition, its complications, and intermediate pathological phenotypes during the development of the condition. For therapeutic applications, beneficial or desired outcomes include clinical results, such as reduction of the incidence of various conditions associated with target antigen of the present disclosure or improvement of one or more symptoms of the condition, reduction of the dosage of other agents required to treat the condition, enhancement of the efficacy of another agent, and/or delay of the progression of the condition associated with the target antigen of the present disclosure in patients.

"Exogenous" refers to substances produced outside the organisms, cells, or humans according to circumstances.

"Endogenous" refers to substances produced inside the organisms, cells, or humans according to circumstances.

"Homology" refers to the sequence similarity between two polynucleotide sequences or between two polypeptide sequences. When a position in both of the two sequences to be compared is occupied by the same base or amino acid monomer subunit, e.g., if a position in each of two DNA molecules is occupied by adenine, then the molecules are homologous at that position. The percentage of homology between two sequences is a function of the number of matching or homologous positions shared by the two sequences divided by the number of positions to be compared and then multiplied by 100. For example, when two sequences are optimally aligned, if 6 out of 10 positions in the two sequences are matched or homologous, then the two sequences are 60% homologous; if 95 out of 100 positions in the two sequences are matched or homologous, then the two sequences are 95% homologous. Generally, when two sequences are aligned, comparison is performed to give the maximum homology percentage. For example, the comparison can be performed by BLAST algorithm, in which the parameters of the algorithm are selected to give the maximum match between sequences over the entire length of each reference sequence. The following references refer to the BLAST algorithm frequently used for sequence analysis: BLAST algorithm (BLAST ALGORITHMS): Altschul, SF et al., (1990) J. Mol. Biol. 215:403-410; Gish, W. et al., (1993) Nature Genet. 3:266-272; Madden, TL et al., (1996) Meth. Enzymol. 266:131-141; Altschul, SF et al., (1997) Nucleic Acids Res. 25:3389-3402; Zhang, J. et al. (1997) Genome Res. 7:649-656. Other conventional BLAST algorithms such as those available from NCBI BLAST are also well known to those skilled in the art.

As used herein, the expressions "cell," "cell line," and "cell culture" are used interchangeably and all such designations include progeny. Thus, the words "transformant" and "transformed cell" include the primary subject cells and cultures derived therefrom regardless of the number of passages. It should be also understood that all progeny may not be precisely identical in DNA content, due to intentional or unintentional mutations. Mutant progeny that has the same function or biological activity as that screened from the originally transformed cells are included in the scope of term. Where distinct designations are intended to, it will be clearly understood from the context.

As used herein, "polymerase chain reaction" or "PCR" refers to a procedure or technique in which minute amounts of a specific portion of nucleic acid, RNA and/or DNA, are amplified as described in, e.g., U.S. Pat. No. 4,683,195. Generally, sequence information at the ends of or beyond the region of interest needs to be available, such that oligonucleotide primers can be designed; these primers will be identical or similar in sequence to opposite strands of the template to be amplified. The 5' terminal nucleotides of the two primers can be consistent with the ends of the material to be amplified. PCR can be used to amplify specific RNA sequences, specific DNA sequences from total genomic DNA, and cDNA transcribed from total cellular RNA, bacteriophage or plasmid sequences, etc. See generally Mullis et al. (1987) Cold Spring Harbor Symp. Ouant. Biol. 51:263; Erlich editors, (1989) PCR TECHNOLOGY (Stockton Press, NY). The PCR used in the present disclosure is considered to be one, but not the only, example of polymerase reaction method for amplifying a test nucleic acid sample. The method comprises use of nucleic acid sequences known as primers together with nucleic acid polymerase to amplify or generate a specific portion of nucleic acid.

"Isolated" refers to a purified state, in which the designated molecule is substantially free of other biological molecules, such as nucleic acids, proteins, lipids, carbohydrates, or other materials, such as cell debris and growth medium. In general, the term "isolated" is not intended to mean the complete absence of these materials or the absence of water, buffers or salts, unless they are present in an amount that significantly interferes with the experimental or therapeutic use of the compound as described herein.

"Optional" or "optionally" means that the event or circumstance that follows the term could occur, but does not necessarily occur, and the description includes the instances in which the event or circumstance does or does not occur.

"Pharmaceutical composition" refers to a mixture comprising one or more compounds according to the present disclosure or a physiologically/pharmaceutically acceptable salt or prodrug thereof and other chemical components, such as physiologically/pharmaceutically acceptable carriers and excipients. The pharmaceutical composition aims at promoting the administration to an organism, facilitating the absorption of the active ingredient and thereby exerting a biological effect.

The term "pharmaceutically acceptable carrier" refers to any inactive substance suitable for use in a formulation for the delivery of antibodies or antigen-binding fragments. A carrier can be an anti-adhesive agent, adhesive agent, coating agent, disintegrating agent, filler or diluent, preservative (such as antioxidant, antibacterial or antifungal agent), sweetener, absorption delaying agent, wetting agent, emulsifier, buffer, and the like. Examples of suitable pharmaceutically acceptable carriers include water, ethanol, polyol (such as glycerol, propylene glycol, polyethylene glycol, and the like) dextrose, vegetable oil (such as olive oil), saline, buffer, buffered saline, and isotonic agent, such as sugars, polyol, sorbitol and sodium chloride.

In addition, the present disclosure includes an agent for treating a disease related to a target antigen (for example, CEA), the agent comprises the anti-CEA antibody of the present disclosure as an active ingredient.

The CEA-related disease of the present disclosure is not limited, as long as it is a disease related to CEA. For example, the therapeutic response induced by the molecule of the present disclosure can be achieved by: binding to human CEA, and then repressing CEA from binding to its receptor/ligand, or by killing the tumor cells over-expressing CEA. Therefore, the molecules of the present disclosure, when comprised in formulations suitable for therapeutic applications, are very useful for such subjects who have tumors or cancers, preferably melanoma, colon cancer, breast cancer, lung cancer, gastric cancer, intestinal cancer, kidney cancer, non-small cell lung cancer, bladder cancer, etc.

In addition, the present disclosure relates to methods for immunodetection or determination of target antigens (for example, CEA), reagents for immunodetection or determination of target antigens (for example, CEA), methods for immunodetection or determination of cells expressing target antigens (for example, CEA), and the diagnostic agents for diagnosing diseases associated with target antigen (for example, CEA), comprising the antibody or antigen binding fragment of the present disclosure as an active ingredient that specifically recognizes and binds to the target antigen (for example, human CEA).

In the present disclosure, the method for detecting or measuring the amount of the target antigen (e.g., CEA) can be any known method. For example, it includes immunoassay or determination methods.

The immunoassay or determination method is a method of detecting or measuring the amount of antibody or antigen using a labeled antigen or antibody. Examples of the immunoassay or determination methods include radio-labelled immunoantibody method (RIA), enzyme immunoassay (EIA or ELISA), fluorescence immunoassay (FIA), luminescence immunoassay, western blotting, physicochemical method, and the like.

The above CEA-related diseases can be diagnosed by detecting or measuring the level of CEA using the antibodies of the present disclosure.

Cells expressing the polypeptide can be detected by the known immunodetection methods, preferably by immunoprecipitation, fluorescent cell staining, immunotissue staining, and the like. In addition, the method such as fluorescent antibody staining method with the FMAT8100HTS system (Applied Biosystem) can be used.

In the present disclosure, samples to be detected or measured for the presence of target antigen (e.g. CEA) are not particularly limited, as long as they are possible to contain target antigen (e.g. CEA), such as tissue cells, blood, plasma, serum, pancreatic juice, urine, faeces, tissue fluid or culture medium.

Dependent on the required diagnostic method, the diagnostic agent containing the antibody or antigen binding fragment thereof of the present disclosure can also contain reagents for performing an antigen-antibody reaction or reagents for detecting the reaction. The reagents for performing an antigen-antibody reaction include buffers, salts and the like. The reagents used for detection include agents commonly used in immunoassay or immunodetection methods, for example, a labelled secondary antibody that recognizes the monoclonal antibody, antigen binding fragment or conjugate thereof, and a substrate corresponding to the label.

The details in one or more embodiments of the present disclosure are set forth in the above specification. The preferred methods and materials are described below, although any method and material similar or identical to those described herein can be used in the practice or testing of the present disclosure. Through the specification and claims, other features, purposes and advantages of the present disclosure will become apparent. In the specification and claims, the singular forms include plural aspects unless the context clearly dictates otherwise. Unless otherwise defined explicitly herein, all technical and scientific terms used herein have the meaning commonly understood by those skilled in the art to which this disclosure pertains. All patents and publications cited in the specification are incorporated by reference. The following examples are presented to more fully illustrate the preferred embodiments of the present disclosure. These examples should not be construed as limiting the scope of the present disclosure in any way.

The following examples and test examples are provided to further describe the present disclosure, but are not intended to limit the scope of the disclosure. Experimental methods in the examples and test examples of the present disclosure (for which the specific conditions are not indicated) are generally carried out according to conventional conditions, such as Sambrook et al., Antibodies: Laboratory Manual, and Molecular Cloning, Cold Spring Harbor Laboratory; or according to the conditions recommended by the manufacturer; Reagents for which the sources are not specifically indicated are commercially available reagents.

### Example 1: Preparation of CEA recombinant protein and stably transfected cells

### 1. Sequences of the recombinant CEA antigen and CEA protein expressed on the cell surface

The sequences encoding human CEA protein with Fc or His tags were respectively cloned into mammalian cell expression vectors, and recombinant proteins were obtained after expression and purification in 293E cells for use in the tests of subsequent examples. The human CEA gene without tags, human CEACAM1 gene and monkey CEA gene were transfected into CHO cells to form a CHO cell line expressing CEA protein on the cell surface for subsequent antibody screening and identification. The amino acid sequences of the relative proteins are as follows:
1. Human CEA-his (hCEA-His) amino acid sequence (soluble human CEA antigen protein used in the test example): 
KLTIESTPFNVAEGKEVLLLVHNLPQHLFGYSWYKGERVDGNRQIIGYVIGTQQA
TPGPAYSGREIIYPNASLLIQNIIQNDTGFYTLHVIKSDLVNEEATGQFRVYPELPK
PSISSNNSKPVEDKDAVAFTCEPETQDATYLWWVNNQSLPVSPRLQLSNGNRTLT
LFNVTRNDTASYKCETQNPVSARRSDSVILNVLYGPDAPTISPLNTSYRSGENLN
LSCHAASNPPAQYSWFVNGTFQQSTQELFIPNITVNNSGSYTCQAHNSDTGLNR
TTVTTITVYAEPPKPFITSNNSNPVEDEDAVALTCEPEIQNTTYLWWVNNQSLPVS
PRLQLSNDNRTLTLLSVTRNDVGPYECGIQNELSVDHSDPVILNVLYGPDDPTISP
SYTYYRPGVNLSLSCHAASNPPAQYSWLIDGNIQQHTQELFISNITEKNSGLYTC
QANNSASGHSRTTVKTITVSAELPKPSISSNNSKPVEDKDAVAFTCEPEAQNTTY
LWWVNGQSLPVSPRLQLSNGNRTLTLFNVTRNDARAYVCGIQNSVSANRSDPV
TLDVLYGPDTPIISPPDSSYLSGANLNLSCHSASNPSPQYSWRINGIPQQHTQVLFI
AKITPNNNGTYACFVSNLATGRNNSIVKSITVSASGTSPGLSAHHHHHH
(SEQ ID NO: 1);
2. Human CEA-Fc (hCEA-Fc) amino acid sequence: 
KLTIESTPFNVAEGKEVLLLVHNLPQHLFGYSWYKGERVDGNRQIIGYVIGTQQA
TPGPAYSGREIIYPNASLLIQNIIQNDTGFYTLHVIKSDLVNEEATGQFRVYPELPK
PSISSNNSKPVEDKDAVAFTCEPETQDATYLWWVNNQSLPVSPRLQLSNGNRTLT
 
LFNVTRNDTASYKCETQNPVSARRSDSVILNVLYGPDAPTISPLNTSYRSGENLN
LSCHAASNPPAQYSWFVNGTFQQSTQELFIPNITVNNSGSYTCQAHNSDTGLNR
TTVTTITVYAEPPKPFITSNNSNPVEDEDAVALTCEPEIQNTTYLWWVNNQSLPVS
PRLQLSNDNRTLTLLSVTRNDVGPYECGIQNELSVDHSDPVILNVLYGPDDPTISP
SYTYYRPGVNLSLSCHAASNPPAQYSWLIDGNIQQHTQELFISNITEKNSGLYTC
QANNSASGHSRTTVKTITVSAELPKPSISSNNSKPVEDKDAVAFTCEPEAQNTTY
LWWVNGQSLPVSPRLQLSNGNRTLTLFNVTRNDARAYVCGIQNSVSANRSDPV
TLDVLYGPDTPIISPPDSSYLSGANLNLSCHSASNPSPQYSWRINGIPQQHTQVLFI
AKITPNNNGTYACFVSNLATGRNNSIVKSITVSASGTSPGLSAEPKSSDKTHTCPP
CPAPELLGGPSVFLFPPKPKDTLMISRTPEVTCVVVDVSHEDPEVKFNWYVDGVEVH
NAKTKPREEQYNSTYRVVSVLTVLHQDWLNGKEYKCKVSNKALPAPIEKTISKAKGQ
PREPQVYTLPPSRDELTKNQVSLTCLVKGFYPSDIAVEWESNGQPENNYKTTPPVLDS
DGSFFLYSKLTVDKSRWQQGNVFSCSVMHEALHNHYTQKSLSLSPGK
(SEQ ID NO: 2);
3. Monkey CEA-His (cynoCEA-His) amino acid sequence: 
QLTIESRPFNVAEGKEVLLLAHNVSQNLFGYIWYKGERVDASRRIGSCVIRTQQI
TPGPAHSGRETIDFNASLLIQNVTQSDTGSYTIQVIKEDLVNEEATGQFRVYPELP
KPYITSNNSNPIEDKDAVALTCEPETQDTTYLWWVNNQSLPVSPRLELSSDNRTL
TVFNIPRNDTTSYKCETQNPVSVRRSDPVTLNVLYGPDAPTISPLNTPYRAGEYL
NLTCHAASNPTAQYFWFVNGTFQQSTQELFIPNITVNNSGSYMCQAHNSATGLN
RTTVTAITVYAELPKPYITSNNSNPIEDKDAVTLTCEPETQDTTYLWWVNNQRLS
VSSRLELSNDNRTLTVFNIPRNDTTFYECETQNPVSVRRSDPVTLNVLYGPDAPTI
SPLNTPYRAGENLNLSCHAASNPAAQYFWFVNGTFQQSTQELFIPNITVNNSGS
YMCQAHNSATGLNRTTVTAITVYVELPKPYISSNNSNPIEDKDAVTLTCEPVAEN
TTYLWWVNNQSLSVSPRLQLSNGNRILTLLSVTRNDTGPYECGIQNSESAKRSD
PVTLNVTYGPDTPIISPPDLSYRSGANLNLSCHSDSNPSPQYSWLINGTLRQHTQ
VLFISKITSNNNGAYACFVSNLATGRNNSIVKNISVSSGDSAPGSSGLSAHHHHH
H (SEQ ID NO: 3);
4. The amino acid sequence of human CEA (hCEA-CHO) expressed on the surface of CHO cells: 
MESPSAPPHRWCIPWQRLLLTASLLTFWNPPTTAKLTIESTPFNVAEGKEVLLLVH
NLPQHLFGYSWYKGERVDGNRQIIGYVIGTQQATPGPAYSGREIIYPNASLLIQNI
IQNDTGFYTLHVIKSDLVNEEATGQFRVYPELPKPSISSNNSKPVEDKDAVAFTCE
 
PETQDATYLWWVNNQSLPVSPRLQLSNGNRTLTLFNVTRNDTASYKCETQNPV
SARRSDSVILNVLYGPDAPTISPLNTSYRSGENLNLSCHAASNPPAQYSWFVNGT
FQQSTQELFIPNITVNNSGSYTCQAHNSDTGLNRTTVTTITVYAEPPKPFITSNNS
NPVEDEDAVALTCEPEIQNTTYLWWVNNQSLPVSPRLQLSNDNRTLTLLSVTRN
DVGPYECGIQNELSVDHSDPVILNVLYGPDDPTISPSYTYYRPGVNLSLSCHAAS
NPPAQYSWLIDGNIQQHTQELFISNITEKNSGLYTCQANNSASGHSRTTVKTITVS
AELPKPSISSNNSKPVEDKDAVAFTCEPEAQNTTYLWWVNGQSLPVSPRLQLSN
GNRTLTLFNVTRNDARAYVCGIQNSVSANRSDPVTLDVLYGPDTPIISPPDSSYLS
GANLNLSCHSASNPSPQYSWRINGIPQQHTQVLFIAKITPNNNGTYACFVSNLAT
GRNNSIVKSITVSASGTSPGLSAGATVGIMIGVLVGVALI
(SEQ ID NO: 4);
5. The amino acid sequence of monkey CEA (cynoCEA-CHO) expressed on the surface of CHO cells: 
MEFGLSWLFLVAILKGVQCQLTIESRPFNVAEGKEVLLLAHNVSQNLFGYIWYK
GERVDASRRIGSCVIRTQQITPGPAHSGRETIDFNASLLIQNVTQSDTGSYTIQVIK
EDLVNEEATGQFRVYPELPKPYITSNNSNPIEDKDAVALTCEPETQDTTYLWWVN
NQSLPVSPRLELSSDNRTLTVFNIPRNDTTSYKCETQNPVSVRRSDPVTLNVLYG
PDAPTISPLNTPYRAGEYLNLTCHAASNPTAQYFWFVNGTFQQSTQELFIPNITV
NNSGSYMCQAHNSATGLNRTTVTAITVYAELPKPYITSNNSNPIEDKDAVTLTCE
PETQDTTYLWWVNNQRLSVSSRLELSNDNRTLTVFNIPRNDTTFYECETQNPVS
VRRSDPVTLNVLYGPDAPTISPLNTPYRAGENLNLSCHAASNPAAQYFWFVNGT
FQQSTQELFIPNITVNNSGSYMCQAHNSATGLNRTTVTAITVYVELPKPYISSNNS
NPIEDKDAVTLTCEPVAENTTYLWWVNNQSLSVSPRLQLSNGNRILTLLSVTRND
TGPYECGIQNSESAKRSDPVTLNVTYGPDTPIISPPDLSYRSGANLNLSCHSDSNP
SPQYSWLINGTLRQHTQVLFISKITSNNNGAYACFVSNLATGRNNSIVKNISVSSG
DSAPGSSGLSARATVGIIIGMLVGVALM (SEQ ID NO: 5);
6. The amino acid sequence of human CEACAM1 expressed on the surface of CHO cells (CEACAM1-CHO):

MGHLSAPLHRVRVPWQGLLLTASLLTFWNPPTTAQLTTESMPFNVAEGKEVLLL
VHNLPQQLFGYSWYKGERVDGNRQIVGYAIGTQQATPGPANSGRETIYPNASLL
IQNVTQNDTGFYTLQVIKSDLVNEEATGQFHVYPELPKPSISSNNSNPVEDKDAV
AFTCEPETQDTTYLWWINNQSLPVSPRLQLSNGNRTLTLLSVTRNDTGPYECEIQ
NPVSANRSDPVTLNVTYGPDTPTISPSDTYYRPGANLSLSCYAASNPPAQYSWLI

NGTFQQSTQELFIPNITVNNSGSYTCHANNSVTGCNRTTVKTIIVTELSPVVAKPQ
IKASKTTVTGDKDSVNLTCSTNDTGISIRWFFKNQSLPSSERMKLSQGNTTLSINP
VKREDAGTYWCEVFNPISKNQSDPIMLNVNYNALPQENGLSPGAIAGIVIGVVA
LVALIAVALACFLHFGKTGRASDQRDLTEHKPSVSNHTQDHSNDPPNKMNEVTY
STLNFEAQQPTQPTSASPSLTATEIIYSEVKKQ (SEQ ID NO: 6).

### 2. Purification of relative proteins

### 1) Purification of His-tagged protein

The cell expression supernatant sample was centrifuged at high speed to remove impurities. The nickel column was equilibrated with PBS buffer (pH 7.4), washed by using 2-5 column volumes, and the supernatant sample was loaded onto the Ni Sepharose excel column at a certain flow rate. The column was washed with PBS buffer until the A₂₈₀ reading dropped to the baseline, then the column was washed with PBS+10mM imidazole to remove non-specifically bound impurity proteins, and the effluent was collected. Finally, the target protein was eluted with PBS solution containing 300mM imidazole, and the elution peaks were collected. After the collected eluate was concentrated, the sample buffer was replaced with a PBS solution using a desalting column for use in the subsequent experiments.

### 2) Purification of Fc-containing protein, chimeric antibody and hybridoma antibody

The cell expression supernatant sample was centrifuged at high speed to remove impurities, the expression supernatant of Fc-containing recombinant protein and the expression supernatant of chimeric antibody were purified by Protein A column, and the expression supernatant of hybridoma was purified by Protein G column. The supernatant was loaded onto the column at a certain flow rate. The column was washed with PBS until the A280 reading dropped to the baseline. The target protein was eluted with 100 mM acetic acid, pH 3.0, and neutralized with 1 M Tris-HCl, pH 8.0. The eluted sample was concentrated and the buffer was replaced with PBS, and then aliquoted for use.

### Example 2: Preparation of mouse anti-human CEA monoclonal antibody

### 1. Immunization and fusion

For mouse immunization, hCEA-His protein and cyno-CEA-His protein, or hCEA-CHO cells and cynoCEA-CHO cells were used for cross-immunization. The dosage for protein immunization was 50 µg for the first immunization, 25 µg for the subsequent immunizations, and the dosage for cellular immunization was 10⁷ cells per immunization, once every two weeks. After 3 immunizations, blood was taken to determine the titer of the antibody in the serum. The mice that exhibited high antibody titer in the serum (wherein the titer has a tend reaching to a plateau) were selected for spleen cell fusion, and hybridoma cells were obtained by fusing splenic lymphocytes with myeloma Sp2/0 cells (ATCC^{®} CRL-8287^{™}) by using a PEG-mediated fusion procedure. The fused hybridoma cells were resuspended in MC semi-solid complete medium (RPMI-1640 medium comprising 20% FBS, 1×HAT, 1×OPI and 2% methylcellulose) at a density of 0.5×10⁶ to 1×10⁶ cells/ml, aliquoted into a 35 mm cell culture dishes, and incubated at 37°C, 5% CO₂ for 7-9 days. 7-9 days after fusion, single cell clones were selected, according to the size of cell clones, and added into a 96-well cell culture plate supplemented with 200 µl/well HT complete medium (RPMI-1640 medium comprising 20% FBS, 1×HT and 1×OPI), and incubated at 37°C, 5% CO₂ for 3 days for testing.

### 2. Screening of Hybridoma cells

The preliminary screening of antibody was carried out by Enzyme-Linked Immunosorbent Assay (ELISA) using cell surface antigens. Cells were plated on an Elisa plate (Corning, Cat# 3599) and incubated overnight in a 37°C incubator. When the cells were completely attached to the wall and about to overgrow the whole well, the supernatant was removed, the cells were washed once with PBS; the cell fixative (Beyotime, Cat# P0098) was added, and placed at room temperature for 45 minutes, and the fixative was removed. The plate was washed with a plate washer for 3 times, and 5% skimmed milk powder was added for blocking at 37°C for more than 3 hours. The blocking solution was removed and the plate was washed with the plate washer for 3 times. After blocking, the cell plate was stored at -20°C or was subjected to immediate use. A gradient of diluted hybridoma cell culture supernatant was added, incubated at 37°C for 1 h, washed with the plate washer for 3 times, added with 100 µl 10,000-fold diluted goat anti-mouse IgG H&L (HRP) secondary antibody (Abcam, Cat# ab205719 ), incubated at 37°C for 1 h, washed with the plate washer for 3 times, added with 100 µl TMB (KPL, Cat# 5120-0077) and placed at 37°C for 10 min for color development, 100 µl 1 M sulfuric acid was added to stop the reaction, and the absorbance at 450 nm wa read with a microplate reader. If the test antibody binds to CEA on the cell surface without being interfered by competition caused by soluble CEA (sCEA), the antibody will be incubated with sCEA for 30 min before being added into the cell plate.

The screened positive clones were expanded for cryopreservation, and sub-cloned for two to three time until a single-cell clone was obtained. The selected hybridoma clones were further prepared by using a serum-free cell culture method, and then purified. The obtained hybridoma antibodies were used for detecting the binding of the antibody to cell surface CEA protein, by using a flow cytometer (the method refers to Test Example 1 of the present disclosure), and hybridoma cell lines with favorable binding activity were selected. Among them, the test results of binding activity of monoclonal hybridoma cell lines mAb47, mAb63, mAb67 and mAb103 are shown in Table 1.

**Table 1: Experimental results showing the binding of murine antibody to cell surface CEA protein**

| Antibody | EC50 (nM) | | |
|---|---|---|---|
| | MKN45 | cynoCEA-CHO | CEACAM1-CHO |
| mAb47 | 2.10 | 1.66 | No binding |
| mAb63 | 3.62 | 2.88 | No binding |
| mAb67 | 1.90 | 6.00 | No binding |
| mAb103 | 3.94 | 0.71 | No binding |

### 3 Sequencing of the hybridoma antibody clone

The monoclonal hybridoma cell lines mAb47, mAb63, mAb67 and mAb103 were selected to clone the sequence of the monoclonal antibody. The process was as follows: hybridoma cells at logarithmic growth phase were collected, RNAs were extracted with Trizol (Invitrogen, Cat# 15596-018), and were reverse-transcribed into cDNAs. cDNAs were used as templates to perform PCR amplification, the product was subjected to sequencing. The antibody amino acid sequences corresponding to the obtained DNA sequences are shown in Table 2 below.

**Table 2: Variable region sequences of murine anti-CEA antibody**

| Antibody name | Heavy chain variable region sequence VH | Light chain variable region sequence VL |
|---|---|---|
| mAb47 | QIQLVQSGPELKKPGETVKISCK ASGYTFTTYGMSWVKQAPGKG LKWMGWINTYSGVPTYADDFK GRFAFSLETSASTAYLQINNLKNE DTATYFCARRGNY GRWDFDVW GTGTTVTVSS (SEQ ID NO: 7) | EIVLTQSPALMAASPGEKVTITC SVSSTISSSNLHWYQQKSETSP KPWIYGTSNLASGVPVRFSGSG SGTSYSLTISSMEAEDAATYYC QQWSIYPLTFGAGTKLELK (SEQ ID NO: 8) |
| mAb63 | EVQLQQSGPELVKPGASVKMSC KASGYTFTDFYMNWVRQSHGK SLEWIGDIFPKNGNTDYNRKFKD KATLTVDKSSNTVYMEFRSLTLE DSAIYFCARSGYGNYVFDYWGQ GTIFTVSS (SEQ ID NO: 9) | DVQMTQTPSALSASLGDRVTIS CRTSQDINIYLNWYQQKPDGT VKLLIYYRSGLLSGVPSRFSGS GSGTDYSLTISNLEPEDIATYFC QQGNTLPPTFGGGTKLEIK (SEQ ID NO: 10) |
| mAb67 | EVQLQQSGPELVKPGASVKIYCK ASGYTFTDYHMNWVKQSHGKS LEWIGDINPDIGGTSYNQNFKGK ATLTVDKSSSTAYMELRSLTSEDS AVYYCSRWDFDSFANWGQGTLV TVSA (SEQ ID NO: 11) | DIVMTQSPASLAMSLGKRATIS CRASESVSIIGTNLIHWYQQKP GQPPKLLIYHASNLETGVPARF SGSGSGADFTLTIDPVEGDDVA LYYCLQSRKIPYTFGGGTKMEI K (SEQ ID NO: 12) |
| mAb 103 | QIQLVQSGPELKKPGETVKISCK ASGYTFTTYGVIWVKQAPGKGL KWMGWINTYSGVPTYADDFKG RFAFSLETSASTAFLQINNLKNED TATYFCARKKTLTTVTPWFAYW GQGTLVTVSA (SEQ ID NO: 13) | DLVVTQSSSASFSLGASAKLTC TLSSQHSTYTIEWYQQQPLKPP KYVMELKKDGSHSTGDGIPDR FSGSSSGADRYLTISNIQPEDEAI YICGVGNTIKEQFVYVFGGGTK VTVL (SEQ ID NO: 14) |

**Table 3: CDR sequence of anti-CEA antibody**

| Antibody name | Heavy chain CDR sequence | | Light chain CDR sequence | |
|---|---|---|---|---|
| mAb47 | HCDR1 | TYGMS (SEQ ID NO: 15) | LCDR1 | SVSSTISSSNLH (SEQ ID NO: 18) |
| | HCDR2 | WINTYSGVPTYADDFKG (SEQ ID NO: 16) | LCDR2 | GTSNLAS (SEQ ID NO: 19) |
| | HCDR3 | RGNYGRWDFDV (SEQ ID NO: 17) | LCDR3 | QQWSIYPLT (SEQ ID NO: 20) |
| mAb63 | HCDR1 | DFYMN (SEQ ID NO: 21) | LCDR1 | RTSQDINIYLN (SEQ ID NO: 24) |
| | HCDR2 | DIFPKNGNTDYNRKFKD (SEQ ID NO: 22) | LCDR2 | YRSGLLS (SEQ ID NO: 25) |
| | HCDR3 | SGYGNYVFDY (SEQ ID NO: 23) | LCDR3 | QQGNTLPPT (SEQ ID NO: 26) |
| mAb67 | HCDR1 | DYHMN (SEQ ID NO: 27) | LCDR1 | RASESVSIIGTNLIH (SEQ ID NO: 30) |
| | HCDR2 | DINPDIGGTSYNQNFKG (SEQ ID NO: 28) | LCDR2 | HASNLET (SEQ ID NO: 31) |
| | HCDR3 | WDFDSFAN (SEQ ID NO: 29) | LCDR3 | LQSRKIPYT (SEQ ID NO: 32) |
| mAb 103 | HCDR1 | TYGVI (SEQ ID NO: 33) | LCDR1 | TLSSQHSTYTIE (SEQ ID NO: 35) |
| | HCDR2 | WINTYSGVPTYADDFKG (SEQ ID NO: 16) | LCDR2 | LKKDGSHSTGD (SEQ ID NO: 36) |
| | HCDR3 | KKTLTTVTPWFAY (SEQ ID NO: 34) | LCDR3 | GVGNTIKEQFVYV (SEQ ID NO: 37) |

| | | | | |
|---|---|---|---|---|
| Notes: The antibody CDR sequences in the table were determined according to the Kabat numbering criteria. | | | | |

### 4. Preparation of human IgG1 chimeric antibody

The candidate molecules mAb47, mAb63, mAb67 and mAb103 were amplified and sequenced to obtain the gene sequences encoding the variable regions. Forward and reverse primers were designed on the basis of the sequences obtained by sequencing, the gene to be sequenced was used as a template to construct the VH/VK gene fragment for each antibody via PCR, and then inserted into the expression vector pHr (with a signal peptide and hIgG1/hkappa/hlambda constant region gene (CH1-Fc/CL) fragment) via homologous recombination to construct an expression plasmid for the full-length of recombinant chimeric antibody VH-CH1-Fc-pHr/VL-CL-pHr, resulting in their chimeric antibodies Ch47, Ch63, Ch67 and Ch103.

### Example 3: Humanization of murine anti-human CEA monoclonal antibody

The heavy and light chain variable region germline genes having high homology were selected as templates by aligning against IMGT database of human antibody heavy and light chain variable germline gene using MOE software. The murine antibody CDRs were grafted onto each of the corresponding human templates to form variable region sequence in the order of FR1-CDR1-FR2-CDR2-FR3-CDR3-FR4. The CDR amino acid residues of the antibodies exemplified below were determined and annotated by the Kabat numbering criteria.

### 1. Humanization of murine antibody mAb47

The heavy and light chain variable region germline genes with high homology were selected as templates, for example, for murine antibody mAb47, IGKV6-21^{∗}01 and IGKJ2^{∗}01 were selected as the humanized light chain templates, and IGHV7-4-1^{∗}02 and IGHJ6^{∗}01 were selected as the humanized heavy chain templates. The murine antibody mAb47 CDRs were grafted onto the corresponding human template for humanization. The back mutation(s) designed for the humanized murine antibody mAb47 are shown in Table 4 below:

**Table 4: Humanized design of murine antibody mAb47**

| Light chain variable region for the humanized antibody of mAb47 | | Heavy chain variable region for the humanized antibody of mAb47 | |
|---|---|---|---|
| h47VL1 | Graft (IGKV6-21 ^{∗}01 +IGKJ2^{∗}01) | h47VH1 | Graft (IGHV7-4-1^{∗}02+IGHJ6^{∗}01) |
| h47VL2 | Graft (IGKV6-21^{∗}01+IGKJ2^{∗}01)+L46P, K49Y, F71Y | h47VH2 | Graft (IGHV7-4-1^{∗}02+IGHJ6^{∗}01) +E46K |
| h47VL3 | Graft (IGKV6-21^{∗}01+IGKJ2^{∗}01)+L46P, L47W, K49Y, F71Y | h47VH3 | Graft (IGHV7-4-1^{∗}02+IGHJ6^{∗}01)+R38K, E46K |
| h47VL4 | Graft (IGKV6-21^{∗}01+IGKJ2^{∗}01)+L46P, L47W, K49Y, D70S, F71Y | | |

| | | | |
|---|---|---|---|
| Notes: The amino acid positions in the table are numbered according to Kabat numbering criteria, for example, "L46P" refers to that the L at position 46 according to Kabat numbering criteria is mutated back to P. "Graft" represents that the murine antibody CDRs are implanted into the human germline FR region sequences. | | | |

In addition, D61S mutation was further introduced in the heavy chain h47VH3 (that is, amino acid mutation was performed on the antibody HCDR2, so that the antibody HCDR2 sequence was changed from WINTYSGVPTYADDFKG (SEQ ID NO: 16) to WINTYSGVPTYASDFKG (SEQ ID NO: 38)), the antibody still had favorable activity.

The specific sequences of the murine antibody mAb47 after humanization are shown in Table 5:

**Table 5: Variable region sequences of the humanized murine antibody mAb47**

| Variable region number | Antibody variable region sequences of the humanized mAb47 |
|---|---|
| h47VH1 | EVQLVQSGSELKKPGASVKVSCKASGYTFTTYGMSWVRQAPGQ GLEWMGWINTYSGVPTYADDFKGRFVFSLDTSVSTAYLQISSLKA EDTAVYYCARRGNYGRWDFDVWGQGTTVTVSS (SEQ ID NO: 39) |
| h47VH2 | EVQLVQSGSELKKPGASVKVSCKASGYTFTTYGMSWVRQAPGQ GLKWMGWINTYSGVPTYADDFKGRFVFSLDTSVSTAYLQISSLKA EDTAVYYCARRGNYGRWDFDVWGQGTTVTVSS (SEQ ID NO: 40) |
| h47VH3 | EVQLVQSGSELKKPGASVKVSCKASGYTFTTYGMSWVKQAPGQ GLKWMGWINTYSGVPTYADDFKGRFVFSLDTSVSTAYLQISSLKA EDTAVYYCARRGNYGRWDFDVWGQGTTVTVSS (SEQ ID NO: 41) |
| h47VH4 | EVQLVQSGSELKKPGASVKVSCKASGYTFTTYGMSWVKQAPGQ GLKWMGWINTYSGVPTYASDFKGRFVFSLDTSVSTAYLQISSLKA EDTAVYYCARRGNYGRWDFDVWGQGTTVTVSS (SEQ ID NO: 42) |
| h47VL1 | EIVLTQSPDFQSVTPKEKVTITCSVSSTISSSNLHWYQQKPDQSPKL LIKGTSNLASGVPSRFSGSGSGTDFTLTINSLEAEDAATYYCQQWS IYPLTFGQGTKLEIK (SEQ ID NO: 43) |
| h47VL2 | EIVLTQSPDFQSVTPKEKVTITCSVSSTISSSNLHWYQQKPDQSPKP LIYGTSNLASGVPSRFSGSGSGTDYTLTINSLEAEDAATYYCQQWS IYPLTFGQGTKLEIK (SEQ ID NO: 44) |
| h47VL3 | EIVLTQSPDFQSVTPKEKVTITCSVSSTISSSNLHWYQQKPDQSPKP WIYGTSNLASGVPSRFSGSGSGTDYTLTINSLEAEDAATYYCQQW SIYPLTFGQGTKLEIK (SEQ ID NO: 45) |
| h47VL4 | EIVLTQSPDFQSVTPKEKVTITCSVSSTISSSNLHWYQQKPDQSPKP WIYGTSNLASGVPSRFSGSGSGTSYTLTINSLEAEDAATYYCQQWS IYPLTFGQGTKLEIK (SEQ ID NO: 46) |

| | |
|---|---|
| Notes: In the table, the underlined portion represents the CDR regions (determined according to the Kabat numbering criteria), and the bold italic portion represents the mutation sites. | |

### 2. Humanization of murine antibody mAb63

The heavy and light chain variable region germline genes with high homology were selected as templates, for example, for murine antibody mAb63, IGKV1-39^{∗}01 and IGKJ4^{∗}01 were selected as the humanized light chain templates, and IGHV1-46^{∗}01 and IGHJ1^{∗}01 were selected as the humanized heavy chain templates. The murine antibody mAb63 CDRs were grafted onto the corresponding human template for humanization. The back mutation(s) designed for the humanized murine antibody mAb63 are shown in Table 6 below:

**Table 6: Humanized design of murine antibody mAb63**

| Light chain variable region for the humanized antibody of mAb63 | | Heavy chain variable region for the humanized antibody of mAb63 | |
|---|---|---|---|
| h63VL1 | Graft (IGKV1-39^{∗}01+IGKJ4^{∗}01) | h63VH1 | Graft (IGHV1-46^{∗}01+IGHJ1^{∗}01) |
| h63VL2 | Graft (IGKV1-39^{∗}01+IGKJ4^{∗}01)+I2V, F71Y | h63VH2 | Graft (IGHV1-46^{∗}01+IGHJI^{∗}01)+M69L, R71V, T73K |
| h63VL3 | Graft (IGKV1-39^{∗}01+IGKJ4^{∗}01)+I2V, K42G, P44V, F71Y | h63VH3 | Graft (IGHV1-46^{∗}01+IGHJ1^{∗}01)+M48I, V67A, M69L, R71V, T73K, L82F |
| | | h63VH4 | Graft (IGHV1-46^{∗}01+IGHJ1^{∗}01)+M48I, R66K, V67A, M69L, R71V, T73K, L82F, S 82A R |

| | | | |
|---|---|---|---|
| Notes: The amino acid positions in the table are numbered according to Kabat numbering criteria, for example, "S 82A R" refers to that the S at position 82A according to Kabat numbering criteria is mutated back to R. "Graft" represents that the murine antibody CDRs are implanted into the human germline FR region sequences. | | | |

In addition, a N54S mutation was further introduced in the heavy chain h63VH1 (that is, amino acid mutation was performed on the antibody HCDR2, so that the antibody HCDR2 sequence was changed from DIFPKNGNTDYNRKFKD (SEQ ID NO: 22) to DIFPKSGNTDYNRKFKD (SEQ ID NO: 47)), and the antibody still had favorable activity.

The specific sequences of the humanizd and mutated murine antibody mAb63 are shown in Table 7:

**Table 7: Variable region sequences of the humanized murine antibody mAb63**

| Variable region number | Antibody variable region sequences of the humanized mAb63 |
|---|---|
| h63VH1 | EVQLVQSGAEVKKPGASVKVSCKASGYTFTDFYMNWVRQAPGQGLE WMGDIFPKNGNTDYNRKFKDRVTMTRDTSTSTVYMELSSLRSEDTAVY YCARSGYGNYVFDYWGQGTLVTVSS (SEQ ID NO: 48) |
| h63VH2 | EVQLVQSGAEVKKPGASVKVSCKASGYTFTDFYMNWVRQAPGQGLE WMGDIFPKNGNTDYNRKFKDRVTLTVDKSTSTVYMELSSLRSEDTAVY YCARSGYGNYVFDYWGQGTLVTVSS (SEQ ID NO: 49) |
| h63VH3 | EVQLVQSGAEVKKPGASVKVSCKASGYTFTDFYMNWVRQAPGQGLE WIGDIFPKNGNTDYNRKFKDRATLTVDKSTSTVYMEFSSLRSEDTAVYY CARSGYGNYVFDYWGQGTLVTVSS (SEQ ID NO: 50) |
| h63VH4 | EVQLVQSGAEVKKPGASVKVSCKASGYTFTDFYMNWVRQAPGQGLE WIGDIFPKNGNTDYNRKFKDKATLTVDKSTSTVYMEFRSLRSEDTAVYY CARSGYGNYVFDYWGQGTLVTVSS (SEQ ID NO: 51) |
| h63VH5 | EVQLVQSGAEVKKPGASVKVSCKASGYTFTDFYMNWVRQAPGQGLE WMGDIFPKSGNTDYNRKFKDRVTMTRDTSTSTVYMELSSLRSEDTAVY YCARSGYGNYVFDYWGQGTLVTVSS (SEQ ID NO: 52) |
| h63VL1 | DIQMTQSPSSLSASVGDRVTITCRTSQDINIYLNWYQQKPGKAPKLLIYY RSGLLSGVPSRFSGSGSGTDFTLTISSLQPEDFATYYCQQGNTLPPTFGGG TKVEIK    (SEQ ID NO: 53) |
| h63VL2 | DVQMTQSPSSLSASVGDRVTITCRTSQDINIYLNWYQQKPGKAPKLLIY YRSGLLSGVPSRFSGSGSGTDYTLTISSLQPEDFATYYCQQGNTLPPTFGG GTKVEIK (SEQ ID NO: 54) |
| h63V L3 | DVQMTQSPSSLSASVGDRVTITCRTSQDINIYLNWYQQKPGGAVKLLIY YRSGLLSGVPSRFSGSGSGTDYTLTISSLQPEDFATYYCQQGNTLPPTFGG GTKVEIK (SEQ ID NO: 55) |

| | |
|---|---|
| Notes: In the table, the underlined portion represents the CDR regions (corresponding to the Kabat numbering criteria), and the bold italic portion represents the mutation sites. | |

### 3. Humanization of murine antibody mAb67

The heavy and light chain variable region germline genes with high homology were selected as templates, for example, for murine antibody mAb67, IGKV4-1^{∗}01 and IGKJ4^{∗}01, IGKV3-15^{∗}01 and IGKJ4^{∗}01, or IGKV1-39^{∗}01 and IGKJ4^{∗}01 were selected as the humanized light chain templates, and IGHV1-3^{∗}01 and IGHJ1^{∗}01, or IGHV5-51^{∗}01 and IGHJ1^{∗}01 were selected as the humanized heavy chain templates. The murine antibody mAb67 CDRs were grafted onto the corresponding human template for humanization. The back mutation(s) designed for the humanized murine antibody mAb67 are shown in Table 8 below:

**Table 8: Humanized design of murine antibody mAb67**

| Light chain variable region for the humanized antibody of mAb67 | | Heavy chain variable region for the humanized antibody of mAb67 | |
|---|---|---|---|
| h67VL1 | Graft (IGKV4-1^{∗}01+ IGKJ4^{∗}01 ) | h67VH1 | Graft (IGHV1-3^{∗}01 +IGHJ1^{∗}01) |
| h67VL2 | Graft (IGKV3-15^{∗}01+ IGKJ4^{∗}01 ) | h67VH2 | Graft (IGHV5-51^{∗}01+IGHJ1^{∗}01) |
| h67VL3 | Graft (IGKV3-15^{∗}01+ IGKJ4^{∗}01)+A43P, I58V | h67VH3 | Graft (IGHV5-51^{∗}01+IGHJ1^{∗}01)+ R38K, Q66K, A71V |
| h67VL4 | Graft (IGKV1-39^{∗}01+ IGKJ4^{∗}01 ) | | |
| h67VL5 | Graft (IGKV1-39^{∗}01+ IGKJ4^{∗}01) +Q3V, A43P | | |

| | | | |
|---|---|---|---|
| Notes: The amino acid positions in the table are numbered according to Kabat numbering criteria, for example, "A43P" refers to that the A at position 43 according to Kabat numbering criteria is mutated back to P. "Graft" represents that the murine antibody CDRs are implanted into the human germline FR region sequences. | | | |

The specific sequences of the murine antibody mAb67 after humanization are shown in Table 9:

**Table 9: Variable region sequences of the humanized murine antibody mAb67**

| Variable region number | Antibody variable region sequences of the humanized mAb67: |
|---|---|
| h67VH1 | EVQLVQSGAEVKKPGASVKVSCKASGYTFTDYHMNWVRQAPG QRLEWMGDINPDIGGTSYNQNFKGRVTITRDTSASTAYMELSSLR SEDTAVYYCARWDFDSFANWGQGTLVTVSS (SEQ ID NO: 56) |
| h67VH2 | EVQLVQSGAEVKKPGESLKISCKGSGYSFTDYHMNWVRQMPGK GLEWMGDINPDIGGTSYNQNFKGQVTISADKSISTAYLQWSSLKA SDTAMYYCARWDFDSFANWGQGTLVTVSS (SEQ ID NO: 57) |
| h67VH3 | EVQLVQSGAEVKKPGESLKISCKGSGYSFTDYHMNWVKQMPGK GLEWMGDINPDIGGTSYNQNFKGKVTISVDKSISTAYLQWSSLKA SDTAMYYCARWDFDSFANWGQGTLVTVSS (SEQ ID NO: 58) |
| h67VL1 | DIVMTQSPDSLAVSLGERATINCRASESVSIIGTNLIHWYQQKPGQ PPKLLIYHASNLETGVPDRFSGSGSGTDFTLTISSLQAEDVAVYYC LQSRKIPYTFGGGTKVEIK (SEQ ID NO: 59) |
| h67VL2 | EIVMTQSPATLSVSPGERATLSCRASESVSIIGTNLIHWYQQKPGQ APRLLIYHASNLETGIPARFSGSGSGTEFTLTISSLQSEDFAVYYCL QSRKIPYTFGGGTKVEIK (SEQ ID NO: 60) |
| h67VL3 | EIVMTQSPATLSVSPGERATLSCRASESVSIIGTNLIHWYQQKPGQP PRLLIYHASNLETGVPARFSGSGSGTEFTLTISSLQSEDFAVYYCLQ SRKIPYTFGGGTKVEIK (SEQ ID NO: 61) |
| h67VL4 | DIQMTQSPSSLSASVGDRVTITCRASESVSIIGTNLIHWYQQKPGK APKLLIYHASNLETGVPSRFSGSGSGTDFTLTISSLQPEDFATYYCL QSRKIPYTFGGGTKVEIK (SEQ ID NO: 62) |
| h67VL5 | DIVMTQSPSSLSASVGDRVTITCRASESVSIIGTNLIHWYQQKPGK PPKLLIYHASNLETGVPSRFSGSGSGTDFTLTISSLQPEDFATYYCL QSRKIPYTFGGGTKVEIK (SEQ ID NO: 63) |

| | |
|---|---|
| Notes: In the table, the underlined portion represents the CDR regions (determined according to the Kabat numbering criteria), and the bold italic portion represents the mutation sites. | |

### 4. Humanization of murine antibody mAbl03

The heavy and light chain variable region germline genes with high homology were selected as templates, for example, for murine antibody mAb103, IGLV4-69^{∗}01 and IGLJ2^{∗}01 were selected as the humanized light chain templates, and IGHV7-4-1^{∗}02 and IGHJ1^{∗}01 were selected as the humanized heavy chain templates. The murine antibody mAb103 CDRs were grafted onto the corresponding human template for humanization. The back mutation(s) designed for the humanized murine antibody mAb103 are shown in Table 10 below:

**Table 10: Humanized design of murine antibody mAb103**

| Light chain variable region for the humanized antibody of mAb103 | | Heavy chain variable region for the humanized antibody of mAb103 | |
|---|---|---|---|
| h103VL1 | Graft (IGLV4-69^{∗}01+IGLJ2^{∗}01) + K49E | h103VH1 | Graft (IGHV7-4-1^{∗}02+IGHJ1^{∗}01) |
| h103VL2 | Graft (IGLV4-69^{∗}01+IGLJ2^{∗}01)+H36Y, K49E | h103VH2 | Graft (IGHV7-4-1^{∗}02+IGHJ1^{∗}01)+R38K, E46K |
| h103VL3 | Graft (IGLV4-69^{∗}01+IGLJ2^{∗}01)+H36Y, L47V, K49E | h103VH3 | Graft (IGHV7-4-1^{∗}02+IGHJ1^{∗}01)+V2I, R38K, E46K |
| h103VL4 | Graft (IGLV4-69^{∗}01+IGLJ2^{∗}01)+L4V, H36Y, L47V, K49E | | |
| h103VL5 | Graft (IGLV4-69^{∗}01+IGLJ2^{∗}01)+H36Y, G43P, L47V, K49E, Y87I | | |
| h103VL6 | Graft (IGLV4-69^{∗}01+IGLJ2^{∗}01)+L4V, H36Y, G43P, L47V, K49E, Y87I | | |
| h103VL7 | Graft (IGLV4-69^{∗}01+IGLJ2^{∗}01)+L4V, H36Y, G43P, L47V, K49E, E70D, Y87I | | |

| | | | |
|---|---|---|---|
| Notes: The amino acid positions in the table are numbered according to Kabat numbering criteria, for example, "K49E" refers to that the K at position 49 according to Kabat numbering criteria is mutated back to E. "Graft" represents that the murine antibody CDRs are implanted into the human germline FR region sequences. | | | |

In addition, a D61S mutation was further introduced in the heavy chain h103VH1 (that is, amino acid mutation was performed on the antibody HCDR2, so that the antibody HCDR2 sequence was changed from WINTYSGVPTYADDFKG (SEQ ID NO: 16) to WINTYSGVPTYASDFKG (SEQ ID NO: 38)), and a D56E mutation was introduced in the light chain h103VL3 (that is, amino acid mutation was performed on the antibody LCDR2, so that the antibody LCDR2 sequence was changed from LKKDGSHSTGD (SEQ ID NO: 36) to LKKDGSHSTGE (SEQ ID NO: 64) and the antibody still had favorable activity.

The specific sequences of the murine antibody mAb103 after humanization are shown in Table 11:

**Table 11: Variable region sequences of the humanized murine antibody mAb 103**

| Variable region | Antibody variable region sequences of the humanized mAb103 |
|---|---|
| h103VH1 | EVQLVQSGSELKKPGASVKVSCKASGYTFTTYGVIWVRQAPGQ GLEWMGWINTYSGVPTYADDFKGRFVFSLDTSVSTAYLQISSLK AEDTAVYYCARKKTLTTVTPWFAYWGQGTLVTVSS (SEQ ID NO: 65) |
| h103VH2 | EVQLVQSGSELKKPGASVKVSCKASGYTFTTYGVIWVKQAPGQ GLKWMGWINTYSGVPTYADDFKGRFVFSLDTSVSTAYLQISSLK |
| | AEDTAVYYCARKKTLTTVTPWFAYWGQGTLVTVSS (SEQ ID NO: 66) |
| h103VH3 | EIQLVQSGSELKKPGASVKVSCKASGYTFTTYGVIWVKQAPGQ GLKWMGWINTYSGVPTYADDFKGRFVFSLDTSVSTAYLQISSLK AEDTAVYYCARKKTLTTVTPWFAYWGQGTLVTVSS (SEQ ID NO: 67) |
| h103VH4 | EVQLVQSGSELKKPGASVKVSCKASGYTFTTYGVIWVRQAPGQ GLEWMGWINTYSGVPTYASDFKGRFVFSLDTSVSTAYLQISSLK AEDTAVYYCARKKTLTTVTPWFAYWGQGTLVTVSS (SEQ ID NO: 68) |
| h103VL1 | ELVLTQSPSASASLGASVKLTCTLSSQHSTYTIEWHQQQPEKGPR YLMELKKDGSHSTGDGIPDRFSGSSSGAERYLTISSLQSEDEADY YCGVGNTIKEQFVYVFGGGTKLTVL (SEQ ID NO: 69) |
| h103VL2 | ELVLTQSPSASASLGASVKLTCTLSSQHSTYTIEWYQQQPEKGPR YLMELKKDGSHSTGDGIPDRFSGSSSGAERYLTISSLQSEDEADY YCGVGNTIKEQFVYVFGGGTKLTVL (SEQ ID NO: 70) |
| h103VL3 | ELVLTQSPSASASLGASVKLTCTLSSQHSTYTIEWYQQQPEKGPR YVMELKKDGSHSTGDGIPDRFSGSSSGAERYLTISSLQSEDEADY YCGVGNTIKEQFVYVFGGGTKLTVL (SEQ ID NO: 71) |
| h103VL4 | ELVVTQSPSASASLGASVKLTCTLSSQHSTYTIEWYQQQPEKGPR YVMELKKDGSHSTGDGIPDRFSGSSSGAERYLTISSLQSEDEADY YCGVGNTIKEQFVYVFGGGTKLTVL (SEQ ID NO: 72) |
| h103VL5 | ELVLTQSPSASASLGASVKLTCTLSSQHSTYTIEWYQQQPEKPPR YVMELKKDGSHSTGDGIPDRFSGSSSGAERYLTISSLQSEDEADY ICGVGNTIKEQFVYVFGGGTKLTVL (SEQ ID NO: 73) |
| h103VL6 | ELVVTQSPSASASLGASVKLTCTLSSQHSTYTIEWYQQQPEKPPR YVMELKKDGSHSTGDGIPDRFSGSSSGAERYLTISSLQSEDEADY ICGVGNTIKEQFVYVFGGGTKLTVL (SEQ ID NO: 74) |
| h103VL7 | ELVVTQSPSASASLGASVKLTCTLSSQHSTYTIEWYQQQPEKPPR YVMELKKDGSHSTGDGIPDRFSGSSSGADRYLTISSLQSEDEAD YICGVGNTIKEQFVYVFGGGTKLTVL (SEQ ID NO: 75) |
| h103VL8 | ELVLTQSPSASASLGASVKLTCTLSSQHSTYTIEWYQQQPEKGPR YVMELKKDGSHSTGEGIPDRFSGSSSGAERYLTISSLQSEDEADY YCGVGNTIKEQFVYVFGGGTKLTVL (SEQ ID NO: 76) |

| | |
|---|---|
| Notes: In the table, the underlined portion represents the CDR regions (corresponding to the Kabat numbering criteria), and the bold italic portion represents the mutation sites. | |

### 5. Preparation of humanized antibody

The expression vectors for the light chain and heavy chain of the antibody were constructed separately; each of the humanized antibody light chains was cross-paired and combined with a heavy chain to transfect 293E cells. The culture supernatant was collected and purified to obtain the humanized full-length antibody. The constant region of the humanized antibody heavy chain can be selected from the constant regions of IgG1, IgG2, IgG3, IgG4 and variants thereof; As an example, a full-length heavy chain of an antibody can be formed by linking the heavy chain constant region of human IgG1 (as shown in SEQ ID NO: 77) to a humanized heavy chain variable region as described above. The humanized antibody light chain constant region can be selected from the constant regions of human kappa, lambda chains or variants thereof; As an example, a full-length light chain of an antibody can be formed by linking the human light chain constant region kappa chain (as shown in SEQ ID NO: 78) or human light chain constant region lambda chain (as shown in SEQ ID NO: 79) to a humanized light chain variable region as described above.

An exemplary antibody constant region sequence is as follows:
Human IgG1 heavy chain constant region: 






 SEQ ID NO: 77;
Human light chain constant region kappa chain: 


 SEQ ID NO: 78;
Human light chain constant region lambda chain:

GQPKANPTVTLFPPSSEELQANKATLVCLISDFYPGAVTVAWKADGSP
VKAGVETTKPSKQSNNKYAASSYLSLTPEQWKSHRSYSCQVTHEGSTVEKTVA
PTECS SEQ ID NO: 79;

As an example, a series of humanized mAb47 antibodies as shown in Table 12 were obtained by linking a humanized antibody heavy chain variable region derived from mAb47 as described in Table 5 to the amino terminus of the human heavy chain IgG1 constant region as shown in SEQ ID NO: 77 to form a full length antibody heavy chain, and meanwhile, linking a humanized antibody light chain variable region as described in Table 5 to the amino terminus of the human light chain kappa constant region as shown in SEQ ID NO: 78 to form a full-length antibody light chain:

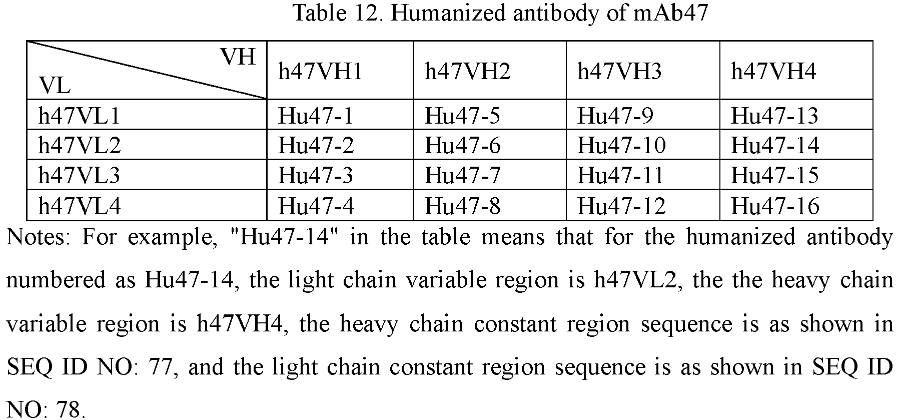

As an example, a series of humanized mAb63 antibodies as shown in Table 13 were obtained by linking a humanized antibody heavy chain variable region derived from mAb63 as described in Table 7 to the amino terminus of the human heavy chain IgG1 constant region as shown in SEQ ID NO: 77 to form a full length antibody heavy chain, and meanwhile, linking a humanized antibody light chain variable region as described in Table 7 to the amino terminus of the human light chain kappa constant region as shown in SEQ ID NO: 78 to form a full-length antibody light chain:

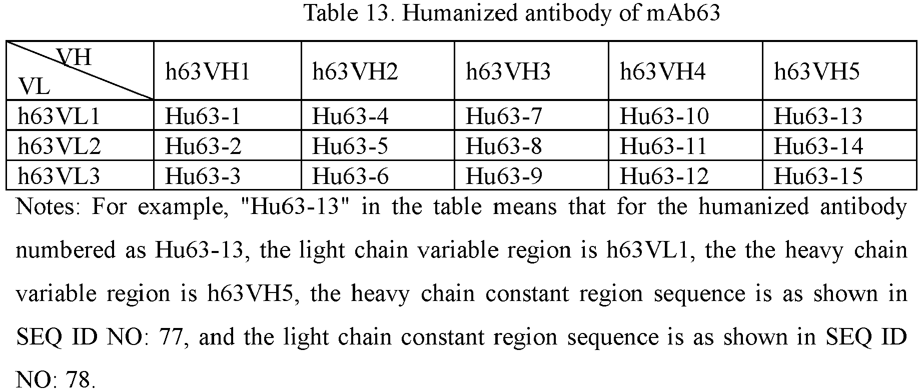

As an example, a series of humanized mAb67 antibodies as shown in Table 14 were obtained by linking a humanized antibody heavy chain variable region derived from mAb67 as described in Table 9 to the amino terminus of the human heavy chain IgG1 constant region as shown in SEQ ID NO: 77 to form a full length antibody heavy chain, and meanwhile, linking a humanized antibody light chain variable region as described in Table 9 to the amino terminus of the human light chain kappa constant region as shown in SEQ ID NO: 78 to form a full-length antibody light chain:

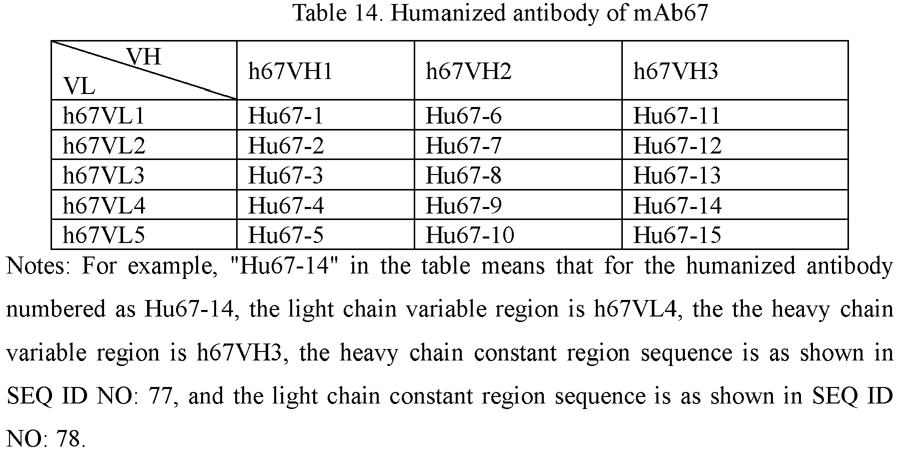

As an example, a series of humanized mAb103 antibodies as shown in Table 15 were obtained by linking a humanized antibody heavy chain variable region derived from mAb103 as described in Table 11 to the amino terminus of the human heavy chain IgG1 constant region as shown in SEQ ID NO: 77 to form a full length antibody heavy chain, and meanwhile, linking a humanized antibody light chain variable region as described in Table 11 to the amino terminus of the human light chain lambda constant region as shown in SEQ ID NO: 79 to form a full-length antibody light chain:

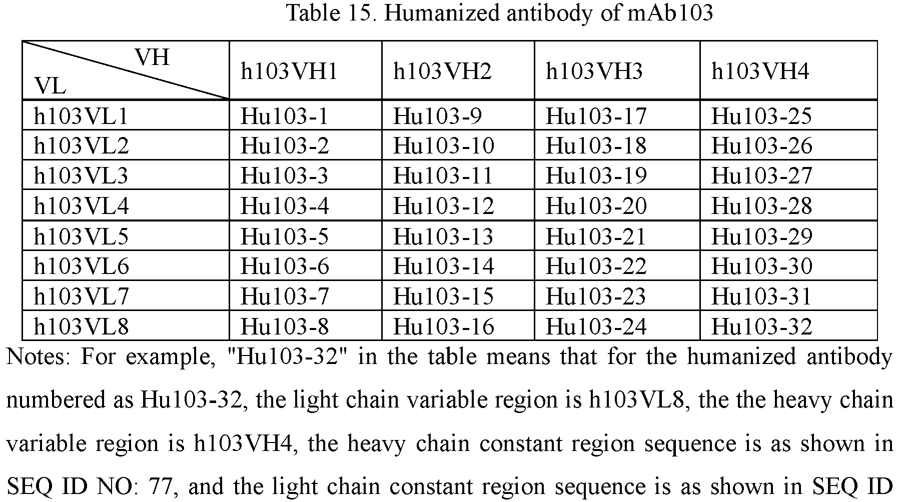

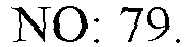

As an example, the full-length sequences of the humanized antibody light/heavy chain are shown in Table 16 below:

**Table 16. Humanized antibody light/heavy chain sequence**

| Antibody number | Heavy chain sequence | Light chain sequence |
|---|---|---|
| Hu63-13 | EVQLVQSGAEVKKPGASVKVSCKASGYTF TDFYMNWVRQAPGQGLEWMGDIFPKSGN TDYNRKFKDRVTMTRDTSTSTVYMELSSL RSEDTAVYYCARSGYGNYVFDYWGQGTLV TVSSASTKGPSVFPLAPSSKSTSGGTAALGCLV KDYFPEPVTVSWNSGALTSGVHTFPAVLQSSG LYSLSSVVTVPSSSLGTQTYICNVNHKPSNTKV DKKVEPKSCDKTHTCPPCPAPELLGGPSVFL FPPKPKDTLMISRTPEVTCVVVDVSHEDPEVK FNWYVDGVEVHNAKTKPREEQYNSTYRVVSV LTVLHQDWLNGKEYKCKVSNKALPAPIEKTIS KAKGQPREPQVYTLPPSRDELTKNQVSLTCLV KGFYPSDIAVEWESNGQPENNYKTTPPVLDS DGSFFLYSKLTVDKSRWQQGNVFSCSVMHEA LHNHYTQKSLSLSPGK SEQ ID NO: 80 | DIQMTQSPSSLSASVG DRVTITCRTSQDINIYL NWYQQKPGKAPKLLI YYRSGLLSGVPSRFSG SGSGTDFTLTISSLQPE DFATYYCQQGNTLPP TFGGGTKVEIKRTVAA PSVFIFPPSDEQLKSGT ASVVCLLNNFYPREAK VQWKVDNALQSGNSQ ESVTEQDSKDSTYSLSS TLTLSKADYEKHKVYAC EVTHQGLSSPVTKSFN RGEC SEQ ID NO: 81 |
| Hu47-14 | EVQLVQSGSELKKPGASVKVSCKASGYTFT TYGMSWVKQAPGQGLKWMGWINTYSGV PTYASDFKGRFVFSLDTSVSTAYLQISSLKA EDTAVYYCARRGNYGRWDFDVWGQGTTV TVSSASTKGPSVFPLAPSSKSTSGGTAALGCLV KDYFPEPVTVSWNSGALTSGVHTFPAVLQSSG LYSLSSVVTVPSSSLGTQTYICNVNHKPSNTKV DKKVEPKSCDKTHTCPPCPAPELLGGPSVFL FPPKPKDTLMISRTPEVTCVVVDVSHEDPEVK FNWYVDGVEVHNAKTKPREEQYNSTYRVVSV LTVLHQDWLNGKEYKCKVSNKALPAPIEKTIS KAKGQPREPQVYTLPPSRDELTKNQVSLTCLV KGFYPSDIAVEWESNGQPENNYKTTPPVLDS DGSFFLYSKLTVDKSRWQQGNVFSCSVMHEA LHNHYTQKSLSLSPGK SEQ ID NO: 82 | EIVLTQSPDFQSVTPK EKVTITCSVSSTISSSN LHWYQQKPDQSPKPL IYGTSNLASGVPSRFS GSGSGTDYTLTINSLE AEDAATYYCQQWSIY PLTFGQGTKLEIKRTV AAPSVFIFPPSDEQLKS GTASVVCLLNNFYPREA KVQWKVDNALQSGNS QESVTEQDSKDSTYSLS STLTLSKADYEKHKVYA CEVTHQGLSSPVTKSF NRGEC SEQ ID NO: 83 |
| Hu67-14 | EVQLVQSGAEVKKPGESLKISCKGSGYSFT DYHMNWVKQMPGKGLEWMGDINPDIGGT SYNQNFKGKVTISVDKSISTAYLQWSSLKA SDTAMYYCARWDFDSFANWGQGTLVTVSS ASTKGPSVFPLAPSSKSTSGGTAALGCLVKDY FPEPVTVSWNSGALTSGVHTFPAVLQSSGLYSL SSVVTVPSSSLGTQTYICNVNHKPSNTKVDKK VEPKSCDKTHTCPPCPAPELLGGPSVFLFPPK PKDTLMISRTPEVTCVVVDVSHEDPEVKFNW YVDGVEVHNAKTKPREEQYNSTYRVVSVLTVL HQDWLNGKEYKCKVSNKALPAPIEKTISKAKG QPREPQVYTLPPSRDELTKNQVSLTCLVKGFY PSDIAVEWESNGQPENNYKTTPPVLDSDGSFF LYSKLTVDKSRWQQGNVFSCSVMHEALHNHY TQKSLSLSPGK SEQ ID NO: 84 | DIQMTQSPSSLSASVG DRVTITCRASESVSIIG TNLIHWYQQKPGKAP KLLIYHASNLETGVPS RFSGSGSGTDFTLTISS LQPEDFATYYCLQSRK IPYTFGGGTKVEIKRT VAAPSVFIFPPSDEQLK SGTASVVCLLNNFYPRE AKVQWKVDNALQSGN SQESVTEQDSKDSTYSL SSTLTLSKADYEKHKVY ACEVTHQGLSSPVTKS FNRGEC SEQ ID NO: 85 |
| Hu103-32 | EVQLVQSGSELKKPGASVKVSCKASGYTFT TYGVIWVRQAPGQGLEWMGWINTYSGVP TYASDFKGRFVFSLDTSVSTAYLQISSLKAE DTAVYYCARKKTLTTVTPWFAYWGQGTLV TVSSASTKGPSVFPLAPSSKSTSGGTAALGCLV KDYFPEPVTVSWNSGALTSGVHTFPAVLQSSG LYSLSSVVTVPSSSLGTQTYICNVNHKPSNTKV DKKVEPKSCDKTHTCPPCPAPELLGGPSVFL FPPKPKDTLMISRTPEVTCVVVDVSHEDPEVK FNWYVDGVEVHNAKTKPREEQYNSTYRVVSV LTVLHQDWLNGKEYKCKVSNKALPAPIEKTIS KAKGQPREPQVYTLPPSRDELTKNQVSLTCLV KGFYPSDIAVEWESNGQPENNYKTTPPVLDS DGSFFLYSKLTVDKSRWQQGNVFSCSVMHEA LHNHYTQKSLSLSPGK SEQ ID NO: 86 | ELVLTQSPSASASLGA SVKLTCTLSSQHSTYT IEWYQQQPEKGPRYV MELKKDGSHSTGEGI PDRFSGSSSGAERYLTI SSLQSEDEADYYCGV GNTIKEQFVYVFGGG TKLTVLGQPKANPTVT LFPPSSEELQANKATLV CLISDFYPGAVTVAWKA DGSPVKAGVETTKPSK QSNNKYAASSYLSLTPE QWKSHRSYSCQVTHEG STVEKTVAPTECS SEQ ID NO: 87 |

| | | |
|---|---|---|
| Notes: The italic portion in the table represents constant region sequences, and the normal characters represent variable region sequences. | | |

There are currently two known ADC molecules targeting CEA: SAR-408701 and Labetuzumab-govitecan (also known as Lmab-CL2A-SN38) conjugate, in which the antibody light/heavy chain sequences are as follows:
Heavy chain sequence of the antibody (referred to as Sanofi) comprised in SAR-408701: 
EVQLQESGPGLVKPGGSLSLSCAASGFVFSSYDMSWVRQTPERGLEW
VAYISSGGGITYAPSTVKGRFTVSRDNAKNTLYLQMNSLTSEDTAVYYCAAHYF
GSSGPFAYWGQGTLVTVSSASTKGPSVFPLAPSSKSTSGGTAALGCLVKDYFPEP
VTVSWNSGALTSGVHTFPAVLQSSGLYSLSSVVTVPSSSLGTQTYICNVNHKPSN
TKVDKKVEPKSCDKTHTCPPCPAPELLGGPSVFLFPPKPKDTLMISRTPEVTCVV
 
VDVSHEDPEVKFNWYVDGVEVHNAKTKPREEQYNSTYRVVSVLTVLHQDWL
NGKEYKCKVSNKALPAPIEKTISKAKGQPREPQVYTLPPSRDELTKNQVSLTCLV
KGFYPSDIAVEWESNGQPENNYKTTPPVLDSDGSFFLYSKLTVDKSRWQQGNVF
SCSVMHEALHNHYTQKSLSLSPGK SEQ ID NO: 88;
Light chain sequence of the antibody (referred to as Sanofi) comprised in SAR-408701 
DIQMTQSPASLSASVGDRVTITCRASENIFSYLAWYQQKPGKSPKLLVY
NTRTLAEGVPSRFSGSGSGTDFSLTISSLQPEDFATYYCQHHYGTPFTFGSGTKLE
IKRTVAAPSVFIFPPSDEQLKSGTASVVCLLNNFYPREAKVQWKVDNALQSGNS
QESVTEQDSKDSTYSLSSTLTLSKADYEKHKVYACEVTHQGLSSPVTKSFNRGE
C SEQ ID NO: 89;
Heavy chain sequence of Labetuzumab (referred to as Lmab) comprised in Lmab-CL2A-SN38 
EVQLVESGGGVVQPGRSLRLSCSASGFDFTTYWMSWVRQAPGKGLE
WIGEIHPDSSTINYAPSLKDRFTISRDNAKNTLFLQMDSLRPEDTGVYFCASLYF
GFPWFAYWGQGTPVTVSSASTKGPSVFPLAPSSKSTSGGTAALGCLVKDYFPEP
VTVSWNSGALTSGVHTFPAVLQSSGLYSLSSVVTVPSSSLGTQTYICNVNHKPSN
TKVDKRVEPKSCDKTHTCPPCPAPELLGGPSVFLFPPKPKDTLMISRTPEVTCVV
VDVSHEDPEVKFNWYVDGVEVHNAKTKPREEQYNSTYRVVSVLTVLHQDWL
NGKEYKCKVSNKALPAPIEKTISKAKGQPREPQVYTLPPSREEMTKNQVSLTCL
VKGFYPSDIAVEWESNGQPENNYKTTPPVLDSDGSFFLYSKLTVDKSRWQQGN
VFSCSVMHEALHNHYTQKSLSLSPGK SEQ ID NO: 90;
Light chain sequence of Labetuzumab (referred to as Lmab) comprised in Lmab-CL2A-SN38

DIQLTQSPSSLSASVGDRVTITCKASQDVGTSVAWYQQKPGKAPKLLIY
WTSTRHTGVPSRFSGSGSGTDFTFTISSLQPEDIATYYCQQYSLYRSFGQGTKVEI
KRTVAAPSVFIFPPSDEQLKSGTASVVCLLNNFYPREAKVQWKVDNALQSGNSQ
ESVTEQDSKDSTYSLSSTLTLSKADYEKHKVYACEVTHQGLSSPVTKSFNRGEC
SEQ ID NO: 91.

### Test Examples

### Test example 1: FACS binding assay

In order to detect the binding of the antibody to the CEA protein on the cell surface, cells expressing CEA on the cell surface were used for the detection of the antibody binding activity by FACS. The cells were collected and centrifuged at 400g for 5 min at 4°C. Pre-cooled PBS with a final concentration of 10% FBS was added and centrifuged at 400 g for 5 min at 4°C, repeated twice. The cells were loaded into a 96-well plate, at 10⁵ cells/well. 100 µl of a gradient of diluted antibody solution was added to each well, incubated at 4°C for 60 min and centrifuged to remove the supernatant. 250 µl of pre-cooled PBS containing a final concentration of 10% FBS was added to each well to re-suspend the cells, centrifuged at 400 g, 4°C for 5 min and the supernatant was removed, repeated twice. 50 µl 1:200 diluted secondary antibody Alexa Fluor@488 goat anti-human IgG (H+L) (Lifetechologies, Cat# A11013) was added, incubated at 4°C in the dark for 45 min and centrifuged to remove the supernatant. 250 µl of pre-cooled PBS containing a final concentration of 10% FBS was added to each well for resuspension, and centrifuged at 400 g, 4°C for 5 min, repeated twice. 100 µl of pre-cooled PBS was added to each well to re-suspend the cells. Flow cytometer (BD, FACSverse) was used for detection to obtain the fluorescence signal value. The higher the signal is, the stronger the antibody binds to the protein on the cell surface. PRISM analysis software was used to plot a binding curve based on the detection results, and the EC50 values representing the binding activity of antibody to cell surface human CEA protein (MKN45 human gastric cancer cells, Nanjing Kebai Biotechnology Co., Ltd., Cat# CBP60488), cynoCEA-CHO and CEACAM1-CHO were obtained by fitting. The binding activities of humanized antibodies are shown in Tables 17 and 18 below:

**Table 17. Binding activity of humanized antibody to cell surface CEA proteins of different species**

| Antibody | EC50 (nM) | | |
|---|---|---|---|
| | MKN45 | cynoCEA-CHO | CEACAM1-CHO |
| Hu63-13 | 2.93 | 1.71 | No binding |
| Hu47-14 | 4.19 | 2.70 | No binding |
| Hu67-14 | 1.58 | 2.89 | No binding |
| Hu103-32 | 1.79 | 1.24 | No binding |
| Sanofi | 2.64 | 17.27 | No binding |
| Lmab | 2.45 | **No binding** | No binding |

The binding activities showing other humanized antibodies to cell surface CEA protein (MKN45, cynoCEA-CHO) are shown in Table 18 below:

**Table 18. Binding activity of humanized antibody to cell surface CEA protein**

| Antibody | EC50 (nM) | | Antibody | EC50 (nM) | |
|---|---|---|---|---|---|
| | MKN45 | cynoCEA-CHO | | MKN45 | cynoCEA-CHO |
| Hu103-1 | 2.37 | 2.49 | Hu47-6 | 2.50 | 0.30 |
| Hu103-2 | 2.18 | 1.26 | Hu47-10 | 2.27 | 0.27 |
| Hu103-3 | 1.91 | 1.18 | Hu63-1 | 4.23 | 1.24 |
| Hu103-4 | 2.62 | 1.32 | Hu63-2 | 3.73 | 2.64 |
| Hu103-5 | 2.38 | 1.47 | Hu63-3 | 3.56 | 2.01 |
| Hu103-6 | 1.27 | 1.56 | Hu63-4 | 5.18 | 1.95 |
| Hu103-7 | 2.20 | 1.62 | Hu63-5 | 3.70 | 2.14 |
| Hu103-9 | 2.95 | 1.61 | Hu63-6 | 3.71 | 2.88 |
| Hu103-10 | 2.23 | 1.00 | Hu63-8 | 3.24 | 2.43 |
| Hu103-11 | 2.71 | 0.92 | Hu63-9 | 4.87 | 2.47 |
| Hu103-12 | 2.38 | 1.08 | Hu63-10 | 4.95 | 1.88 |
| Hu103-13 | 2.53 | 1.01 | Hu63-11 | 4.35 | 2.29 |
| Hu103-14 | 2.41 | 1.37 | Hu63-12 | 4.04 | 2.23 |
| Hu103-15 | 2.13 | 1.36 | Hu67-1 | 2.46 | 4.57 |
| Hu103-17 | 2.92 | 1.52 | Hu67-4 | 3.25 | 3.06 |
| Hu103-18 | 2.75 | 0.89 | Hu67-5 | 2.86 | 3.50 |
| Hu103-19 | 2.42 | 0.96 | Hu67-7 | 3.88 | 7.20 |
| Hu103-20 | 2.19 | 0.98 | Hu67-9 | 3.75 | 3.72 |
| Hu103-21 | 1.34 | 1.17 | Hu67-10 | 3.19 | 5.37 |
| Hu103-22 | 3.64 | 1.02 | Hu67-11 | 2.26 | 4.66 |
| Hu103-23 | 2.90 | 0.93 | Hu67-12 | 3.20 | 5.31 |

The experimental results show that the humanized antibodies obtained by screening in the present disclosure maintain a binding activity similar to that of murine antibodies, and all of them can bind to the human CEA protein on the cell surface; the humanized antibodies obtained by screening in the present disclosure can bind to the cell surface monkey CEA protein, and the binding activity to monkey CEA protein is superior to that of the positive control antibody.

### Test example 2: Competitive assay with soluble CEA (sCEA)

In order to detect whether the antibody preferentially binds to CEA present on the surface of cell membrane in the presence of sCEA (as shown in SEQ ID NO:1), the inventors pre-incubated a gradient of diluted antibodies with a certain concentration of sCEA (5 µg/ml) for 30 min, then MKN45 cells were collected (human gastric cancer cells, Nanjing Kebai Biotechnology Co., Ltd., Cat# CBP60488) and loaded into 96-well plate. The gradient of diluted antibodies as well as the mixture of pre-incubated antibody and sCEA were separately added to each well, incubated at 4°C for 60 min and centrifuged to remove the supernatant. Pre-cooled PBS containing a final concentration of 10% FBS was added to wash the cells, repeated twice. 50 µl of 1:200 diluted secondary antibody Alexa Fluor@488 goat anti-human IgG (H+L) (Lifetechologies, Cat# A11013) was added, incubated at 4°C in dark for 45 min and centrifuged to remove the supernatant. 250 µl of pre-cooled PBS containing a final concentration of 10% FBS was added to wash the cells; repeated twice. 100 µl of pre-cooled PBS was added to each well for resuspension. Flow cytometer (FACS) was used for detection to obtain the fluorescence signal value. If the ratio of the signals (without sCEA v.s. with sCEA) at each antibody concentration is less than 2, it means that the binding curve of the antibody does not change significantly in the presence of sCEA, and the antibody still preferentially binds to CEA on the surface of cell membrane.

The experimental results are shown in Table 19 and Table 20. For different concentrations of Hu63-13, Hu47-14, Hu67-14, Hu103-32 and positive control Lmab, the ratios of the fluorescence signals (without sCEA v.s. with sCEA) are shown in Table 19 below. For other test antibodies, the maximum ratios of the fluorescence signals (without sCEA v.s. with sCEA) are shown in Table 20 below:

**Table 19. Fluorescence signal ratio of humanized antibody (without sCEA v.s with sCEA)**

| | Fluorescence signal ratio | | | | |
|---|---|---|---|---|---|
| Antibody concentration (µg/ml) | Hu63-13 | Hu47-14 | Hu67-14 | Hu103-32 | Lmab |
| 40 | 0.92 | 1.09 | 0.94 | 0.91 | 1.10 |
| 1.6 | 1.36 | 1.18 | 1.73 | 1.67 | 2.78 |
| 0.32 | 1.59 | 1.23 | 1.78 | 1.24 | 5.18 |
| 0.064 | 1.59 | 1.24 | 1.25 | 1.44 | 4.15 |
| 0.0128 | 1.43 | 1.09 | 1.50 | 1.28 | 2.72 |
| 0.00256 | 1.24 | 1.15 | 1.58 | 1.09 | 1.74 |
| 0.000512 | 1.02 | 1.07 | 1.08 | 0.86 | 1.20 |

**Table 20. Maximum fluorescence signal ratio of antibody (without sCEA v.s with sCEA)**

| Antibody | Maximum fluorescence signal ratio | Antibody | Maximum fluorescence signal ratio |
|---|---|---|---|
| Hu47-2 | 1.04 | Hu103-1 | 1.30 |
| Hu47-3 | 1.10 | Hu103-2 | 1.17 |
| Hu47-4 | 1.13 | Hu103-3 | 1.14 |
| Hu47-6 | 1.26 | Hu103-4 | 1.17 |
| Hu47-7 | 1.11 | Hu103-5 | 1.15 |
| Hu47-8 | 1.04 | Hu103-6 | 1.15 |
| Hu47-10 | 1.13 | Hu103-7 | 1.07 |
| Hu47-12 | 1.08 | Hu103-9 | 1.57 |
| Hu63-1 | 1.31 | Hu103-10 | 1.19 |
| Hu63-3 | 1.14 | Hu103-11 | 1.16 |
| Hu67-1 | 1.35 | Hu103-12 | 1.24 |
| Hu67-2 | 1.21 | Hu103-13 | 1.16 |
| Hu67-4 | 1.25 | Hu103-14 | 1.10 |
| Hu67-5 | 1.09 | Hu103-15 | 1.23 |
| Hu67-6 | 1.22 | Hu103-17 | 1.12 |
| Hu67-7 | 1.03 | Hu103-18 | 1.22 |
| Hu67-9 | 1.10 | Hu103-19 | 1.36 |
| Hu67-10 | 1.34 | Hu103-20 | 1.22 |
| Hu67-11 | 1.18 | Hu103-21 | 1.27 |
| Hu67-12 | 1.31 | Hu103-22 | 1.33 |
| Hu67-15 | 1.05 | Hu103-23 | 1.31 |
| mAb63 | 1.43 | mAb103 | 1.02 |
| mAb47 | 1.08 | Sanofi | 2.65 |

The experimental results show that, in the absence of and in the presence of sCEA, the humanized antibodies Hu63-13, Hu47-14, Hu67-14 and Hu103-32 screened in the present disclosure show a fluorescence signal ratio less than 2, at various antibody concentrations. For example, the maximum ratio for Hu63-13 is 1.59, and the maximum ratio for the positive control Lmab is 5.18. The antibodies screened in this disclosure are superior to the control antibody. The maximum ratio of the fluorescence signals (without sCEA *v.s* with sCEA) for the humanized antibodies screened in the present disclosure is less than 2 and less than that of the positive control antibody, indicating that the humanized antibodies screened in the present disclosure preferentially bind to CEA on the surface of cell membrane, even in the presence of sCEA, and is better than the positive control antibody.

### Test example 3: Affinity of antibodies to soluble CEA detected by Biacore

Biacore (GE, T200) instrument was used to determine the affinity of the humanized antibodies to be tested to human or monkey soluble CEA. Human anti-capture antibody was covalently coupled to the biosensor chip CM5 (GE, Cat# BR-1005-30) of the Biacore instrument, according to the method described in the instruction provided by the human anti-capture kit (GE, Cat# BR-1008-39), such that a certain amount of antibody to be tested was affinity captured, and then a series of gradient concentrations of soluble CEA antigen (as shown in SEQ ID NO: 1) were allowed to flow through the surface of the chip. The reaction signal was detected in real time by Biacore to generate a binding and dissociation curve. After the dissociation was completed for each cycle, the biochip was washed and regenerated with the regeneration solution supplied in the Human anti-Capture Kit. The data obtained from the experiment were fitted with the (1:1) Langmuir model by using BIAevaluation version 4.1 software, resulting in the affinity values. The lower the affinity of an antibody to soluble CEA is, the better the antibody would be, since the present disclose would like to obtain such an antibody that exhibits a stronger binding activity to CEA on the surface of cell membrane than that to soluble CEA. The test results of the affinity of humanized antibodies to soluble CEA are shown in Table 21 below:

**Table 21. Affinity of humanized antibody to human soluble CEA**

| | Human soluble CEA | | |
|---|---|---|---|
| Antibody | ka (1/Ms) | kd (1/s) | KD (M) |
| Hu63-13 | 2.99E+04 | 4.31E-03 | 1.44E-07 |
| Hu47-14 | 1.31E+05 | 1.88E-01 | 1.43E-06 |
| Hu67-14 | 3.76E+04 | 8.59E-03 | 2.28E-07 |
| Sanofi | 5.95E+04 | 3.08E-03 | 5.17E-08 |
| Lmab | 5.58E+04 | 3.31E-04 | 5.93E-09 |

The test results show that the humanized antibodies Hu63-13, Hu47-14 and Hu67-14 exhibit a lower affinity to soluble CEA protein, which is significantly lower than that of the control antibodies Sanofi and Lmab. This indicates that Hu63-13, Hu47-14 and Hu67-14 are not susceptible to the neutralization by the soluble CEA which is present in blood *in vivo,* and a large amount of antibodies can bind to cells expressing CEA on the surface of cell membrane.

### Test example 4: Endocytosis activity of anti-CEA antibody in MKN45 cells highly expressing CEA

The anti-CEA antibody conjugate in the present disclosure can release toxins to kill the cells after being internalized into cells, so the endocytosis activity of CEA antibody by CEA-expressing cells can promote ADC activity. In order to evaluate the endocytosis activity of humanized antibodies in MKN45 cells (human gastric cancer cells, Nanjing Kebai Biotechnology Co., Ltd., Cat# CBP60488), MKN45 were plated in a 96-well plate (Coring, Cat# 3795) and cultured overnight. On the next day, humanized CEA antibodies Hu63-13, Hu47-14, Hu67-14 and Hu103-3 were separately pre-incubated with iFL Green Human IgG Labeling Reagent (Invitrogen, Cat# Z25611) for 15 min; the iFL agent was bound to Fc of the humanized antibody. Then the antibody-iFL complex was added to the cell culture plate, the cell culture solution was removed after 6 hours and 24 hours, respectively, PBS was added to wash twice, the cells were digested and collected, and FACS was used to detect the fluorescence signal intensity of the cells. Once the antibody is internalized into the cell, the iFL bound to the antibody Fc will be brought into the cell. Once the iFL is internalized into the cells, the fluorescent signal can be detected only in an acidic environment. Therefore, a stronger detection signal represents a higher endocytosis activity of the antibody. The endocytosis activity of each humanized antibody is shown in Figure 1: All humanized antibodies can be internalized into MKN45. More antibodies would be internalized along with time.

### Test example 5: In vivo pharmacokinetic test

*In vivo* pharmacokinetic tests were performed in SD rats. SD rats (Sippr-BK Laboratory Animal Co., Ltd.) were randomly divided into groups, three in each group, administered intravenously at a dose of 3 mg/kg. For administration groups, 0.3 ml of whole blood sample was collected before dosing, and 5 min, 8 h, 1 d, 2 d, 4 d, 7 d, 10 d, 14 d, 21 d, 28 d after dosing, without addition of anticoagulation agent. The blood samples were placed at 4°C for 30, centrifuged at 1000 g for 15 min, the upper serum was taken and placed in an EP tube and stored at -80°C. The serum concentration of the drugs was detected by ELISA method, and the pharmacokinetic parameters of the test drugs were calculated by Winnolin software. The results are shown in Table 22.

**Table 22. In vivo pharmacokinetic parameters of the molecules**

| | Antibody sample | Hu63-13 | Hu47-14 | Hu67-14 | Hu103-32 |
|---|---|---|---|---|---|
| parameter | | mean | mean | mean | mean |
| t1/2 (h) | | 192.7 | 188.8 | 180.6 | 157.87 |
| t1/2(d) | | 8.0 | 7.9 | 7.5 | 6.58 |
| Cmax (µg/ml) | | 67.6 | 55.2 | 49.6 | 66.35 |
| AUC 0-t (µg/ml^{∗}h) | | 4949 | 3444 | 4038 | 3235 |
| AUC 0-∞ (µg/ml^{∗}h) | | 5451 | 3741 | 4356 | 3357 |
| Vz (ml/kg) | | 153.0 | 218.8 | 179.9 | 206.29 |
| CL (ml/day/kg) | | 13.2 | 19.3 | 16.6 | 21.75 |
| MRT 0-∞ (h) | | 239.0 | 216.6 | 237.7 | 160.14 |

The antibodies of the present disclosure all exhibit favorable pharmacokinetic activity.

## Claims

1. An anti-CEA antibody comprising a heavy chain variable region and a light chain variable region, wherein:
i) a heavy chain variable region, wherein the HCDR1 and HCDR3 thereof are respectively the same as the HCDR1 and HCDR3 comprised in the heavy chain variable region as shown in SEQ ID NO: 7; and the HCDR2 thereof is the same as the HCDR2 comprised in the heavy chain variable region as shown in SEQ ID NO: 7, or has one amino acid difference thereto; and
a light chain variable region, wherein the LCDR1, LCDR2 and LCDR3 are respectively the same as the LCDR1, LCDR2 and LCDR3 comprised in the light chain variable region as shown in SEQ ID NO: 8; or
ii) a heavy chain variable region, wherein the HCDR1 and HCDR3 thereof are respectively the same as the HCDR1 and HCDR3 comprised in the heavy chain variable region as shown in SEQ ID NO: 9; and the HCDR2 thereof is the same as the HCDR2 comprised in the heavy chain variable region as shown in SEQ ID NO: 9, or has one amino acid difference thereto; and
a light chain variable region, wherein the LCDR1, LCDR2 and LCDR3 are respectively the same as the LCDR1, LCDR2 and LCDR3 comprised in the light chain variable region as shown in SEQ ID NO: 10; or
iii) a heavy chain variable region, wherein the HCDR1, HCDR2 and HCDR3 thereof are respectively the same as the HCDR1, HCDR2 and HCDR3 comprised in the heavy chain variable region as shown in SEQ ID NO: 11; and
a light chain variable region, wherein the LCDR1, LCDR2 and LCDR3 are respectively the same as the LCDR1, LCDR2 and LCDR3 comprised in the light chain variable region as shown in SEQ ID NO: 12; or
iv) a heavy chain variable region, wherein the HCDR1 and HCDR3 thereof are respectively the same as the HCDR1 and HCDR3 comprised in the heavy chain variable region as shown in SEQ ID NO: 13; and the HCDR2 thereof is the same as the HCDR2 comprised in the heavy chain variable region as shown in SEQ ID NO: 13, or has one amino acid difference thereto; and
a light chain variable region, wherein the LCDR1 and LCDR3 thereof are respectively the same as the LCDR1 and LCDR3 comprised in the light chain variable region as shown in SEQ ID NO: 14; and the LCDR2 thereof is the same as the LCDR2 comprised in the light chain variable region as shown in SEQ ID NO: 14, or has one amino acid difference thereto.

2. The anti-CEA antibody according to claim 1, comprising a heavy chain variable region and a light chain variable region, wherein:
v) the heavy chain variable region comprises HCDR1, HCDR2 and HCDR3 as shown in SEQ ID NO: 15, SEQ ID NO: 16 and SEQ ID NO: 17, respectively, and the light chain variable region comprises LCDR1, LCDR2 and LCDR3 as shown in NO: 18, SEQ ID NO: 19 and SEQ ID NO: 20, respectively; or
the heavy chain variable region comprises HCDR1, HCDR2 and HCDR3 as shown in SEQ ID NO: 15, SEQ ID NO: 38 and SEQ ID NO: 17, respectively, and the light chain variable region comprises LCDR1, LCDR2 and LCDR3 as shown in NO: 18, SEQ ID NO: 19 and SEQ ID NO: 20, respectively; or
vi) the heavy chain variable region comprises HCDR1, HCDR2 and HCDR3 as shown in SEQ ID NO: 21, SEQ ID NO: 22 and SEQ ID NO: 23, respectively, and the light chain variable region comprises LCDR1, LCDR2 and LCDR3 as shown in NO: 24, SEQ ID NO: 25 and SEQ ID NO: 26, respectively; or
the heavy chain variable region comprises HCDR1, HCDR2 and HCDR3 as shown in SEQ ID NO: 21, SEQ ID NO: 47 and SEQ ID NO: 23, respectively, and the light chain variable region comprises LCDR1, LCDR2 and LCDR3 as shown in SEQ ID NO: 24, SEQ ID NO: 25 and SEQ ID NO: 26, respectively; or
vii) the heavy chain variable region comprises HCDR1, HCDR2 and HCDR3 as shown in SEQ ID NO: 27, SEQ ID NO: 28 and SEQ ID NO: 29, respectively, and the light chain variable region comprises LCDR1, LCDR2 and LCDR3 as shown in NO: 30, SEQ ID NO: 31 and SEQ ID NO: 32, respectively; or
viii) the heavy chain variable region comprises HCDR1 and HCDR3 as shown in SEQ ID NO: 33 and SEQ ID NO: 34 respectively, and HCDR2 as shown in SEQ ID NO: 16 or SEQ ID NO: 38;
the light chain variable region comprises LCDR1 and LCDR3 as shown in SEQ ID NO: 35 and SEQ ID NO: 37 respectively, and LCDR2 as shown in SEQ ID NO: 36 or SEQ ID NO: 64;
preferably, the heavy chain variable region comprises HCDR1, HCDR2 and HCDR3 as shown in SEQ ID NO: 33, SEQ ID NO: 38 and SEQ ID NO: 34, respectively; and the light chain variable region comprises LCDR1, LCDR2 and LCDR3 as shown in SEQ ID NO: 35, SEQ ID NO: 64 and SEQ ID NO: 37, respectively.

3. The anti-CEA antibody according to claim 1 or 2, wherein the anti-CEA antibody is a murine antibody, a chimeric antibody or a humanized antibody.

4. The anti-CEA antibody according to claim 3, wherein the anti-CEA antibody is a murine antibody or a chimeric antibody, which comprises a heavy chain variable region and a light chain variable region, wherein:
(a) the amino acid sequence of the heavy chain variable region is as shown in SEQ ID NO: 7 or has at least 90% sequence identity to SEQ ID NO: 7; and/or
the amino acid sequence of the light chain variable region is as shown in SEQ ID NO:8 or has at least 90% sequence identity to SEQ ID NO: 8; or
(b) the amino acid sequence of the heavy chain variable region is as shown in SEQ ID NO: 9 or has at least 90% sequence identity to SEQ ID NO: 9; and/or
the amino acid sequence of the light chain variable region is as shown in SEQ ID NO:10 or has at least 90% sequence identity to SEQ ID NO: 10; or
(c) the amino acid sequence of the heavy chain variable region is as shown in SEQ ID NO: 11 or has at least 90% sequence identity to SEQ ID NO: 11; and/or
the amino acid sequence of the light chain variable region is as shown in SEQ ID NO:12 or has at least 90% sequence identity to SEQ ID NO: 12; or
(d) the amino acid sequence of the heavy chain variable region is as shown in SEQ ID NO: 13 or has at least 90% sequence identity to SEQ ID NO: 13; and/or
the amino acid sequence of the light chain variable region is as shown in SEQ ID NO:14 or has at least 90% sequence identity to SEQ ID NO: 14.

5. The anti-CEA antibody according to any one of claims 1 to 3, wherein the anti-CEA antibody is a humanized antibody comprising a heavy chain variable region and a light chain variable region, wherein:
(e) the heavy chain variable region is as shown in SEQ ID NO:39, 40, 41 or 42, or has at least 90% sequence identity to SEQ ID NO:39, 40, 41 or 42 respectively; and/or
the light chain variable region is as shown in SEQ ID NO:43, 44, 45 or 46, or has at least 90% sequence identity to SEQ ID NO:43, 44, 45 or 46 respectively; or
(f) the heavy chain variable region is as shown in SEQ ID NO:48, 49, 50, 51 or 52, or has at least 90% sequence identity to SEQ ID NO:48, 49, 50, 51 or 52 respectively; and/or
the light chain variable region is as shown in SEQ ID NO:53, 54 or 55, or has at least 90% sequence identity to SEQ ID NO:53, 54 or 55 respectively; or
(g) the heavy chain variable region is as shown in SEQ ID NO:56, 57, or 58, or has at least 90% sequence identity to SEQ ID NO:56, 57, or 58 respectively; and/or
the light chain variable region is as shown in SEQ ID NO:59, 60, 61, 62 or 63, or has at least 90% sequence identity to SEQ ID NO:59, 60, 61, 62 or 63 respectively; or
(h) the heavy chain variable region is as shown in SEQ ID NO:65, 66, 67 or 68, or has at least 90% sequence identity to SEQ ID NO:65, 66, 67 or 68 respectively; and/or
the light chain variable region is as shown in SEQ ID NO:69, 70, 71, 72, 73, 74, 75 or 76, or has at least 90% sequence identity to SEQ ID NO:69, 70, 71, 72, 73, 74, 75 or 76 respectively;
preferably, the light chain variable region is as shown in SEQ ID NO:44, and the heavy chain variable region is as shown in SEQ ID NO:42; or
preferably, the light chain variable region is as shown in SEQ ID NO:53, and the heavy chain variable region is as shown in SEQ ID NO:52; or
preferably, the light chain variable region is as shown in SEQ ID NO:62, and the heavy chain variable region is as shown in SEQ ID NO:58; or
preferably, the light chain variable region is as shown in SEQ ID NO:76, and the heavy chain variable region is as shown in SEQ ID NO:68.

6. The anti-CEA antibody according to claim 1, 2, 3 or 5, wherein the anti-CEA antibody comprises framework region(s) derived from a human antibody or framework region variant(s) thereof, and the framework region variant has 1 to 10 back mutation(s) on each of the human antibody light chain framework region and/or heavy chain framework region;
preferably, the anti-CEA antibody comprises:
(i) a light chain variable region comprising LCDR1, LCDR2 and LCDR3 as shown in SEQ ID NO: 18, SEQ ID NO: 19 and SEQ ID NO: 20, respectively; and
a heavy chain variable region comprising HCDR1 as shown in SEQ ID NO: 15, HCDR2 as shown in SEQ ID NO: 16 or SEQ ID NO: 38, and HCDR3 as shown in SEQ ID NO: 17; and
wherein the framework region(s) of the light chain variable region comprise(s) one or more amino acid back mutation(s) selected from the group consisting of 46P, 47W, 49Y, 70S and 71Y, and/or the framework region(s) of the heavy chain variable region comprise(s) one or more amino acid back mutation(s) selected from 38K and 46K;
(j) a light chain variable region comprising LCDR1, LCDR2 and LCDR3 as shown in SEQ ID NO: 24, SEQ ID NO: 25 and SEQ ID NO: 26, respectively; and
a heavy chain variable region comprising HCDR1 as shown in SEQ ID NO: 21, HCDR2 as shown in SEQ ID NO: 22 or SEQ ID NO: 47, and HCDR3 as shown in SEQ ID NO: 23; and
wherein the framework region(s) of the light chain variable region comprise(s) one or more amino acid back mutation(s) selected from the group consisting of 2V, 42G, 44V and 71Y, and/or the framework region(s) of the heavy chain variable region comprise(s) one or more amino acid back mutation(s) selected from the group consisting of 481, 66K, 67A, 69L, 71V, 73K, 82F and 82A R;
(k) a light chain variable region comprising LCDR1, LCDR2 and LCDR3 as shown in SEQ ID NO: 30, SEQ ID NO: 31 and SEQ ID NO: 32, respectively; and
a heavy chain variable region comprising HCDR1, HCDR2 and HCDR3 as shown in SEQ ID NO: 27, SEQ ID NO: 28 and SEQ ID NO: 29, respectively, and
wherein the framework region(s) of the light chain variable region comprise(s) one or more amino acid back mutation(s) selected from the group consisting of 3V, 43P and 58V, and/or the framework region(s) of the heavy chain variable region comprise(s) one or more amino acid back mutation(s) selected from the group consisting of 38K, 66K and 71V;
(l) a light chain variable region comprising LCDR1 as shown in SEQ ID NO:35, LCDR2 as shown in SEQ ID NO:36 or SEQ ID NO:64, and LCDR3 as shown in SEQ ID NO:37; and
a heavy chain variable region comprising HCDR1 as shown in SEQ ID NO: 33, HCDR2 as shown in SEQ ID NO: 16 or SEQ ID NO: 38, and HCDR3 as shown in SEQ ID NO: 34; and
wherein the framework region(s) of the light chain variable region comprise(s) one or more amino acid back mutation(s) selected from the group consisting of 4V, 36Y, 43P, 47V, 49E, 70D and 871, and/or
the framework region(s) of the heavy chain variable region comprise(s) one or more amino acid back mutation(s) selected from the group consisting of 21, 38K and 46K;
wherein, the back mutation sites are numbered according to Kabat numbering criteria.

7. The anti-CEA antibody according to any one of claims 1 to 6, wherein the anti-CEA antibody further comprises a heavy chain constant region and a light chain constant region; preferably, the heavy chain constant region is selected from the group consisting of human IgG1, IgG2, IgG3 and IgG4 constant regions and conventional variant(s) thereof, and the light chain constant region is selected from the group consisting of human antibody κ and λ chain constant regions and conventional variant(s) thereof; more preferably, the antibody comprises a heavy chain constant region as shown in SEQ ID NO: 77 and a light chain constant region as shown in SEQ ID NO: 78 or SEQ ID NO: 79;
most preferably, the anti-CEA antibody comprises:
(m) a heavy chain as shown in SEQ ID NO: 80 or having at least 85% sequence identity to SEQ ID NO: 80, and/or
a light chain as shown in SEQ ID NO: 81 or having at least 85% sequence identity to SEQ ID NO: 81;
(n) a heavy chain as shown in SEQ ID NO: 82 or having at least 85% sequence identity to SEQ ID NO: 82, and/or
a light chain as shown in SEQ ID NO: 83 or having at least 85% sequence identity to SEQ ID NO: 83;
(o) a heavy chain as shown in SEQ ID NO: 84 or having at least 85% sequence identity to SEQ ID NO: 84, and/or
a light chain as shown in SEQ ID NO: 85 or having at least 85% sequence identity to SEQ ID NO: 85; or
(p) a heavy chain as shown in SEQ ID NO: 86 or having at least 85% sequence identity to SEQ ID NO: 86, and/or
a light chain as shown in SEQ ID NO: 87 or having at least 85% sequence identity to SEQ ID NO: 87.

8. An isolated anti-CEA antibody, which competes with the anti-CEA antibody of any one of claims 1 to 7 for binding to human CEA or the epitope thereof.

9. A nucleic acid molecule encoding the anti-CEA antibody of any one of claims 1 to 8.

10. A host cell comprising the nucleic acid molecule of claim 9.

11. An antibody-drug conjugate comprising or consisting of:
the anti-CEA antibody of any one of claims 1 to 8, and
a cytotoxic agent;
wherein, the cytotoxic agent is conjugated to the anti-CEA antibody.

12. A pharmaceutical composition comprising:
a prophylactically effective amount or a therapeutically effective amount of the anti-CEA antibody according to any one of claims 1 to 8, or the nucleic acid molecule according to claim 9, or the antibody-drug conjugate according to claim 11, and
one or more pharmaceutically acceptable carriers, diluents, buffers or excipients.

13. A method for the immunoassay or determination of CEA, the method comprising a step of contacting the anti-CEA antibody of any one of claims 1 to 8 with a sample.

14. A kit comprising the anti-CEA antibody according to any one of claims 1 to 8.

15. A method of preventing or treating a disease, the method comprising:
administering to a subject a prophylactically effective amount or a therapeutically effective amount of the anti-CEA antibody of any one of claims 1 to 8, or the nucleic acid molecule of claim 9, or the antibody-drug conjugate of claim 11, or the pharmaceutical composition of claim 12,
wherein the disease is a CEA-related disease, and the disease is preferably a tumor;
more preferably, the disease is selected from the group consisting of: head and neck squamous cell cancer, head and neck cancer, brain cancer, glioma, glioblastoma multiforme, neuroblastoma, central nervous system cancer, neuroendocrine tumors, throat cancer, nasopharyngeal cancer, esophageal cancer, thyroid cancer, malignant pleural mesothelioma, lung cancer, breast cancer, liver cancer, hepatocellular tumor, hepatocellular cancer, liver and gallbladder cancer, pancreatic cancer, gastric cancer, gastrointestinal cancer, intestinal cancer, colon cancer, colorectal cancer, kidney cancer, clear cell renal cell carcinoma, ovarian cancer, endometrial cancer, cervical cancer, bladder cancer, prostate cancer, testicular cancer, skin cancer, melanoma, leukemia, lymphoma, bone cancer, chondrosarcoma, myeloma, multiple myeloma, myelodysplastic syndrome, Krukenberg tumor, myeloproliferative tumor, squamous cell carcer, Ewing sarcoma, systemic light chain amyloidosis and Merkel cell carcer;
more preferably, the lymphoma is selected from the group consisting of: Hodgkin's lymphoma, non-Hodgkin's lymphoma, diffuse large B-cell lymphoma, follicular lymphoma, primary mediastinal large B-cell lymphoma, mantle cell lymphoma, small lymphocytic lymphoma, large B-cell lymphoma rich in T-cells/histiocytes, and lymphoplasmacytic lymphoma;
the lung cancer is selected from the group consisting of: non-small cell lung cancer and small cell lung cancer;
the leukemia is selected from the group consisting of: chronic myeloid leukemia, acute myeloid leukemia, lymphocytic leukemia, lymphoblastic leukemia, acute lymphoblastic leukemia, chronic lymphocytic leukemia, and myeloid leukemia.


Sequence Listing

<110>  JIANGSU HENGRUI MEDICINE CO., LTD.;
       SHANGHAI HENGRUI PHARMACEUTICAL CO., LTD.

<120>  ANTI-CEA ANTIBODY AND APPLICATION THEREOF

<130>  702059

<140>  PCT/CN2020/097984
<141>  2020-06-24

<150>  201910560329.6
<151>  2019-06-26

<160>  91

<170>  SIPOSequenceListing 1.0

<210>  1
<211>  657
<212>  PRT
<213>  Artificial Sequence

<220>
<223>  hCEA-His protein sequence


<400>  1
Lys Leu Thr Ile Glu Ser Thr Pro Phe Asn Val Ala Glu Gly Lys Glu
1               5                   10                  15
Val Leu Leu Leu Val His Asn Leu Pro Gln His Leu Phe Gly Tyr Ser
            20                  25                  30
Trp Tyr Lys Gly Glu Arg Val Asp Gly Asn Arg Gln Ile Ile Gly Tyr
        35                  40                  45
Val Ile Gly Thr Gln Gln Ala Thr Pro Gly Pro Ala Tyr Ser Gly Arg
    50                  55                  60
Glu Ile Ile Tyr Pro Asn Ala Ser Leu Leu Ile Gln Asn Ile Ile Gln
65                  70                  75                  80
Asn Asp Thr Gly Phe Tyr Thr Leu His Val Ile Lys Ser Asp Leu Val
                85                  90                  95
Asn Glu Glu Ala Thr Gly Gln Phe Arg Val Tyr Pro Glu Leu Pro Lys
            100                 105                 110
Pro Ser Ile Ser Ser Asn Asn Ser Lys Pro Val Glu Asp Lys Asp Ala
        115                 120                 125
Val Ala Phe Thr Cys Glu Pro Glu Thr Gln Asp Ala Thr Tyr Leu Trp
    130                 135                 140
Trp Val Asn Asn Gln Ser Leu Pro Val Ser Pro Arg Leu Gln Leu Ser
145                 150                 155                 160
Asn Gly Asn Arg Thr Leu Thr Leu Phe Asn Val Thr Arg Asn Asp Thr
                165                 170                 175
Ala Ser Tyr Lys Cys Glu Thr Gln Asn Pro Val Ser Ala Arg Arg Ser
            180                 185                 190
Asp Ser Val Ile Leu Asn Val Leu Tyr Gly Pro Asp Ala Pro Thr Ile
        195                 200                 205
Ser Pro Leu Asn Thr Ser Tyr Arg Ser Gly Glu Asn Leu Asn Leu Ser
    210                 215                 220
Cys His Ala Ala Ser Asn Pro Pro Ala Gln Tyr Ser Trp Phe Val Asn
225                 230                 235                 240
Gly Thr Phe Gln Gln Ser Thr Gln Glu Leu Phe Ile Pro Asn Ile Thr
                245                 250                 255
Val Asn Asn Ser Gly Ser Tyr Thr Cys Gln Ala His Asn Ser Asp Thr
            260                 265                 270

Gly Leu Asn Arg Thr Thr Val Thr Thr Ile Thr Val Tyr Ala Glu Pro
        275                 280                 285
Pro Lys Pro Phe Ile Thr Ser Asn Asn Ser Asn Pro Val Glu Asp Glu
    290                 295                 300
Asp Ala Val Ala Leu Thr Cys Glu Pro Glu Ile Gln Asn Thr Thr Tyr
305                 310                 315                 320
Leu Trp Trp Val Asn Asn Gln Ser Leu Pro Val Ser Pro Arg Leu Gln
                325                 330                 335
Leu Ser Asn Asp Asn Arg Thr Leu Thr Leu Leu Ser Val Thr Arg Asn
            340                 345                 350
Asp Val Gly Pro Tyr Glu Cys Gly Ile Gln Asn Glu Leu Ser Val Asp
        355                 360                 365
His Ser Asp Pro Val Ile Leu Asn Val Leu Tyr Gly Pro Asp Asp Pro
    370                 375                 380
Thr Ile Ser Pro Ser Tyr Thr Tyr Tyr Arg Pro Gly Val Asn Leu Ser
385                 390                 395                 400
Leu Ser Cys His Ala Ala Ser Asn Pro Pro Ala Gln Tyr Ser Trp Leu
                405                 410                 415
Ile Asp Gly Asn Ile Gln Gln His Thr Gln Glu Leu Phe Ile Ser Asn
            420                 425                 430
Ile Thr Glu Lys Asn Ser Gly Leu Tyr Thr Cys Gln Ala Asn Asn Ser
        435                 440                 445
Ala Ser Gly His Ser Arg Thr Thr Val Lys Thr Ile Thr Val Ser Ala
    450                 455                 460
Glu Leu Pro Lys Pro Ser Ile Ser Ser Asn Asn Ser Lys Pro Val Glu
465                 470                 475                 480
Asp Lys Asp Ala Val Ala Phe Thr Cys Glu Pro Glu Ala Gln Asn Thr
                485                 490                 495
Thr Tyr Leu Trp Trp Val Asn Gly Gln Ser Leu Pro Val Ser Pro Arg
            500                 505                 510
Leu Gln Leu Ser Asn Gly Asn Arg Thr Leu Thr Leu Phe Asn Val Thr
        515                 520                 525
Arg Asn Asp Ala Arg Ala Tyr Val Cys Gly Ile Gln Asn Ser Val Ser
    530                 535                 540
Ala Asn Arg Ser Asp Pro Val Thr Leu Asp Val Leu Tyr Gly Pro Asp
545                 550                 555                 560
Thr Pro Ile Ile Ser Pro Pro Asp Ser Ser Tyr Leu Ser Gly Ala Asn
                565                 570                 575
Leu Asn Leu Ser Cys His Ser Ala Ser Asn Pro Ser Pro Gln Tyr Ser
            580                 585                 590
Trp Arg Ile Asn Gly Ile Pro Gln Gln His Thr Gln Val Leu Phe Ile
        595                 600                 605
Ala Lys Ile Thr Pro Asn Asn Asn Gly Thr Tyr Ala Cys Phe Val Ser
    610                 615                 620
Asn Leu Ala Thr Gly Arg Asn Asn Ser Ile Val Lys Ser Ile Thr Val
625                 630                 635                 640
Ser Ala Ser Gly Thr Ser Pro Gly Leu Ser Ala His His His His His
                645                 650                 655
His


<210>  2
<211>  883
<212>  PRT
<213>  Artificial Sequence

<220>
<223>  hCEA-Fc protein sequence


<400>  2
Lys Leu Thr Ile Glu Ser Thr Pro Phe Asn Val Ala Glu Gly Lys Glu
1               5                   10                  15

Val Leu Leu Leu Val His Asn Leu Pro Gln His Leu Phe Gly Tyr Ser
            20                  25                  30
Trp Tyr Lys Gly Glu Arg Val Asp Gly Asn Arg Gln Ile Ile Gly Tyr
        35                  40                  45
Val Ile Gly Thr Gln Gln Ala Thr Pro Gly Pro Ala Tyr Ser Gly Arg
    50                  55                  60
Glu Ile Ile Tyr Pro Asn Ala Ser Leu Leu Ile Gln Asn Ile Ile Gln
65                  70                  75                  80
Asn Asp Thr Gly Phe Tyr Thr Leu His Val Ile Lys Ser Asp Leu Val
                85                  90                  95
Asn Glu Glu Ala Thr Gly Gln Phe Arg Val Tyr Pro Glu Leu Pro Lys
            100                 105                 110
Pro Ser Ile Ser Ser Asn Asn Ser Lys Pro Val Glu Asp Lys Asp Ala
        115                 120                 125
Val Ala Phe Thr Cys Glu Pro Glu Thr Gln Asp Ala Thr Tyr Leu Trp
    130                 135                 140
Trp Val Asn Asn Gln Ser Leu Pro Val Ser Pro Arg Leu Gln Leu Ser
145                 150                 155                 160
Asn Gly Asn Arg Thr Leu Thr Leu Phe Asn Val Thr Arg Asn Asp Thr
                165                 170                 175
Ala Ser Tyr Lys Cys Glu Thr Gln Asn Pro Val Ser Ala Arg Arg Ser
            180                 185                 190
Asp Ser Val Ile Leu Asn Val Leu Tyr Gly Pro Asp Ala Pro Thr Ile
        195                 200                 205
Ser Pro Leu Asn Thr Ser Tyr Arg Ser Gly Glu Asn Leu Asn Leu Ser
    210                 215                 220
Cys His Ala Ala Ser Asn Pro Pro Ala Gln Tyr Ser Trp Phe Val Asn
225                 230                 235                 240
Gly Thr Phe Gln Gln Ser Thr Gln Glu Leu Phe Ile Pro Asn Ile Thr
                245                 250                 255
Val Asn Asn Ser Gly Ser Tyr Thr Cys Gln Ala His Asn Ser Asp Thr
            260                 265                 270
Gly Leu Asn Arg Thr Thr Val Thr Thr Ile Thr Val Tyr Ala Glu Pro
        275                 280                 285
Pro Lys Pro Phe Ile Thr Ser Asn Asn Ser Asn Pro Val Glu Asp Glu
    290                 295                 300
Asp Ala Val Ala Leu Thr Cys Glu Pro Glu Ile Gln Asn Thr Thr Tyr
305                 310                 315                 320
Leu Trp Trp Val Asn Asn Gln Ser Leu Pro Val Ser Pro Arg Leu Gln
                325                 330                 335
Leu Ser Asn Asp Asn Arg Thr Leu Thr Leu Leu Ser Val Thr Arg Asn
            340                 345                 350
Asp Val Gly Pro Tyr Glu Cys Gly Ile Gln Asn Glu Leu Ser Val Asp
        355                 360                 365
His Ser Asp Pro Val Ile Leu Asn Val Leu Tyr Gly Pro Asp Asp Pro
    370                 375                 380
Thr Ile Ser Pro Ser Tyr Thr Tyr Tyr Arg Pro Gly Val Asn Leu Ser
385                 390                 395                 400
Leu Ser Cys His Ala Ala Ser Asn Pro Pro Ala Gln Tyr Ser Trp Leu
                405                 410                 415
Ile Asp Gly Asn Ile Gln Gln His Thr Gln Glu Leu Phe Ile Ser Asn
            420                 425                 430
Ile Thr Glu Lys Asn Ser Gly Leu Tyr Thr Cys Gln Ala Asn Asn Ser
        435                 440                 445
Ala Ser Gly His Ser Arg Thr Thr Val Lys Thr Ile Thr Val Ser Ala
    450                 455                 460
Glu Leu Pro Lys Pro Ser Ile Ser Ser Asn Asn Ser Lys Pro Val Glu
465                 470                 475                 480
Asp Lys Asp Ala Val Ala Phe Thr Cys Glu Pro Glu Ala Gln Asn Thr
                485                 490                 495
Thr Tyr Leu Trp Trp Val Asn Gly Gln Ser Leu Pro Val Ser Pro Arg
            500                 505                 510
Leu Gln Leu Ser Asn Gly Asn Arg Thr Leu Thr Leu Phe Asn Val Thr

Arg Asn Asp Ala Arg Ala Tyr Val Cys Gly Ile Gln Asn Ser Val Ser
        530                 535                 540
Ala Asn Arg Ser Asp Pro Val Thr Leu Asp Val Leu Tyr Gly Pro Asp
545                 550                 555                 560
Thr Pro Ile Ile Ser Pro Pro Asp Ser Ser Tyr Leu Ser Gly Ala Asn
                565                 570                 575
Leu Asn Leu Ser Cys His Ser Ala Ser Asn Pro Ser Pro Gln Tyr Ser
            580                 585                 590
Trp Arg Ile Asn Gly Ile Pro Gln Gln His Thr Gln Val Leu Phe Ile
        595                 600                 605
Ala Lys Ile Thr Pro Asn Asn Asn Gly Thr Tyr Ala Cys Phe Val Ser
    610                 615                 620
Asn Leu Ala Thr Gly Arg Asn Asn Ser Ile Val Lys Ser Ile Thr Val
625                 630                 635                 640
Ser Ala Ser Gly Thr Ser Pro Gly Leu Ser Ala Glu Pro Lys Ser Ser
                645                 650                 655
Asp Lys Thr His Thr Cys Pro Pro Cys Pro Ala Pro Glu Leu Leu Gly
            660                 665                 670
Gly Pro Ser Val Phe Leu Phe Pro Pro Lys Pro Lys Asp Thr Leu Met
        675                 680                 685
Ile Ser Arg Thr Pro Glu Val Thr Cys Val Val Val Asp Val Ser His
    690                 695                 700
Glu Asp Pro Glu Val Lys Phe Asn Trp Tyr Val Asp Gly Val Glu Val
705                 710                 715                 720
His Asn Ala Lys Thr Lys Pro Arg Glu Glu Gln Tyr Asn Ser Thr Tyr
                725                 730                 735
Arg Val Val Ser Val Leu Thr Val Leu His Gln Asp Trp Leu Asn Gly
            740                 745                 750
Lys Glu Tyr Lys Cys Lys Val Ser Asn Lys Ala Leu Pro Ala Pro Ile
        755                 760                 765
Glu Lys Thr Ile Ser Lys Ala Lys Gly Gln Pro Arg Glu Pro Gln Val
    770                 775                 780
Tyr Thr Leu Pro Pro Ser Arg Asp Glu Leu Thr Lys Asn Gln Val Ser
785                 790                 795                 800
Leu Thr Cys Leu Val Lys Gly Phe Tyr Pro Ser Asp Ile Ala Val Glu
                805                 810                 815
Trp Glu Ser Asn Gly Gln Pro Glu Asn Asn Tyr Lys Thr Thr Pro Pro
            820                 825                 830
Val Leu Asp Ser Asp Gly Ser Phe Phe Leu Tyr Ser Lys Leu Thr Val
        835                 840                 845
Asp Lys Ser Arg Trp Gln Gln Gly Asn Val Phe Ser Cys Ser Val Met
    850                 855                 860
His Glu Ala Leu His Asn His Tyr Thr Gln Lys Ser Leu Ser Leu Ser
865                 870                 875                 880
Pro Gly Lys

<210>  3
<211>  660
<212>  PRT
<213>  Artificial Sequence

<220>
<223>  cyno CEA-His protein sequence

<400>  3
Gln Leu Thr Ile Glu Ser Arg Pro Phe Asn Val Ala Glu Gly Lys Glu
1               5                   10                  15
Val Leu Leu Leu Ala His Asn Val Ser Gln Asn Leu Phe Gly Tyr Ile
            20                  25                  30
Trp Tyr Lys Gly Glu Arg Val Asp Ala Ser Arg Arg Ile Gly Ser Cys

35                  40                  45
Val Ile Arg Thr Gln Gln Ile Thr Pro Gly Pro Ala His Ser Gly Arg
    50                  55                  60
Glu Thr Ile Asp Phe Asn Ala Ser Leu Leu Ile Gln Asn Val Thr Gln
65                  70                  75                  80
Ser Asp Thr Gly Ser Tyr Thr Ile Gln Val Ile Lys Glu Asp Leu Val
                85                  90                  95
Asn Glu Glu Ala Thr Gly Gln Phe Arg Val Tyr Pro Glu Leu Pro Lys
            100                 105                 110
Pro Tyr Ile Thr Ser Asn Asn Ser Asn Pro Ile Glu Asp Lys Asp Ala
        115                 120                 125
Val Ala Leu Thr Cys Glu Pro Glu Thr Gln Asp Thr Thr Tyr Leu Trp
    130                 135                 140
Trp Val Asn Asn Gln Ser Leu Pro Val Ser Pro Arg Leu Glu Leu Ser
145                 150                 155                 160
Ser Asp Asn Arg Thr Leu Thr Val Phe Asn Ile Pro Arg Asn Asp Thr
                165                 170                 175
Thr Ser Tyr Lys Cys Glu Thr Gln Asn Pro Val Ser Val Arg Arg Ser
            180                 185                 190
Asp Pro Val Thr Leu Asn Val Leu Tyr Gly Pro Asp Ala Pro Thr Ile
        195                 200                 205
Ser Pro Leu Asn Thr Pro Tyr Arg Ala Gly Glu Tyr Leu Asn Leu Thr
    210                 215                 220
Cys His Ala Ala Ser Asn Pro Thr Ala Gln Tyr Phe Trp Phe Val Asn
225                 230                 235                 240
Gly Thr Phe Gln Gln Ser Thr Gln Glu Leu Phe Ile Pro Asn Ile Thr
                245                 250                 255
Val Asn Asn Ser Gly Ser Tyr Met Cys Gln Ala His Asn Ser Ala Thr
            260                 265                 270
Gly Leu Asn Arg Thr Thr Val Thr Ala Ile Thr Val Tyr Ala Glu Leu
        275                 280                 285
Pro Lys Pro Tyr Ile Thr Ser Asn Asn Ser Asn Pro Ile Glu Asp Lys
    290                 295                 300
Asp Ala Val Thr Leu Thr Cys Glu Pro Glu Thr Gln Asp Thr Thr Tyr
305                 310                 315                 320
Leu Trp Trp Val Asn Asn Gln Arg Leu Ser Val Ser Ser Arg Leu Glu
                325                 330                 335
Leu Ser Asn Asp Asn Arg Thr Leu Thr Val Phe Asn Ile Pro Arg Asn
            340                 345                 350
Asp Thr Thr Phe Tyr Glu Cys Glu Thr Gln Asn Pro Val Ser Val Arg
        355                 360                 365
Arg Ser Asp Pro Val Thr Leu Asn Val Leu Tyr Gly Pro Asp Ala Pro
    370                 375                 380
Thr Ile Ser Pro Leu Asn Thr Pro Tyr Arg Ala Gly Glu Asn Leu Asn
385                 390                 395                 400
Leu Ser Cys His Ala Ala Ser Asn Pro Ala Ala Gln Tyr Phe Trp Phe
                405                 410                 415
Val Asn Gly Thr Phe Gln Gln Ser Thr Gln Glu Leu Phe Ile Pro Asn
            420                 425                 430
Ile Thr Val Asn Asn Ser Gly Ser Tyr Met Cys Gln Ala His Asn Ser
        435                 440                 445
Ala Thr Gly Leu Asn Arg Thr Thr Val Thr Ala Ile Thr Val Tyr Val
    450                 455                 460
Glu Leu Pro Lys Pro Tyr Ile Ser Ser Asn Asn Ser Asn Pro Ile Glu
465                 470                 475                 480
Asp Lys Asp Ala Val Thr Leu Thr Cys Glu Pro Val Ala Glu Asn Thr
                485                 490                 495
Thr Tyr Leu Trp Trp Val Asn Asn Gln Ser Leu Ser Val Ser Pro Arg
            500                 505                 510
Leu Gln Leu Ser Asn Gly Asn Arg Ile Leu Thr Leu Leu Ser Val Thr
        515                 520                 525
Arg Asn Asp Thr Gly Pro Tyr Glu Cys Gly Ile Gln Asn Ser Glu Ser
    530                 535                 540

Ala Lys Arg Ser Asp Pro Val Thr Leu Asn Val Thr Tyr Gly Pro Asp
545                 550                 555                 560
Thr Pro Ile Ile Ser Pro Pro Asp Leu Ser Tyr Arg Ser Gly Ala Asn
                565                 570                 575
Leu Asn Leu Ser Cys His Ser Asp Ser Asn Pro Ser Pro Gln Tyr Ser
            580                 585                 590
Trp Leu Ile Asn Gly Thr Leu Arg Gln His Thr Gln Val Leu Phe Ile
        595                 600                 605
Ser Lys Ile Thr Ser Asn Asn Asn Gly Ala Tyr Ala Cys Phe Val Ser
    610                 615                 620
Asn Leu Ala Thr Gly Arg Asn Asn Ser Ile Val Lys Asn Ile Ser Val
625                 630                 635                 640
Ser Ser Gly Asp Ser Ala Pro Gly Ser Ser Gly Leu Ser Ala His His
                645                 650                 655
His His His His
            660

<210>  4
<211>  702
<212>  PRT
<213>  Artificial Sequence

<220>
<223>  human CEA protein sequence expressed on CHO cell surface
       (hCEA-CHO)


<400>  4
Met Glu Ser Pro Ser Ala Pro Pro His Arg Trp Cys Ile Pro Trp Gln
1               5                   10                  15
Arg Leu Leu Leu Thr Ala Ser Leu Leu Thr Phe Trp Asn Pro Pro Thr
            20                  25                  30
Thr Ala Lys Leu Thr Ile Glu Ser Thr Pro Phe Asn Val Ala Glu Gly
        35                  40                  45
Lys Glu Val Leu Leu Leu Val His Asn Leu Pro Gln His Leu Phe Gly
    50                  55                  60
Tyr Ser Trp Tyr Lys Gly Glu Arg Val Asp Gly Asn Arg Gln Ile Ile
65                  70                  75                  80
Gly Tyr Val Ile Gly Thr Gln Gln Ala Thr Pro Gly Pro Ala Tyr Ser
                85                  90                  95
Gly Arg Glu Ile Ile Tyr Pro Asn Ala Ser Leu Leu Ile Gln Asn Ile
            100                 105                 110
Ile Gln Asn Asp Thr Gly Phe Tyr Thr Leu His Val Ile Lys Ser Asp
        115                 120                 125
Leu Val Asn Glu Glu Ala Thr Gly Gln Phe Arg Val Tyr Pro Glu Leu
    130                 135                 140
Pro Lys Pro Ser Ile Ser Ser Asn Asn Ser Lys Pro Val Glu Asp Lys
145                 150                 155                 160
Asp Ala Val Ala Phe Thr Cys Glu Pro Glu Thr Gln Asp Ala Thr Tyr
                165                 170                 175
Leu Trp Trp Val Asn Asn Gln Ser Leu Pro Val Ser Pro Arg Leu Gln
            180                 185                 190
Leu Ser Asn Gly Asn Arg Thr Leu Thr Leu Phe Asn Val Thr Arg Asn
        195                 200                 205
Asp Thr Ala Ser Tyr Lys Cys Glu Thr Gln Asn Pro Val Ser Ala Arg
    210                 215                 220
Arg Ser Asp Ser Val Ile Leu Asn Val Leu Tyr Gly Pro Asp Ala Pro
225                 230                 235                 240
Thr Ile Ser Pro Leu Asn Thr Ser Tyr Arg Ser Gly Glu Asn Leu Asn
                245                 250                 255
Leu Ser Cys His Ala Ala Ser Asn Pro Pro Ala Gln Tyr Ser Trp Phe
            260                 265                 270
Val Asn Gly Thr Phe Gln Gln Ser Thr Gln Glu Leu Phe Ile Pro Asn

        275                 280                 285
Ile Thr Val Asn Asn Ser Gly Ser Tyr Thr Cys Gln Ala His Asn Ser
    290                 295                 300
Asp Thr Gly Leu Asn Arg Thr Thr Val Thr Thr Ile Thr Val Tyr Ala
305                 310                 315                 320
Glu Pro Pro Lys Pro Phe Ile Thr Ser Asn Asn Ser Asn Pro Val Glu
                325                 330                 335
Asp Glu Asp Ala Val Ala Leu Thr Cys Glu Pro Glu Ile Gln Asn Thr
            340                 345                 350
Thr Tyr Leu Trp Trp Val Asn Asn Gln Ser Leu Pro Val Ser Pro Arg
        355                 360                 365
Leu Gln Leu Ser Asn Asp Asn Arg Thr Leu Thr Leu Leu Ser Val Thr
    370                 375                 380
Arg Asn Asp Val Gly Pro Tyr Glu Cys Gly Ile Gln Asn Glu Leu Ser
385                 390                 395                 400
Val Asp His Ser Asp Pro Val Ile Leu Asn Val Leu Tyr Gly Pro Asp
                405                 410                 415
Asp Pro Thr Ile Ser Pro Ser Tyr Thr Tyr Tyr Arg Pro Gly Val Asn
            420                 425                 430
Leu Ser Leu Ser Cys His Ala Ala Ser Asn Pro Pro Ala Gln Tyr Ser
        435                 440                 445
Trp Leu Ile Asp Gly Asn Ile Gln Gln His Thr Gln Glu Leu Phe Ile
    450                 455                 460
Ser Asn Ile Thr Glu Lys Asn Ser Gly Leu Tyr Thr Cys Gln Ala Asn
465                 470                 475                 480
Asn Ser Ala Ser Gly His Ser Arg Thr Thr Val Lys Thr Ile Thr Val
                485                 490                 495
Ser Ala Glu Leu Pro Lys Pro Ser Ile Ser Ser Asn Asn Ser Lys Pro
            500                 505                 510
Val Glu Asp Lys Asp Ala Val Ala Phe Thr Cys Glu Pro Glu Ala Gln
        515                 520                 525
Asn Thr Thr Tyr Leu Trp Trp Val Asn Gly Gln Ser Leu Pro Val Ser
    530                 535                 540
Pro Arg Leu Gln Leu Ser Asn Gly Asn Arg Thr Leu Thr Leu Phe Asn
545                 550                 555                 560
Val Thr Arg Asn Asp Ala Arg Ala Tyr Val Cys Gly Ile Gln Asn Ser
                565                 570                 575
Val Ser Ala Asn Arg Ser Asp Pro Val Thr Leu Asp Val Leu Tyr Gly
            580                 585                 590
Pro Asp Thr Pro Ile Ile Ser Pro Pro Asp Ser Ser Tyr Leu Ser Gly
        595                 600                 605
Ala Asn Leu Asn Leu Ser Cys His Ser Ala Ser Asn Pro Ser Pro Gln
    610                 615                 620
Tyr Ser Trp Arg Ile Asn Gly Ile Pro Gln Gln His Thr Gln Val Leu
625                 630                 635                 640
Phe Ile Ala Lys Ile Thr Pro Asn Asn Asn Gly Thr Tyr Ala Cys Phe
                645                 650                 655
Val Ser Asn Leu Ala Thr Gly Arg Asn Asn Ser Ile Val Lys Ser Ile
            660                 665                 670
Thr Val Ser Ala Ser Gly Thr Ser Pro Gly Leu Ser Ala Gly Ala Thr
        675                 680                 685
Val Gly Ile Met Ile Gly Val Leu Val Gly Val Ala Leu Ile
    690                 695                 700

<210> 5
<211> 690
<212> PRT
<213> Artificial Sequence

<220>
<223> cyno CEA protein sequence expresssed on CHO cell surface
(cynoCEA-CHO)

<400> 5
Met Glu Phe Gly Leu Ser Trp Leu Phe Leu Val Ala Ile Leu Lys Gly
1               5                   10                  15
Val Gln Cys Gln Leu Thr Ile Glu Ser Arg Pro Phe Asn Val Ala Glu
            20                  25                  30
Gly Lys Glu Val Leu Leu Leu Ala His Asn Val Ser Gln Asn Leu Phe
        35                  40                  45
Gly Tyr Ile Trp Tyr Lys Gly Glu Arg Val Asp Ala Ser Arg Arg Ile
    50                  55                  60
Gly Ser Cys Val Ile Arg Thr Gln Gln Ile Thr Pro Gly Pro Ala His
65                  70                  75                  80
Ser Gly Arg Glu Thr Ile Asp Phe Asn Ala Ser Leu Leu Ile Gln Asn
                85                  90                  95
Val Thr Gln Ser Asp Thr Gly Ser Tyr Thr Ile Gln Val Ile Lys Glu
            100                 105                 110
Asp Leu Val Asn Glu Glu Ala Thr Gly Gln Phe Arg Val Tyr Pro Glu
        115                 120                 125
Leu Pro Lys Pro Tyr Ile Thr Ser Asn Asn Ser Asn Pro Ile Glu Asp
    130                 135                 140
Lys Asp Ala Val Ala Leu Thr Cys Glu Pro Glu Thr Gln Asp Thr Thr
145                 150                 155                 160
Tyr Leu Trp Trp Val Asn Asn Gln Ser Leu Pro Val Ser Pro Arg Leu
                165                 170                 175
Glu Leu Ser Ser Asp Asn Arg Thr Leu Thr Val Phe Asn Ile Pro Arg
            180                 185                 190
Asn Asp Thr Thr Ser Tyr Lys Cys Glu Thr Gln Asn Pro Val Ser Val
        195                 200                 205
Arg Arg Ser Asp Pro Val Thr Leu Asn Val Leu Tyr Gly Pro Asp Ala
    210                 215                 220
Pro Thr Ile Ser Pro Leu Asn Thr Pro Tyr Arg Ala Gly Glu Tyr Leu
225                 230                 235                 240
Asn Leu Thr Cys His Ala Ala Ser Asn Pro Thr Ala Gln Tyr Phe Trp
                245                 250                 255
Phe Val Asn Gly Thr Phe Gln Gln Ser Thr Gln Glu Leu Phe Ile Pro
            260                 265                 270
Asn Ile Thr Val Asn Asn Ser Gly Ser Tyr Met Cys Gln Ala His Asn
        275                 280                 285
Ser Ala Thr Gly Leu Asn Arg Thr Thr Val Thr Ala Ile Thr Val Tyr
    290                 295                 300
Ala Glu Leu Pro Lys Pro Tyr Ile Thr Ser Asn Asn Ser Asn Pro Ile
305                 310                 315                 320
Glu Asp Lys Asp Ala Val Thr Leu Thr Cys Glu Pro Glu Thr Gln Asp
                325                 330                 335
Thr Thr Tyr Leu Trp Trp Val Asn Asn Gln Arg Leu Ser Val Ser Ser
            340                 345                 350
Arg Leu Glu Leu Ser Asn Asp Asn Arg Thr Leu Thr Val Phe Asn Ile
        355                 360                 365
Pro Arg Asn Asp Thr Thr Phe Tyr Glu Cys Glu Thr Gln Asn Pro Val
    370                 375                 380
Ser Val Arg Arg Ser Asp Pro Val Thr Leu Asn Val Leu Tyr Gly Pro
385                 390                 395                 400
Asp Ala Pro Thr Ile Ser Pro Leu Asn Thr Pro Tyr Arg Ala Gly Glu
                405                 410                 415
Asn Leu Asn Leu Ser Cys His Ala Ala Ser Asn Pro Ala Ala Gln Tyr
            420                 425                 430
Phe Trp Phe Val Asn Gly Thr Phe Gln Gln Ser Thr Gln Glu Leu Phe
        435                 440                 445
Ile Pro Asn Ile Thr Val Asn Asn Ser Gly Ser Tyr Met Cys Gln Ala
    450                 455                 460
His Asn Ser Ala Thr Gly Leu Asn Arg Thr Thr Val Thr Ala Ile Thr
465                 470                 475                 480
Val Tyr Val Glu Leu Pro Lys Pro Tyr Ile Ser Ser Asn Asn Ser Asn

485                 490                 495
Pro Ile Glu Asp Lys Asp Ala Val Thr Leu Thr Cys Glu Pro Val Ala
            500                 505                 510
Glu Asn Thr Thr Tyr Leu Trp Trp Val Asn Asn Gln Ser Leu Ser Val
        515                 520                 525
Ser Pro Arg Leu Gln Leu Ser Asn Gly Asn Arg Ile Leu Thr Leu Leu
    530                 535                 540
Ser Val Thr Arg Asn Asp Thr Gly Pro Tyr Glu Cys Gly Ile Gln Asn
545                 550                 555                 560
Ser Glu Ser Ala Lys Arg Ser Asp Pro Val Thr Leu Asn Val Thr Tyr
                565                 570                 575
Gly Pro Asp Thr Pro Ile Ile Ser Pro Pro Asp Leu Ser Tyr Arg Ser
            580                 585                 590
Gly Ala Asn Leu Asn Leu Ser Cys His Ser Asp Ser Asn Pro Ser Pro
        595                 600                 605
Gln Tyr Ser Trp Leu Ile Asn Gly Thr Leu Arg Gln His Thr Gln Val
    610                 615                 620
Leu Phe Ile Ser Lys Ile Thr Ser Asn Asn Asn Gly Ala Tyr Ala Cys
625                 630                 635                 640
Phe Val Ser Asn Leu Ala Thr Gly Arg Asn Asn Ser Ile Val Lys Asn
                645                 650                 655
Ile Ser Val Ser Ser Gly Asp Ser Ala Pro Gly Ser Ser Gly Leu Ser
            660                 665                 670
Ala Arg Ala Thr Val Gly Ile Ile Ile Gly Met Leu Val Gly Val Ala
        675                 680                 685
Leu Met
    690

<210>  6
<211>  526
<212>  PRT
<213>  Artificial Sequence

<220>
<223>  human CEACAM1 protein sequence expresssed on CHO cell
       surface (CEACAM1-CHO)


<400>  6
Met Gly His Leu Ser Ala Pro Leu His Arg Val Arg Val Pro Trp Gln
1               5                   10                  15
Gly Leu Leu Leu Thr Ala Ser Leu Leu Thr Phe Trp Asn Pro Pro Thr
            20                  25                  30
Thr Ala Gln Leu Thr Thr Glu Ser Met Pro Phe Asn Val Ala Glu Gly
        35                  40                  45
Lys Glu Val Leu Leu Leu Val His Asn Leu Pro Gln Gln Leu Phe Gly
    50                  55                  60
Tyr Ser Trp Tyr Lys Gly Glu Arg Val Asp Gly Asn Arg Gln Ile Val
65                  70                  75                  80
Gly Tyr Ala Ile Gly Thr Gln Gln Ala Thr Pro Gly Pro Ala Asn Ser
                85                  90                  95
Gly Arg Glu Thr Ile Tyr Pro Asn Ala Ser Leu Leu Ile Gln Asn Val
            100                 105                 110
Thr Gln Asn Asp Thr Gly Phe Tyr Thr Leu Gln Val Ile Lys Ser Asp
        115                 120                 125
Leu Val Asn Glu Glu Ala Thr Gly Gln Phe His Val Tyr Pro Glu Leu
    130                 135                 140
Pro Lys Pro Ser Ile Ser Ser Asn Asn Ser Asn Pro Val Glu Asp Lys
145                 150                 155                 160
Asp Ala Val Ala Phe Thr Cys Glu Pro Glu Thr Gln Asp Thr Thr Tyr
                165                 170                 175
Leu Trp Trp Ile Asn Asn Gln Ser Leu Pro Val Ser Pro Arg Leu Gln
            180                 185                 190

Leu Ser Asn Gly Asn Arg Thr Leu Thr Leu Leu Ser Val Thr Arg Asn
        195                 200                 205
Asp Thr Gly Pro Tyr Glu Cys Glu Ile Gln Asn Pro Val Ser Ala Asn
    210                 215                 220
Arg Ser Asp Pro Val Thr Leu Asn Val Thr Tyr Gly Pro Asp Thr Pro
225                 230                 235                 240
Thr Ile Ser Pro Ser Asp Thr Tyr Tyr Arg Pro Gly Ala Asn Leu Ser
                245                 250                 255
Leu Ser Cys Tyr Ala Ala Ser Asn Pro Pro Ala Gln Tyr Ser Trp Leu
            260                 265                 270
Ile Asn Gly Thr Phe Gln Gln Ser Thr Gln Glu Leu Phe Ile Pro Asn
        275                 280                 285
Ile Thr Val Asn Asn Ser Gly Ser Tyr Thr Cys His Ala Asn Asn Ser
    290                 295                 300
Val Thr Gly Cys Asn Arg Thr Thr Val Lys Thr Ile Ile Val Thr Glu
305                 310                 315                 320
Leu Ser Pro Val Val Ala Lys Pro Gln Ile Lys Ala Ser Lys Thr Thr
                325                 330                 335
Val Thr Gly Asp Lys Asp Ser Val Asn Leu Thr Cys Ser Thr Asn Asp
            340                 345                 350
Thr Gly Ile Ser Ile Arg Trp Phe Phe Lys Asn Gln Ser Leu Pro Ser
        355                 360                 365
Ser Glu Arg Met Lys Leu Ser Gln Gly Asn Thr Thr Leu Ser Ile Asn
    370                 375                 380
Pro Val Lys Arg Glu Asp Ala Gly Thr Tyr Trp Cys Glu Val Phe Asn
385                 390                 395                 400
Pro Ile Ser Lys Asn Gln Ser Asp Pro Ile Met Leu Asn Val Asn Tyr
                405                 410                 415
Asn Ala Leu Pro Gln Glu Asn Gly Leu Ser Pro Gly Ala Ile Ala Gly
            420                 425                 430
Ile Val Ile Gly Val Val Ala Leu Val Ala Leu Ile Ala Val Ala Leu
        435                 440                 445
Ala Cys Phe Leu His Phe Gly Lys Thr Gly Arg Ala Ser Asp Gln Arg
    450                 455                 460
Asp Leu Thr Glu His Lys Pro Ser Val Ser Asn His Thr Gln Asp His
465                 470                 475                 480
Ser Asn Asp Pro Pro Asn Lys Met Asn Glu Val Thr Tyr Ser Thr Leu
                485                 490                 495
Asn Phe Glu Ala Gln Gln Pro Thr Gln Pro Thr Ser Ala Ser Pro Ser
            500                 505                 510
Leu Thr Ala Thr Glu Ile Ile Tyr Ser Glu Val Lys Lys Gln
        515                 520                 525

<210>  7
<211>  120
<212>  PRT
<213>  Mus musculus

<220>
<223>  mAb47 heavy chain variable region



<400>  7
Gln Ile Gln Leu Val Gln Ser Gly Pro Glu Leu Lys Lys Pro Gly Glu
1               5                   10                  15
Thr Val Lys Ile Ser Cys Lys Ala Ser Gly Tyr Thr Phe Thr Thr Tyr
            20                  25                  30
Gly Met Ser Trp Val Lys Gln Ala Pro Gly Lys Gly Leu Lys Trp Met
        35                  40                  45
Gly Trp Ile Asn Thr Tyr Ser Gly Val Pro Thr Tyr Ala Asp Asp Phe
    50                  55                  60
Lys Gly Arg Phe Ala Phe Ser Leu Glu Thr Ser Ala Ser Thr Ala Tyr
65                  70                  75                  80

Leu Gln Ile Asn Asn Leu Lys Asn Glu Asp Thr Ala Thr Tyr Phe Cys
                85                  90                  95
Ala Arg Arg Gly Asn Tyr Gly Arg Trp Asp Phe Asp Val Trp Gly Thr
            100                 105                 110
Gly Thr Thr Val Thr Val Ser Ser
        115                 120

<210>  8
<211>  108
<212>  PRT
<213>  Mus musculus

<220>
<223>  mAb47 light chain variable region


<400>  8
Glu Ile Val Leu Thr Gln Ser Pro Ala Leu Met Ala Ala Ser Pro Gly
1               5                   10                  15
Glu Lys Val Thr Ile Thr Cys Ser Val Ser Ser Thr Ile Ser Ser Ser
            20                  25                  30
Asn Leu His Trp Tyr Gln Gln Lys Ser Glu Thr Ser Pro Lys Pro Trp
        35                  40                  45
Ile Tyr Gly Thr Ser Asn Leu Ala Ser Gly Val Pro Val Arg Phe Ser
    50                  55                  60
Gly Ser Gly Ser Gly Thr Ser Tyr Ser Leu Thr Ile Ser Ser Met Glu
65                  70                  75                  80
Ala Glu Asp Ala Ala Thr Tyr Tyr Cys Gln Gln Trp Ser Ile Tyr Pro
                85                  90                  95
Leu Thr Phe Gly Ala Gly Thr Lys Leu Glu Leu Lys
            100                 105

<210>  9
<211>  119
<212>  PRT
<213>  Mus musculus

<220>
<223>  mAb63 heavy chain variable region


<400>  9
Glu Val Gln Leu Gln Gln Ser Gly Pro Glu Leu Val Lys Pro Gly Ala
1               5                   10                  15
Ser Val Lys Met Ser Cys Lys Ala Ser Gly Tyr Thr Phe Thr Asp Phe
            20                  25                  30
Tyr Met Asn Trp Val Arg Gln Ser His Gly Lys Ser Leu Glu Trp Ile
        35                  40                  45
Gly Asp Ile Phe Pro Lys Asn Gly Asn Thr Asp Tyr Asn Arg Lys Phe
    50                  55                  60
Lys Asp Lys Ala Thr Leu Thr Val Asp Lys Ser Ser Asn Thr Val Tyr
65                  70                  75                  80
Met Glu Phe Arg Ser Leu Thr Leu Glu Asp Ser Ala Ile Tyr Phe Cys
                85                  90                  95
Ala Arg Ser Gly Tyr Gly Asn Tyr Val Phe Asp Tyr Trp Gly Gln Gly
            100                 105                 110
Thr Ile Phe Thr Val Ser Ser
        115

<210>  10
<211>  107
<212>  PRT
<213>  Mus musculus

<220>
<223>  mAb63 light chain variable region


<400>  10
Asp Val Gln Met Thr Gln Thr Pro Ser Ala Leu Ser Ala Ser Leu Gly
1               5                   10                  15
Asp Arg Val Thr Ile Ser Cys Arg Thr Ser Gln Asp Ile Asn Ile Tyr
            20                  25                  30
Leu Asn Trp Tyr Gln Gln Lys Pro Asp Gly Thr Val Lys Leu Leu Ile
        35                  40                  45
Tyr Tyr Arg Ser Gly Leu Leu Ser Gly Val Pro Ser Arg Phe Ser Gly
    50                  55                  60
Ser Gly Ser Gly Thr Asp Tyr Ser Leu Thr Ile Ser Asn Leu Glu Pro
65                  70                  75                  80
Glu Asp Ile Ala Thr Tyr Phe Cys Gln Gln Gly Asn Thr Leu Pro Pro
                85                  90                  95
Thr Phe Gly Gly Gly Thr Lys Leu Glu Ile Lys
            100                 105

<210>  11
<211>  117
<212>  PRT
<213>  Mus musculus

<220>
<223>  mAb67 heavy chain variable region


<400>  11
Glu Val Gln Leu Gln Gln Ser Gly Pro Glu Leu Val Lys Pro Gly Ala
1               5                   10                  15
Ser Val Lys Ile Tyr Cys Lys Ala Ser Gly Tyr Thr Phe Thr Asp Tyr
            20                  25                  30
His Met Asn Trp Val Lys Gln Ser His Gly Lys Ser Leu Glu Trp Ile
        35                  40                  45
Gly Asp Ile Asn Pro Asp Ile Gly Gly Thr Ser Tyr Asn Gln Asn Phe
    50                  55                  60
Lys Gly Lys Ala Thr Leu Thr Val Asp Lys Ser Ser Ser Thr Ala Tyr
65                  70                  75                  80
Met Glu Leu Arg Ser Leu Thr Ser Glu Asp Ser Ala Val Tyr Tyr Cys
                85                  90                  95
Ser Arg Trp Asp Phe Asp Ser Phe Ala Asn Trp Gly Gln Gly Thr Leu
            100                 105                 110
Val Thr Val Ser Ala
        115

<210>  12
<211>  111
<212>  PRT
<213>  Mus musculus

<220>
<223>  mAb67 light chain variable region


<400>  12
Asp Ile Val Met Thr Gln Ser Pro Ala Ser Leu Ala Met Ser Leu Gly
1               5                   10                  15
Lys Arg Ala Thr Ile Ser Cys Arg Ala Ser Glu Ser Val Ser Ile Ile
            20                  25                  30
Gly Thr Asn Leu Ile His Trp Tyr Gln Gln Lys Pro Gly Gln Pro Pro

35                  40                  45
Lys Leu Leu Ile Tyr His Ala Ser Asn Leu Glu Thr Gly Val Pro Ala
    50                  55                  60
Arg Phe Ser Gly Ser Gly Ser Gly Ala Asp Phe Thr Leu Thr Ile Asp
65                  70                  75                  80
Pro Val Glu Gly Asp Asp Val Ala Leu Tyr Tyr Cys Leu Gln Ser Arg
                85                  90                  95
Lys Ile Pro Tyr Thr Phe Gly Gly Gly Thr Lys Met Glu Ile Lys
            100                 105                 110

<210>  13
<211>  122
<212>  PRT
<213>  Mus musculus

<220>
<223>  mAb103 heavy chain variable region


<400>  13
Gln Ile Gln Leu Val Gln Ser Gly Pro Glu Leu Lys Lys Pro Gly Glu
1               5                   10                  15
Thr Val Lys Ile Ser Cys Lys Ala Ser Gly Tyr Thr Phe Thr Thr Tyr
            20                  25                  30
Gly Val Ile Trp Val Lys Gln Ala Pro Gly Lys Gly Leu Lys Trp Met
        35                  40                  45
Gly Trp Ile Asn Thr Tyr Ser Gly Val Pro Thr Tyr Ala Asp Asp Phe
    50                  55                  60
Lys Gly Arg Phe Ala Phe Ser Leu Glu Thr Ser Ala Ser Thr Ala Phe
65                  70                  75                  80
Leu Gln Ile Asn Asn Leu Lys Asn Glu Asp Thr Ala Thr Tyr Phe Cys
                85                  90                  95
Ala Arg Lys Lys Thr Leu Thr Thr Val Thr Pro Trp Phe Ala Tyr Trp
            100                 105                 110
Gly Gln Gly Thr Leu Val Thr Val Ser Ala
        115                 120

<210>  14
<211>  115
<212>  PRT
<213>  Mus musculus

<220>
<223>  mAb103 light chain variable region


<400>  14
Asp Leu Val Val Thr Gln Ser Ser Ser Ala Ser Phe Ser Leu Gly Ala
1               5                   10                  15
Ser Ala Lys Leu Thr Cys Thr Leu Ser Ser Gln His Ser Thr Tyr Thr
            20                  25                  30
Ile Glu Trp Tyr Gln Gln Gln Pro Leu Lys Pro Pro Lys Tyr Val Met
        35                  40                  45
Glu Leu Lys Lys Asp Gly Ser His Ser Thr Gly Asp Gly Ile Pro Asp
    50                  55                  60
Arg Phe Ser Gly Ser Ser Ser Gly Ala Asp Arg Tyr Leu Thr Ile Ser
65                  70                  75                  80
Asn Ile Gln Pro Glu Asp Glu Ala Ile Tyr Ile Cys Gly Val Gly Asn
                85                  90                  95
Thr Ile Lys Glu Gln Phe Val Tyr Val Phe Gly Gly Gly Thr Lys Val
            100                 105                 110
Thr Val Leu
        115

<210>  15
<211>  5
<212>  PRT
<213>  Mus musculus

<220>
<223>  mAb47 heavy chain HCDR1 sequence


<400>  15
Thr Tyr Gly Met Ser
1               5

<210>  16
<211>  17
<212>  PRT
<213>  Mus musculus

<220>
<223>  mAb47/mAb103 heavy chain HCDR2 sequence


<400>  16
Trp Ile Asn Thr Tyr Ser Gly Val Pro Thr Tyr Ala Asp Asp Phe Lys
1               5                   10                  15
Gly


<210>  17
<211>  11
<212>  PRT
<213>  Mus musculus

<220>
<223>  mAb47 heavy chain HCDR3 sequence


<400>  17
Arg Gly Asn Tyr Gly Arg Trp Asp Phe Asp Val
1               5                   10

<210>  18
<211>  12
<212>  PRT
<213>  Mus musculus

<220>
<223>  mAb47 light chain LCDR1 sequence


<400>  18
Ser Val Ser Ser Thr Ile Ser Ser Ser Asn Leu His
1               5                   10

<210>  19
<211>  7
<212>  PRT
<213>  Mus musculus

<220>
<223>  mAb47 light chain LCDR2 sequence

<400>  19
Gly Thr Ser Asn Leu Ala Ser
1               5

<210>  20
<211>  9
<212>  PRT
<213>  Mus musculus

<220>
<223>  mAb47 light chain LCDR3 sequence


<400>  20
Gln Gln Trp Ser Ile Tyr Pro Leu Thr
1               5

<210>  21
<211>  5
<212>  PRT
<213>  Mus musculus

<220>
<223>  mAb63 heavy chain HCDR1 sequence


<400>  21
Asp Phe Tyr Met Asn
1               5

<210>  22
<211>  17
<212>  PRT
<213>  Mus musculus

<220>
<223>  mAb63 heavy chain HCDR2 sequence


<400>  22
Asp Ile Phe Pro Lys Asn Gly Asn Thr Asp Tyr Asn Arg Lys Phe Lys
1               5                   10                  15
Asp


<210>  23
<211>  10
<212>  PRT
<213>  Mus musculus

<220>
<223>  mAb63 heavy chain HCDR3 sequence


<400>  23
Ser Gly Tyr Gly Asn Tyr Val Phe Asp Tyr
1               5                   10

<210>  24
<211>  11
<212>  PRT
<213>  Mus musculus

<220>
<223>  mAb63 light chain LCDR1 sequence


<400>  24
Arg Thr Ser Gln Asp Ile Asn Ile Tyr Leu Asn
1               5                   10

<210>  25
<211>  7
<212>  PRT
<213>  Mus musculus

<220>
<223>  mAb63 light chain LCDR2 sequence


<400>  25
Tyr Arg Ser Gly Leu Leu Ser
1               5

<210>  26
<211>  9
<212>  PRT
<213>  Mus musculus

<220>
<223>  mAb63 light chain LCDR3 sequence


<400>  26
Gln Gln Gly Asn Thr Leu Pro Pro Thr
1               5

<210>  27
<211>  5
<212>  PRT
<213>  Mus musculus

<220>
<223>  mAb67 heavy chain HCDR1 sequence


<400>  27
Asp Tyr His Met Asn
1               5

<210>  28
<211>  17
<212>  PRT
<213>  Mus musculus

<220>
<223>  mAb67 heavy chain HCDR2 sequence


<400>  28
Asp Ile Asn Pro Asp Ile Gly Gly Thr Ser Tyr Asn Gln Asn Phe Lys
1               5                   10                  15
Gly

<210>  29
<211>  8
<212>  PRT
<213>  Mus musculus

<220>
<223>  mAb67 heavy chain HCDR3 sequence


<400>  29
Trp Asp Phe Asp Ser Phe Ala Asn
1               5

<210>  30
<211>  15
<212>  PRT
<213>  Mus musculus

<220>
<223>  mAb67 light chain LCDR1 sequence


<400>  30
Arg Ala Ser Glu Ser Val Ser Ile Ile Gly Thr Asn Leu Ile His
1               5                   10                  15

<210>  31
<211>  7
<212>  PRT
<213>  Mus musculus

<220>
<223>  mAb67 light chain LCDR2 sequence


<400>  31
His Ala Ser Asn Leu Glu Thr
1               5

<210>  32
<211>  9
<212>  PRT
<213>  Mus musculus

<220>
<223>  mAb67 light chain LCDR3 sequence


<400>  32
Leu Gln Ser Arg Lys Ile Pro Tyr Thr
1               5

<210>  33
<211>  5
<212>  PRT
<213>  Mus musculus

<220>
<223>  mAb103 heavy chain HCDR1 sequence


<400>  33
Thr Tyr Gly Val Ile

1               5

<210>  34
<211>  13
<212>  PRT
<213>  Mus musculus

<220>
<223>  mAb103 heavy chain HCDR3 sequence


<400>  34
Lys Lys Thr Leu Thr Thr Val Thr Pro Trp Phe Ala Tyr
1               5                   10

<210>  35
<211>  12
<212>  PRT
<213>  Mus musculus

<220>
<223>  mAb103 light chain LCDR1 sequence


<400>  35
Thr Leu Ser Ser Gln His Ser Thr Tyr Thr Ile Glu
1               5                   10

<210>  36
<211>  11
<212>  PRT
<213>  Mus musculus

<220>
<223>  mAb103 light chain LCDR2 sequence


<400>  36
Leu Lys Lys Asp Gly Ser His Ser Thr Gly Asp
1               5                   10

<210>  37
<211>  13
<212>  PRT
<213>  Mus musculus

<220>
<223>  mAb103 light chain LCDR3 sequence


<400>  37
Gly Val Gly Asn Thr Ile Lys Glu Gln Phe Val Tyr Val
1               5                   10

<210>  38
<211>  17
<212>  PRT
<213>  Artificial Sequence

<220>
<223>  mAb47/mAb103 heavy chain HCDR2  mutation (D61S) sequence

<400>  38
Trp Ile Asn Thr Tyr Ser Gly Val Pro Thr Tyr Ala Ser Asp Phe Lys
1               5                   10                  15
Gly


<210>  39
<211>  120
<212>  PRT
<213>  Artificial Sequence

<220>
<223>  mAb47 humanized antibody heavy chain variable region h47VH1
       sequence


<400>  39
Glu Val Gln Leu Val Gln Ser Gly Ser Glu Leu Lys Lys Pro Gly Ala
1               5                   10                  15
Ser Val Lys Val Ser Cys Lys Ala Ser Gly Tyr Thr Phe Thr Thr Tyr
            20                  25                  30
Gly Met Ser Trp Val Arg Gln Ala Pro Gly Gln Gly Leu Glu Trp Met
        35                  40                  45
Gly Trp Ile Asn Thr Tyr Ser Gly Val Pro Thr Tyr Ala Asp Asp Phe
    50                  55                  60
Lys Gly Arg Phe Val Phe Ser Leu Asp Thr Ser Val Ser Thr Ala Tyr
65                  70                  75                  80
Leu Gln Ile Ser Ser Leu Lys Ala Glu Asp Thr Ala Val Tyr Tyr Cys
                85                  90                  95
Ala Arg Arg Gly Asn Tyr Gly Arg Trp Asp Phe Asp Val Trp Gly Gln
            100                 105                 110
Gly Thr Thr Val Thr Val Ser Ser
        115                 120

<210>  40
<211>  120
<212>  PRT
<213>  Artificial Sequence

<220>
<223>  mAb47 humanized antibody heavy chain variable region h47VH2
       sequence


<400>  40
Glu Val Gln Leu Val Gln Ser Gly Ser Glu Leu Lys Lys Pro Gly Ala
1               5                   10                  15
Ser Val Lys Val Ser Cys Lys Ala Ser Gly Tyr Thr Phe Thr Thr Tyr
            20                  25                  30
Gly Met Ser Trp Val Arg Gln Ala Pro Gly Gln Gly Leu Lys Trp Met
        35                  40                  45
Gly Trp Ile Asn Thr Tyr Ser Gly Val Pro Thr Tyr Ala Asp Asp Phe
    50                  55                  60
Lys Gly Arg Phe Val Phe Ser Leu Asp Thr Ser Val Ser Thr Ala Tyr
65                  70                  75                  80
Leu Gln Ile Ser Ser Leu Lys Ala Glu Asp Thr Ala Val Tyr Tyr Cys
                85                  90                  95
Ala Arg Arg Gly Asn Tyr Gly Arg Trp Asp Phe Asp Val Trp Gly Gln
            100                 105                 110
Gly Thr Thr Val Thr Val Ser Ser
        115                 120

<210>  41

<211>  120
<212>  PRT
<213>  Artificial Sequence

<220>
<223>  mAb47 humanized antibody heavy chain variable region h47VH3
      sequence

<400>  41
Glu Val Gln Leu Val Gln Ser Gly Ser Glu Leu Lys Lys Pro Gly Ala
1               5                   10                  15
Ser Val Lys Val Ser Cys Lys Ala Ser Gly Tyr Thr Phe Thr Thr Tyr
            20                  25                  30
Gly Met Ser Trp Val Lys Gln Ala Pro Gly Gln Gly Leu Lys Trp Met
        35                  40                  45
Gly Trp Ile Asn Thr Tyr Ser Gly Val Pro Thr Tyr Ala Asp Asp Phe
    50                  55                  60
Lys Gly Arg Phe Val Phe Ser Leu Asp Thr Ser Val Ser Thr Ala Tyr
65                  70                  75                  80
Leu Gln Ile Ser Ser Leu Lys Ala Glu Asp Thr Ala Val Tyr Tyr Cys
                85                  90                  95
Ala Arg Arg Gly Asn Tyr Gly Arg Trp Asp Phe Asp Val Trp Gly Gln
            100                 105                 110
Gly Thr Thr Val Thr Val Ser Ser
        115                 120

<210>  42
<211>  120
<212>  PRT
<213>  Artificial Sequence

<220>
<223>  mAb47 humanized antibody heavy chain variable region h47VH4
      sequence

<400>  42
Glu Val Gln Leu Val Gln Ser Gly Ser Glu Leu Lys Lys Pro Gly Ala
1               5                   10                  15
Ser Val Lys Val Ser Cys Lys Ala Ser Gly Tyr Thr Phe Thr Thr Tyr
            20                  25                  30
Gly Met Ser Trp Val Lys Gln Ala Pro Gly Gln Gly Leu Lys Trp Met
        35                  40                  45
Gly Trp Ile Asn Thr Tyr Ser Gly Val Pro Thr Tyr Ala Ser Asp Phe
    50                  55                  60
Lys Gly Arg Phe Val Phe Ser Leu Asp Thr Ser Val Ser Thr Ala Tyr
65                  70                  75                  80
Leu Gln Ile Ser Ser Leu Lys Ala Glu Asp Thr Ala Val Tyr Tyr Cys
                85                  90                  95
Ala Arg Arg Gly Asn Tyr Gly Arg Trp Asp Phe Asp Val Trp Gly Gln
            100                 105                 110
Gly Thr Thr Val Thr Val Ser Ser
        115                 120

<210>  43
<211>  108
<212>  PRT
<213>  Artificial Sequence

<220>
<223>  mAb47 humanized antibody light chain variable region h47VL1
      sequence

<400>  43
Glu Ile Val Leu Thr Gln Ser Pro Asp Phe Gln Ser Val Thr Pro Lys
1               5                   10                  15
Glu Lys Val Thr Ile Thr Cys Ser Val Ser Ser Thr Ile Ser Ser Ser
            20                  25                  30
Asn Leu His Trp Tyr Gln Gln Lys Pro Asp Gln Ser Pro Lys Leu Leu
        35                  40                  45
Ile Lys Gly Thr Ser Asn Leu Ala Ser Gly Val Pro Ser Arg Phe Ser
    50                  55                  60
Gly Ser Gly Ser Gly Thr Asp Phe Thr Leu Thr Ile Asn Ser Leu Glu
65                  70                  75                  80
Ala Glu Asp Ala Ala Thr Tyr Tyr Cys Gln Gln Trp Ser Ile Tyr Pro
                85                  90                  95
Leu Thr Phe Gly Gln Gly Thr Lys Leu Glu Ile Lys
            100                 105

<210>  44
<211>  108
<212>  PRT
<213>  Artificial Sequence

<220>
<223>  mAb47 humanized antibody light chain variable region h47VL2
       sequence


<400>  44
Glu Ile Val Leu Thr Gln Ser Pro Asp Phe Gln Ser Val Thr Pro Lys
1               5                   10                  15
Glu Lys Val Thr Ile Thr Cys Ser Val Ser Ser Thr Ile Ser Ser Ser
            20                  25                  30
Asn Leu His Trp Tyr Gln Gln Lys Pro Asp Gln Ser Pro Lys Pro Leu
        35                  40                  45
Ile Tyr Gly Thr Ser Asn Leu Ala Ser Gly Val Pro Ser Arg Phe Ser
    50                  55                  60
Gly Ser Gly Ser Gly Thr Asp Tyr Thr Leu Thr Ile Asn Ser Leu Glu
65                  70                  75                  80
Ala Glu Asp Ala Ala Thr Tyr Tyr Cys Gln Gln Trp Ser Ile Tyr Pro
                85                  90                  95
Leu Thr Phe Gly Gln Gly Thr Lys Leu Glu Ile Lys
            100                 105

<210>  45
<211>  108
<212>  PRT
<213>  Artificial Sequence

<220>
<223>  mAb47 humanized antibody light chain variable region h47VL3
       sequence


<400>  45
Glu Ile Val Leu Thr Gln Ser Pro Asp Phe Gln Ser Val Thr Pro Lys
1               5                   10                  15
Glu Lys Val Thr Ile Thr Cys Ser Val Ser Ser Thr Ile Ser Ser Ser
            20                  25                  30
Asn Leu His Trp Tyr Gln Gln Lys Pro Asp Gln Ser Pro Lys Pro Trp
        35                  40                  45
Ile Tyr Gly Thr Ser Asn Leu Ala Ser Gly Val Pro Ser Arg Phe Ser
    50                  55                  60

Gly Ser Gly Ser Gly Thr Asp Tyr Thr Leu Thr Ile Asn Ser Leu Glu
65                  70                  75                  80
Ala Glu Asp Ala Ala Thr Tyr Tyr Cys Gln Gln Trp Ser Ile Tyr Pro
                85                  90                  95
Leu Thr Phe Gly Gln Gly Thr Lys Leu Glu Ile Lys
            100                 105

<210>  46
<211>  108
<212>  PRT
<213>  Artificial Sequence

<220>
<223>  mAb47 humanized antibody light chain variable region h47VL4
       sequence

<400>  46
Glu Ile Val Leu Thr Gln Ser Pro Asp Phe Gln Ser Val Thr Pro Lys
1               5                   10                  15
Glu Lys Val Thr Ile Thr Cys Ser Val Ser Ser Thr Ile Ser Ser Ser
            20                  25                  30
Asn Leu His Trp Tyr Gln Gln Lys Pro Asp Gln Ser Pro Lys Pro Trp
        35                  40                  45
Ile Tyr Gly Thr Ser Asn Leu Ala Ser Gly Val Pro Ser Arg Phe Ser
    50                  55                  60
Gly Ser Gly Ser Gly Thr Ser Tyr Thr Leu Thr Ile Asn Ser Leu Glu
65                  70                  75                  80
Ala Glu Asp Ala Ala Thr Tyr Tyr Cys Gln Gln Trp Ser Ile Tyr Pro
                85                  90                  95
Leu Thr Phe Gly Gln Gly Thr Lys Leu Glu Ile Lys
            100                 105

<210>  47
<211>  17
<212>  PRT
<213>  Artificial Sequence

<220>
<223>  mAb63 heavy chain HCDR2 mutation (N54S) sequence

<400>  47
Asp Ile Phe Pro Lys Ser Gly Asn Thr Asp Tyr Asn Arg Lys Phe Lys
1               5                   10                  15
Asp

<210>  48
<211>  119
<212>  PRT
<213>  Artificial Sequence

<220>
<223>  mAb63 humanized antibody heavy chain variable region h63VH1
       sequence

<400>  48
Glu Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Asp Phe
            20                  25                  30

Tyr Met Asn Trp Val Arg Gln Ala Pro Gly Gln Gly Leu Glu Trp Met
        35                  40                  45
Gly Asp Ile Phe Pro Lys Asn Gly Asn Thr Asp Tyr Asn Arg Lys Phe
    50                  55                  60
Lys Asp Arg Val Thr Met Thr Arg Asp Thr Ser Thr Ser Thr Val Tyr
65                  70                  75                  80
Met Glu Leu Ser Ser Leu Arg Ser Glu Asp Thr Ala Val Tyr Tyr Cys
                85                  90                  95
Ala Arg Ser Gly Tyr Gly Asn Tyr Val Phe Asp Tyr Trp Gly Gln Gly
            100                 105                 110
Thr Leu Val Thr Val Ser Ser
        115

<210>  49
<211>  119
<212>  PRT
<213>  Artificial Sequence

<220>
<223>  mAb63 humanized antibody heavy chain variable region h63VH2
       sequence


<400>  49
Glu Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Asp Phe
            20                  25                  30
Tyr Met Asn Trp Val Arg Gln Ala Pro Gly Gln Gly Leu Glu Trp Met
        35                  40                  45
Gly Asp Ile Phe Pro Lys Asn Gly Asn Thr Asp Tyr Asn Arg Lys Phe
    50                  55                  60
Lys Asp Arg Val Thr Leu Thr Val Asp Lys Ser Thr Ser Thr Val Tyr
65                  70                  75                  80
Met Glu Leu Ser Ser Leu Arg Ser Glu Asp Thr Ala Val Tyr Tyr Cys
                85                  90                  95
Ala Arg Ser Gly Tyr Gly Asn Tyr Val Phe Asp Tyr Trp Gly Gln Gly
            100                 105                 110
Thr Leu Val Thr Val Ser Ser
        115

<210>  50
<211>  119
<212>  PRT
<213>  Artificial Sequence

<220>
<223>  mAb63 humanized antibody heavy chain variable region h63VH3
       sequence


<400>  50
Glu Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Asp Phe
            20                  25                  30
Tyr Met Asn Trp Val Arg Gln Ala Pro Gly Gln Gly Leu Glu Trp Ile
        35                  40                  45
Gly Asp Ile Phe Pro Lys Asn Gly Asn Thr Asp Tyr Asn Arg Lys Phe
    50                  55                  60
Lys Asp Arg Ala Thr Leu Thr Val Asp Lys Ser Thr Ser Thr Val Tyr
65                  70                  75                  80
Met Glu Phe Ser Ser Leu Arg Ser Glu Asp Thr Ala Val Tyr Tyr Cys

85                  90                  95
Ala Arg Ser Gly Tyr Gly Asn Tyr Val Phe Asp Tyr Trp Gly Gln Gly
            100                 105                 110
Thr Leu Val Thr Val Ser Ser
        115

<210>  51
<211>  119
<212>  PRT
<213>  Artificial Sequence

<220>
<223>  mAb63 humanized antibody heavy chain variable region h63VH4
       sequence


<400>  51
Glu Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Asp Phe
            20                  25                  30
Tyr Met Asn Trp Val Arg Gln Ala Pro Gly Gln Gly Leu Glu Trp Ile
        35                  40                  45
Gly Asp Ile Phe Pro Lys Asn Gly Asn Thr Asp Tyr Asn Arg Lys Phe
    50                  55                  60
Lys Asp Lys Ala Thr Leu Thr Val Asp Lys Ser Thr Ser Thr Val Tyr
65                  70                  75                  80
Met Glu Phe Arg Ser Leu Arg Ser Glu Asp Thr Ala Val Tyr Tyr Cys
                85                  90                  95
Ala Arg Ser Gly Tyr Gly Asn Tyr Val Phe Asp Tyr Trp Gly Gln Gly
            100                 105                 110
Thr Leu Val Thr Val Ser Ser
        115

<210>  52
<211>  119
<212>  PRT
<213>  Artificial Sequence

<220>
<223>  mAb63 humanized antibody heavy chain variable region h63VH5
       sequence


<400>  52
Glu Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Asp Phe
            20                  25                  30
Tyr Met Asn Trp Val Arg Gln Ala Pro Gly Gln Gly Leu Glu Trp Met
        35                  40                  45
Gly Asp Ile Phe Pro Lys Ser Gly Asn Thr Asp Tyr Asn Arg Lys Phe
    50                  55                  60
Lys Asp Arg Val Thr Met Thr Arg Asp Thr Ser Thr Ser Thr Val Tyr
65                  70                  75                  80
Met Glu Leu Ser Ser Leu Arg Ser Glu Asp Thr Ala Val Tyr Tyr Cys
                85                  90                  95
Ala Arg Ser Gly Tyr Gly Asn Tyr Val Phe Asp Tyr Trp Gly Gln Gly
            100                 105                 110
Thr Leu Val Thr Val Ser Ser
        115

<210>  53

<211>  107
<212>  PRT
<213>  Artificial Sequence

<220>
<223>  mAb63 humanized antibody light chain variable region h63VL1
       sequence


<400>  53
Asp Ile Gln Met Thr Gln Ser Pro Ser Ser Leu Ser Ala Ser Val Gly
1               5                   10                  15
Asp Arg Val Thr Ile Thr Cys Arg Thr Ser Gln Asp Ile Asn Ile Tyr
            20                  25                  30
Leu Asn Trp Tyr Gln Gln Lys Pro Gly Lys Ala Pro Lys Leu Leu Ile
        35                  40                  45
Tyr Tyr Arg Ser Gly Leu Leu Ser Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Phe Ala Thr Tyr Tyr Cys Gln Gln Gly Asn Thr Leu Pro Pro
                85                  90                  95
Thr Phe Gly Gly Gly Thr Lys Val Glu Ile Lys
            100                 105

<210>  54
<211>  107
<212>  PRT
<213>  Artificial Sequence

<220>
<223>  mAb63 humanized antibody light chain variable region h63VL2
       sequence


<400>  54
Asp Val Gln Met Thr Gln Ser Pro Ser Ser Leu Ser Ala Ser Val Gly
1               5                   10                  15
Asp Arg Val Thr Ile Thr Cys Arg Thr Ser Gln Asp Ile Asn Ile Tyr
            20                  25                  30
Leu Asn Trp Tyr Gln Gln Lys Pro Gly Lys Ala Pro Lys Leu Leu Ile
        35                  40                  45
Tyr Tyr Arg Ser Gly Leu Leu Ser Gly Val Pro Ser Arg Phe Ser Gly
    50                  55                  60
Ser Gly Ser Gly Thr Asp Tyr Thr Leu Thr Ile Ser Ser Leu Gln Pro
65                  70                  75                  80
Glu Asp Phe Ala Thr Tyr Tyr Cys Gln Gln Gly Asn Thr Leu Pro Pro
                85                  90                  95
Thr Phe Gly Gly Gly Thr Lys Val Glu Ile Lys
            100                 105

<210>  55
<211>  107
<212>  PRT
<213>  Artificial Sequence

<220>
<223>  mAb63 humanized antibody light chain variable region h63VL3
       sequence


<400>  55
Asp Val Gln Met Thr Gln Ser Pro Ser Ser Leu Ser Ala Ser Val Gly

1               5                   10                  15
Asp Arg Val Thr Ile Thr Cys Arg Thr Ser Gln Asp Ile Asn Ile Tyr
            20                  25                  30
Leu Asn Trp Tyr Gln Gln Lys Pro Gly Gly Ala Val Lys Leu Leu Ile
        35                  40                  45
Tyr Tyr Arg Ser Gly Leu Leu Ser Gly Val Pro Ser Arg Phe Ser Gly
    50                  55                  60
Ser Gly Ser Gly Thr Asp Tyr Thr Leu Thr Ile Ser Ser Leu Gln Pro
65                  70                  75                  80
Glu Asp Phe Ala Thr Tyr Tyr Cys Gln Gln Gly Asn Thr Leu Pro Pro
                85                  90                  95
Thr Phe Gly Gly Gly Thr Lys Val Glu Ile Lys
            100                 105

<210>  56
<211>  117
<212>  PRT
<213>  Artificial Sequence

<220>
<223>  mAb67 humanized antibody heavy chain variable region h67VH1
       sequence


<400>  56
Glu Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Asp Tyr
            20                  25                  30
His Met Asn Trp Val Arg Gln Ala Pro Gly Gln Arg Leu Glu Trp Met
        35                  40                  45
Gly Asp Ile Asn Pro Asp Ile Gly Gly Thr Ser Tyr Asn Gln Asn Phe
    50                  55                  60
Lys Gly Arg Val Thr Ile Thr Arg Asp Thr Ser Ala Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Trp Asp Phe Asp Ser Phe Ala Asn Trp Gly Gln Gly Thr Leu
            100                 105                 110
Val Thr Val Ser Ser
        115

<210>  57
<211>  117
<212>  PRT
<213>  Artificial Sequence

<220>
<223>  mAb67 humanized antibody heavy chain variable region h67VH2
       sequence


<400>  57
Glu Val Gln Leu Val Gln Ser Gly Ala Glu Val Lys Lys Pro Gly Glu
1               5                   10                  15
Ser Leu Lys Ile Ser Cys Lys Gly Ser Gly Tyr Ser Phe Thr Asp Tyr
            20                  25                  30
His Met Asn Trp Val Arg Gln Met Pro Gly Lys Gly Leu Glu Trp Met
        35                  40                  45
Gly Asp Ile Asn Pro Asp Ile Gly Gly Thr Ser Tyr Asn Gln Asn Phe
    50                  55                  60
Lys Gly Gln Val Thr Ile Ser Ala Asp Lys Ser Ile Ser Thr Ala Tyr
65                  70                  75                  80

Leu Gln Trp Ser Ser Leu Lys Ala Ser Asp Thr Ala Met Tyr Tyr Cys
                85                  90                  95
Ala Arg Trp Asp Phe Asp Ser Phe Ala Asn Trp Gly Gln Gly Thr Leu
            100                 105                 110
Val Thr Val Ser Ser
        115

<210>  58
<211>  117
<212>  PRT
<213>  Artificial Sequence

<220>

<223>  mAb67 humanized antibody heavy chain variable region h67VH3
       sequence


<400>  58
Glu Val Gln Leu Val Gln Ser Gly Ala Glu Val Lys Lys Pro Gly Glu
1               5                   10                  15
Ser Leu Lys Ile Ser Cys Lys Gly Ser Gly Tyr Ser Phe Thr Asp Tyr
            20                  25                  30
His Met Asn Trp Val Lys Gln Met Pro Gly Lys Gly Leu Glu Trp Met
        35                  40                  45
Gly Asp Ile Asn Pro Asp Ile Gly Gly Thr Ser Tyr Asn Gln Asn Phe
    50                  55                  60
Lys Gly Lys Val Thr Ile Ser Val Asp Lys Ser Ile Ser Thr Ala Tyr
65                  70                  75                  80
Leu Gln Trp Ser Ser Leu Lys Ala Ser Asp Thr Ala Met Tyr Tyr Cys
                85                  90                  95
Ala Arg Trp Asp Phe Asp Ser Phe Ala Asn Trp Gly Gln Gly Thr Leu
            100                 105                 110
Val Thr Val Ser Ser
        115

<210>  59
<211>  111
<212>  PRT
<213>  Artificial Sequence

<220>
<223>  mAb67 humanized antibody light chain variable region h67VL1
       sequence


<400>  59
Asp Ile Val Met Thr Gln Ser Pro Asp Ser Leu Ala Val Ser Leu Gly
1               5                   10                  15
Glu Arg Ala Thr Ile Asn Cys Arg Ala Ser Glu Ser Val Ser Ile Ile
            20                  25                  30
Gly Thr Asn Leu Ile His Trp Tyr Gln Gln Lys Pro Gly Gln Pro Pro
        35                  40                  45
Lys Leu Leu Ile Tyr His Ala Ser Asn Leu Glu Thr Gly Val Pro Asp
    50                  55                  60
Arg Phe Ser Gly Ser Gly Ser Gly Thr Asp Phe Thr Leu Thr Ile Ser
65                  70                  75                  80
Ser Leu Gln Ala Glu Asp Val Ala Val Tyr Tyr Cys Leu Gln Ser Arg
                85                  90                  95
Lys Ile Pro Tyr Thr Phe Gly Gly Gly Thr Lys Val Glu Ile Lys
            100                 105                 110

<210>  60

<211>  111
<212>  PRT
<213>  Artificial Sequence

<220>
<223>  mAb67 humanized antibody light chain variable region h67VL2
       sequence


<400>  60
Glu Ile Val Met Thr Gln Ser Pro Ala Thr Leu Ser Val Ser Pro Gly
1               5                   10                  15
Glu Arg Ala Thr Leu Ser Cys Arg Ala Ser Glu Ser Val Ser Ile Ile
            20                  25                  30
Gly Thr Asn Leu Ile His Trp Tyr Gln Gln Lys Pro Gly Gln Ala Pro
        35                  40                  45
Arg Leu Leu Ile Tyr His Ala Ser Asn Leu Glu Thr Gly Ile Pro Ala
    50                  55                  60
Arg Phe Ser Gly Ser Gly Ser Gly Thr Glu Phe Thr Leu Thr Ile Ser
65                  70                  75                  80
Ser Leu Gln Ser Glu Asp Phe Ala Val Tyr Tyr Cys Leu Gln Ser Arg
                85                  90                  95
Lys Ile Pro Tyr Thr Phe Gly Gly Gly Thr Lys Val Glu Ile Lys
            100                 105                 110

<210>  61
<211>  111
<212>  PRT
<213>  Artificial Sequence

<220>
<223>  mAb67 humanized antibody light chain variable region h67VL3
       sequence


<400>  61
Glu Ile Val Met Thr Gln Ser Pro Ala Thr Leu Ser Val Ser Pro Gly
1               5                   10                  15
Glu Arg Ala Thr Leu Ser Cys Arg Ala Ser Glu Ser Val Ser Ile Ile
            20                  25                  30
Gly Thr Asn Leu Ile His Trp Tyr Gln Gln Lys Pro Gly Gln Pro Pro
        35                  40                  45
Arg Leu Leu Ile Tyr His Ala Ser Asn Leu Glu Thr Gly Val Pro Ala
    50                  55                  60
Arg Phe Ser Gly Ser Gly Ser Gly Thr Glu Phe Thr Leu Thr Ile Ser
65                  70                  75                  80
Ser Leu Gln Ser Glu Asp Phe Ala Val Tyr Tyr Cys Leu Gln Ser Arg
                85                  90                  95
Lys Ile Pro Tyr Thr Phe Gly Gly Gly Thr Lys Val Glu Ile Lys
            100                 105                 110

<210>  62
<211>  111
<212>  PRT
<213>  Artificial Sequence

<220>
<223>  mAb67 humanized antibody light chain variable region h67VL4
       sequence


<400>  62
Asp Ile Gln Met Thr Gln Ser Pro Ser Ser Leu Ser Ala Ser Val Gly

1                   5                   10                  15
Asp Arg Val Thr Ile Thr Cys Arg Ala Ser Glu Ser Val Ser Ile Ile
            20                  25                  30
Gly Thr Asn Leu Ile His Trp Tyr Gln Gln Lys Pro Gly Lys Ala Pro
        35                  40                  45
Lys Leu Leu Ile Tyr His Ala Ser Asn Leu Glu Thr Gly Val Pro Ser
    50                  55                  60
Arg Phe Ser Gly Ser Gly Ser Gly Thr Asp Phe Thr Leu Thr Ile Ser
65                  70                  75                  80
Ser Leu Gln Pro Glu Asp Phe Ala Thr Tyr Tyr Cys Leu Gln Ser Arg
                85                  90                  95
Lys Ile Pro Tyr Thr Phe Gly Gly Gly Thr Lys Val Glu Ile Lys
            100                 105                 110

<210>  63
<211>  111
<212>  PRT
<213>  Artificial Sequence

<220>
<223>  mAb67 humanized antibody light chain variable region h67VL5
       sequence


<400>  63
Asp Ile Val Met Thr Gln Ser Pro Ser Ser Leu Ser Ala Ser Val Gly
1               5                   10                  15
Asp Arg Val Thr Ile Thr Cys Arg Ala Ser Glu Ser Val Ser Ile Ile
            20                  25                  30
Gly Thr Asn Leu Ile His Trp Tyr Gln Gln Lys Pro Gly Lys Pro Pro
        35                  40                  45
Lys Leu Leu Ile Tyr His Ala Ser Asn Leu Glu Thr Gly Val Pro Ser
    50                  55                  60
Arg Phe Ser Gly Ser Gly Ser Gly Thr Asp Phe Thr Leu Thr Ile Ser
65                  70                  75                  80
Ser Leu Gln Pro Glu Asp Phe Ala Thr Tyr Tyr Cys Leu Gln Ser Arg
                85                  90                  95
Lys Ile Pro Tyr Thr Phe Gly Gly Gly Thr Lys Val Glu Ile Lys
            100                 105                 110

<210>  64
<211>  11
<212>  PRT
<213>  Artificial Sequence

<220>
<223>  mAb103 light chain LCDR2 mutation (D56E) sequence


<400>  64
Leu Lys Lys Asp Gly Ser His Ser Thr Gly Glu
1               5                   10

<210>  65
<211>  122
<212>  PRT
<213>  Artificial Sequence

<220>
<223>  mAb103 humanized antibody heavy chain variable region
       h103VH1 sequence

<400>  65
Glu Val Gln Leu Val Gln Ser Gly Ser Glu Leu Lys Lys Pro Gly Ala
1               5                   10                  15
Ser Val Lys Val Ser Cys Lys Ala Ser Gly Tyr Thr Phe Thr Thr Tyr
            20                  25                  30
Gly Val Ile Trp Val Arg Gln Ala Pro Gly Gln Gly Leu Glu Trp Met
        35                  40                  45
Gly Trp Ile Asn Thr Tyr Ser Gly Val Pro Thr Tyr Ala Asp Asp Phe
    50                  55                  60
Lys Gly Arg Phe Val Phe Ser Leu Asp Thr Ser Val Ser Thr Ala Tyr
65                  70                  75                  80
Leu Gln Ile Ser Ser Leu Lys Ala Glu Asp Thr Ala Val Tyr Tyr Cys
                85                  90                  95
Ala Arg Lys Lys Thr Leu Thr Thr Val Thr Pro Trp Phe Ala Tyr Trp
            100                 105                 110
Gly Gln Gly Thr Leu Val Thr Val Ser Ser
        115                 120

<210>  66
<211>  122
<212>  PRT
<213>  Artificial Sequence

<220>
<223>  mAb103 humanized antibody heavy chain variable region
       h103VH2 sequence


<400>  66
Glu Val Gln Leu Val Gln Ser Gly Ser Glu Leu Lys Lys Pro Gly Ala
1               5                   10                  15
Ser Val Lys Val Ser Cys Lys Ala Ser Gly Tyr Thr Phe Thr Thr Tyr
            20                  25                  30
Gly Val Ile Trp Val Lys Gln Ala Pro Gly Gln Gly Leu Lys Trp Met
        35                  40                  45
Gly Trp Ile Asn Thr Tyr Ser Gly Val Pro Thr Tyr Ala Asp Asp Phe
    50                  55                  60
Lys Gly Arg Phe Val Phe Ser Leu Asp Thr Ser Val Ser Thr Ala Tyr
65                  70                  75                  80
Leu Gln Ile Ser Ser Leu Lys Ala Glu Asp Thr Ala Val Tyr Tyr Cys
                85                  90                  95
Ala Arg Lys Lys Thr Leu Thr Thr Val Thr Pro Trp Phe Ala Tyr Trp
            100                 105                 110
Gly Gln Gly Thr Leu Val Thr Val Ser Ser
        115                 120

<210>  67
<211>  122
<212>  PRT
<213>  Artificial Sequence

<220>
<223>  mAb103 humanized antibody heavy chain variable region
       h103VH3 sequence


<400>  67
Glu Ile Gln Leu Val Gln Ser Gly Ser Glu Leu Lys Lys Pro Gly Ala
1               5                   10                  15
Ser Val Lys Val Ser Cys Lys Ala Ser Gly Tyr Thr Phe Thr Thr Tyr
            20                  25                  30
Gly Val Ile Trp Val Lys Gln Ala Pro Gly Gln Gly Leu Lys Trp Met
        35                  40                  45

Gly Trp Ile Asn Thr Tyr Ser Gly Val Pro Thr Tyr Ala Asp Asp Phe
    50                  55                  60
Lys Gly Arg Phe Val Phe Ser Leu Asp Thr Ser Val Ser Thr Ala Tyr
65                  70                  75                  80
Leu Gln Ile Ser Ser Leu Lys Ala Glu Asp Thr Ala Val Tyr Tyr Cys
                85                  90                  95
Ala Arg Lys Lys Thr Leu Thr Thr Val Thr Pro Trp Phe Ala Tyr Trp
            100                 105                 110
Gly Gln Gly Thr Leu Val Thr Val Ser Ser
        115                 120

<210>  68
<211>  122
<212>  PRT
<213>  Artificial Sequence

<220>
<223>  mAb103 humanized antibody heavy chain variable region
       h103VH4 sequence


<400>  68
Glu Val Gln Leu Val Gln Ser Gly Ser Glu Leu Lys Lys Pro Gly Ala
1               5                   10                  15
Ser Val Lys Val Ser Cys Lys Ala Ser Gly Tyr Thr Phe Thr Thr Tyr
            20                  25                  30
Gly Val Ile Trp Val Arg Gln Ala Pro Gly Gln Gly Leu Glu Trp Met
        35                  40                  45
Gly Trp Ile Asn Thr Tyr Ser Gly Val Pro Thr Tyr Ala Ser Asp Phe
    50                  55                  60
Lys Gly Arg Phe Val Phe Ser Leu Asp Thr Ser Val Ser Thr Ala Tyr
65                  70                  75                  80
Leu Gln Ile Ser Ser Leu Lys Ala Glu Asp Thr Ala Val Tyr Tyr Cys
                85                  90                  95
Ala Arg Lys Lys Thr Leu Thr Thr Val Thr Pro Trp Phe Ala Tyr Trp
            100                 105                 110
Gly Gln Gly Thr Leu Val Thr Val Ser Ser
        115                 120

<210>  69
<211>  115
<212>  PRT
<213>  Artificial Sequence

<220>
<223>  mAb103 humanized antibody light chain variable region
       h103VL1 sequence


<400>  69
Glu Leu Val Leu Thr Gln Ser Pro Ser Ala Ser Ala Ser Leu Gly Ala
1               5                   10                  15
Ser Val Lys Leu Thr Cys Thr Leu Ser Ser Gln His Ser Thr Tyr Thr
            20                  25                  30
Ile Glu Trp His Gln Gln Gln Pro Glu Lys Gly Pro Arg Tyr Leu Met
        35                  40                  45
Glu Leu Lys Lys Asp Gly Ser His Ser Thr Gly Asp Gly Ile Pro Asp
    50                  55                  60
Arg Phe Ser Gly Ser Ser Ser Gly Ala Glu Arg Tyr Leu Thr Ile Ser
65                  70                  75                  80
Ser Leu Gln Ser Glu Asp Glu Ala Asp Tyr Tyr Cys Gly Val Gly Asn
                85                  90                  95
Thr Ile Lys Glu Gln Phe Val Tyr Val Phe Gly Gly Gly Thr Lys Leu

            100                 105                 110
Thr Val Leu
        115

<210> 70
<211> 115
<212> PRT
<213> Artificial Sequence

<220>
<223> mAb103 humanized antibody light chain variable region
h103VL2 sequence

<400> 70
Glu Leu Val Leu Thr Gln Ser Pro Ser Ala Ser Ala Ser Leu Gly Ala
1               5                   10                  15
Ser Val Lys Leu Thr Cys Thr Leu Ser Ser Gln His Ser Thr Tyr Thr
            20                  25                  30
Ile Glu Trp Tyr Gln Gln Gln Pro Glu Lys Gly Pro Arg Tyr Leu Met
        35                  40                  45
Glu Leu Lys Lys Asp Gly Ser His Ser Thr Gly Asp Gly Ile Pro Asp
    50                  55                  60
Arg Phe Ser Gly Ser Ser Ser Gly Ala Glu Arg Tyr Leu Thr Ile Ser
65                  70                  75                  80
Ser Leu Gln Ser Glu Asp Glu Ala Asp Tyr Tyr Cys Gly Val Gly Asn
                85                  90                  95
Thr Ile Lys Glu Gln Phe Val Tyr Val Phe Gly Gly Gly Thr Lys Leu
            100                 105                 110
Thr Val Leu
        115

<210> 71
<211> 115
<212> PRT
<213> Artificial Sequence

<220>
<223> mAb103 humanized antibody light chain variable region
h103VL3 sequence

<400> 71
Glu Leu Val Leu Thr Gln Ser Pro Ser Ala Ser Ala Ser Leu Gly Ala
1               5                   10                  15
Ser Val Lys Leu Thr Cys Thr Leu Ser Ser Gln His Ser Thr Tyr Thr
            20                  25                  30
Ile Glu Trp Tyr Gln Gln Gln Pro Glu Lys Gly Pro Arg Tyr Val Met
        35                  40                  45
Glu Leu Lys Lys Asp Gly Ser His Ser Thr Gly Asp Gly Ile Pro Asp
    50                  55                  60
Arg Phe Ser Gly Ser Ser Ser Gly Ala Glu Arg Tyr Leu Thr Ile Ser
65                  70                  75                  80
Ser Leu Gln Ser Glu Asp Glu Ala Asp Tyr Tyr Cys Gly Val Gly Asn
                85                  90                  95
Thr Ile Lys Glu Gln Phe Val Tyr Val Phe Gly Gly Gly Thr Lys Leu
            100                 105                 110
Thr Val Leu
        115

<210> 72
<211> 115
<212> PRT

<213>  Artificial Sequence

<220>
<223>  mAb103 humanized antibody light chain variable region
       h103VL4 sequence

<400>  72
Glu Leu Val Val Thr Gln Ser Pro Ser Ala Ser Ala Ser Leu Gly Ala
1               5                   10                  15
Ser Val Lys Leu Thr Cys Thr Leu Ser Ser Gln His Ser Thr Tyr Thr
            20                  25                  30
Ile Glu Trp Tyr Gln Gln Gln Pro Glu Lys Gly Pro Arg Tyr Val Met
        35                  40                  45
Glu Leu Lys Lys Asp Gly Ser His Ser Thr Gly Asp Gly Ile Pro Asp
    50                  55                  60
Arg Phe Ser Gly Ser Ser Ser Gly Ala Glu Arg Tyr Leu Thr Ile Ser
65                  70                  75                  80
Ser Leu Gln Ser Glu Asp Glu Ala Asp Tyr Tyr Cys Gly Val Gly Asn
                85                  90                  95
Thr Ile Lys Glu Gln Phe Val Tyr Val Phe Gly Gly Gly Thr Lys Leu
            100                 105                 110
Thr Val Leu
        115

<210>  73
<211>  115
<212>  PRT
<213>  Artificial Sequence

<220>
<223>  mAb103 humanized antibody light chain variable region
       h103VL5 sequence

<400>  73
Glu Leu Val Leu Thr Gln Ser Pro Ser Ala Ser Ala Ser Leu Gly Ala
1               5                   10                  15
Ser Val Lys Leu Thr Cys Thr Leu Ser Ser Gln His Ser Thr Tyr Thr
            20                  25                  30
Ile Glu Trp Tyr Gln Gln Gln Pro Glu Lys Pro Pro Arg Tyr Val Met
        35                  40                  45
Glu Leu Lys Lys Asp Gly Ser His Ser Thr Gly Asp Gly Ile Pro Asp
    50                  55                  60
Arg Phe Ser Gly Ser Ser Ser Gly Ala Glu Arg Tyr Leu Thr Ile Ser
65                  70                  75                  80
Ser Leu Gln Ser Glu Asp Glu Ala Asp Tyr Ile Cys Gly Val Gly Asn
                85                  90                  95
Thr Ile Lys Glu Gln Phe Val Tyr Val Phe Gly Gly Gly Thr Lys Leu
            100                 105                 110
Thr Val Leu
        115

<210>  74
<211>  115
<212>  PRT
<213>  Artificial Sequence

<220>
<223>  mAb103 humanized antibody light chain variable region
       h103VL6 sequence

<400>  74
Glu Leu Val Val Thr Gln Ser Pro Ser Ala Ser Ala Ser Leu Gly Ala
1               5                   10                  15
Ser Val Lys Leu Thr Cys Thr Leu Ser Ser Gln His Ser Thr Tyr Thr
            20                  25                  30
Ile Glu Trp Tyr Gln Gln Gln Pro Glu Lys Pro Pro Arg Tyr Val Met
        35                  40                  45
Glu Leu Lys Lys Asp Gly Ser His Ser Thr Gly Asp Gly Ile Pro Asp
    50                  55                  60
Arg Phe Ser Gly Ser Ser Ser Gly Ala Glu Arg Tyr Leu Thr Ile Ser
65                  70                  75                  80
Ser Leu Gln Ser Glu Asp Glu Ala Asp Tyr Ile Cys Gly Val Gly Asn
                85                  90                  95
Thr Ile Lys Glu Gln Phe Val Tyr Val Phe Gly Gly Gly Thr Lys Leu
            100                 105                 110
Thr Val Leu
        115

<210>  75
<211>  115
<212>  PRT
<213>  Artificial Sequence

<220>
<223>  mAb103 humanized antibody light chain variable region
       h103VL7 sequence


<400>  75
Glu Leu Val Val Thr Gln Ser Pro Ser Ala Ser Ala Ser Leu Gly Ala
1               5                   10                  15
Ser Val Lys Leu Thr Cys Thr Leu Ser Ser Gln His Ser Thr Tyr Thr
            20                  25                  30
Ile Glu Trp Tyr Gln Gln Gln Pro Glu Lys Pro Pro Arg Tyr Val Met
        35                  40                  45
Glu Leu Lys Lys Asp Gly Ser His Ser Thr Gly Asp Gly Ile Pro Asp
    50                  55                  60
Arg Phe Ser Gly Ser Ser Ser Gly Ala Asp Arg Tyr Leu Thr Ile Ser
65                  70                  75                  80
Ser Leu Gln Ser Glu Asp Glu Ala Asp Tyr Ile Cys Gly Val Gly Asn
                85                  90                  95
Thr Ile Lys Glu Gln Phe Val Tyr Val Phe Gly Gly Gly Thr Lys Leu
            100                 105                 110
Thr Val Leu
        115

<210>  76
<211>  115
<212>  PRT
<213>  Artificial Sequence

<220>
<223>  mAb103 humanized antibody light chain variable region
       h103VL8 sequence


<400>  76
Glu Leu Val Leu Thr Gln Ser Pro Ser Ala Ser Ala Ser Leu Gly Ala
1               5                   10                  15
Ser Val Lys Leu Thr Cys Thr Leu Ser Ser Gln His Ser Thr Tyr Thr
            20                  25                  30
Ile Glu Trp Tyr Gln Gln Gln Pro Glu Lys Gly Pro Arg Tyr Val Met
        35                  40                  45

Glu Leu Lys Lys Asp Gly Ser His Ser Thr Gly Glu Gly Ile Pro Asp
    50                  55                  60
Arg Phe Ser Gly Ser Ser Ser Gly Ala Glu Arg Tyr Leu Thr Ile Ser
65                  70                  75                  80
Ser Leu Gln Ser Glu Asp Glu Ala Asp Tyr Tyr Cys Gly Val Gly Asn
                85                  90                  95
Thr Ile Lys Glu Gln Phe Val Tyr Val Phe Gly Gly Gly Thr Lys Leu
            100                 105                 110
Thr Val Leu
        115

<210>  77
<211>  330
<212>  PRT
<213>  Artificial Sequence

<220>
<223>  human IgG1 heavy chain constant region


<400>  77

<210>  78
<211>  107
<212>  PRT
<213>  Artificial Sequence

<220>
<223>  human light chain constant region kappa chain sequence


<400>  78

<210>  79
<211>  106
<212>  PRT
<213>  Artificial Sequence

<220>
<223>  human light chain constant region lambda chain sequence


<400>  79
Gly Gln Pro Lys Ala Asn Pro Thr Val Thr Leu Phe Pro Pro Ser Ser
1               5                   10                  15
Glu Glu Leu Gln Ala Asn Lys Ala Thr Leu Val Cys Leu Ile Ser Asp
            20                  25                  30
Phe Tyr Pro Gly Ala Val Thr Val Ala Trp Lys Ala Asp Gly Ser Pro
        35                  40                  45
Val Lys Ala Gly Val Glu Thr Thr Lys Pro Ser Lys Gln Ser Asn Asn
    50                  55                  60
Lys Tyr Ala Ala Ser Ser Tyr Leu Ser Leu Thr Pro Glu Gln Trp Lys
65                  70                  75                  80
Ser His Arg Ser Tyr Ser Cys Gln Val Thr His Glu Gly Ser Thr Val
                85                  90                  95
Glu Lys Thr Val Ala Pro Thr Glu Cys Ser
            100                 105

<210>  80
<211>  449
<212>  PRT
<213>  Artificial Sequence

<220>
<223>  Hu63-13 heavy chain sequence


<400>  80
Glu Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Asp Phe
            20                  25                  30
Tyr Met Asn Trp Val Arg Gln Ala Pro Gly Gln Gly Leu Glu Trp Met
        35                  40                  45
Gly Asp Ile Phe Pro Lys Ser Gly Asn Thr Asp Tyr Asn Arg Lys Phe
    50                  55                  60
Lys Asp Arg Val Thr Met Thr Arg Asp Thr Ser Thr Ser Thr Val Tyr
65                  70                  75                  80
Met Glu Leu Ser Ser Leu Arg Ser Glu Asp Thr Ala Val Tyr Tyr Cys
                85                  90                  95
Ala Arg Ser Gly Tyr Gly Asn Tyr Val Phe Asp Tyr Trp Gly Gln Gly
            100                 105                 110
Thr Leu Val Thr Val Ser Ser Ala Ser Thr Lys Gly Pro Ser Val Phe
        115                 120                 125
Pro Leu Ala Pro Ser Ser Lys Ser Thr Ser Gly Gly Thr Ala Ala Leu
    130                 135                 140
Gly Cys Leu Val Lys Asp Tyr Phe Pro Glu Pro Val Thr Val Ser Trp
145                 150                 155                 160
Asn Ser Gly Ala Leu Thr Ser Gly Val His Thr Phe Pro Ala Val Leu
                165                 170                 175
Gln Ser Ser Gly Leu Tyr Ser Leu Ser Ser Val Val Thr Val Pro Ser
            180                 185                 190
Ser Ser Leu Gly Thr Gln Thr Tyr Ile Cys Asn Val Asn His Lys Pro
        195                 200                 205
Ser Asn Thr Lys Val Asp Lys Lys Val Glu Pro Lys Ser Cys Asp Lys
    210                 215                 220
Thr His Thr Cys Pro Pro Cys Pro Ala Pro Glu Leu Leu Gly Gly Pro
225                 230                 235                 240
Ser Val Phe Leu Phe Pro Pro Lys Pro Lys Asp Thr Leu Met Ile Ser
                245                 250                 255
Arg Thr Pro Glu Val Thr Cys Val Val Val Asp Val Ser His Glu Asp
            260                 265                 270
Pro Glu Val Lys Phe Asn Trp Tyr Val Asp Gly Val Glu Val His Asn
        275                 280                 285
Ala Lys Thr Lys Pro Arg Glu Glu Gln Tyr Asn Ser Thr Tyr Arg Val
    290                 295                 300
Val Ser Val Leu Thr Val Leu His Gln Asp Trp Leu Asn Gly Lys Glu
305                 310                 315                 320
Tyr Lys Cys Lys Val Ser Asn Lys Ala Leu Pro Ala Pro Ile Glu Lys
                325                 330                 335
Thr Ile Ser Lys Ala Lys Gly Gln Pro Arg Glu Pro Gln Val Tyr Thr
            340                 345                 350
Leu Pro Pro Ser Arg Asp Glu Leu Thr Lys Asn Gln Val Ser Leu Thr
        355                 360                 365
Cys Leu Val Lys Gly Phe Tyr Pro Ser Asp Ile Ala Val Glu Trp Glu
    370                 375                 380
Ser Asn Gly Gln Pro Glu Asn Asn Tyr Lys Thr Thr Pro Pro Val Leu
385                 390                 395                 400
Asp Ser Asp Gly Ser Phe Phe Leu Tyr Ser Lys Leu Thr Val Asp Lys
                405                 410                 415
Ser Arg Trp Gln Gln Gly Asn Val Phe Ser Cys Ser Val Met His Glu
            420                 425                 430
Ala Leu His Asn His Tyr Thr Gln Lys Ser Leu Ser Leu Ser Pro Gly
        435                 440                 445
Lys


<210>  81
<211>  214
<212>  PRT
<213>  Artificial Sequence

<220>

<223>  Hu63-13 light chain sequence

<400>  81
Asp Ile Gln Met Thr Gln Ser Pro Ser Ser Leu Ser Ala Ser Val Gly
1               5                   10                  15
Asp Arg Val Thr Ile Thr Cys Arg Thr Ser Gln Asp Ile Asn Ile Tyr
            20                  25                  30
Leu Asn Trp Tyr Gln Gln Lys Pro Gly Lys Ala Pro Lys Leu Leu Ile
        35                  40                  45
Tyr Tyr Arg Ser Gly Leu Leu Ser Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Phe Ala Thr Tyr Tyr Cys Gln Gln Gly Asn Thr Leu Pro Pro
                85                  90                  95
Thr Phe Gly Gly Gly Thr Lys Val Glu Ile Lys Arg Thr Val Ala Ala
            100                 105                 110
Pro Ser Val Phe Ile Phe Pro Pro Ser Asp Glu Gln Leu Lys Ser Gly
        115                 120                 125
Thr Ala Ser Val Val Cys Leu Leu Asn Asn Phe Tyr Pro Arg Glu Ala
    130                 135                 140
Lys Val Gln Trp Lys Val Asp Asn Ala Leu Gln Ser Gly Asn Ser Gln
145                 150                 155                 160
Glu Ser Val Thr Glu Gln Asp Ser Lys Asp Ser Thr Tyr Ser Leu Ser
                165                 170                 175
Ser Thr Leu Thr Leu Ser Lys Ala Asp Tyr Glu Lys His Lys Val Tyr
            180                 185                 190
Ala Cys Glu Val Thr His Gln Gly Leu Ser Ser Pro Val Thr Lys Ser
        195                 200                 205
Phe Asn Arg Gly Glu Cys
    210

<210>  82
<211>  450
<212>  PRT
<213>  Artificial Sequence

<220>
<223>  Hu47-14 heavy chain sequence

<400>  82
Glu Val Gln Leu Val Gln Ser Gly Ser Glu Leu Lys Lys Pro Gly Ala
1               5                   10                  15
Ser Val Lys Val Ser Cys Lys Ala Ser Gly Tyr Thr Phe Thr Thr Tyr
            20                  25                  30
Gly Met Ser Trp Val Lys Gln Ala Pro Gly Gln Gly Leu Lys Trp Met
        35                  40                  45
Gly Trp Ile Asn Thr Tyr Ser Gly Val Pro Thr Tyr Ala Ser Asp Phe
    50                  55                  60
Lys Gly Arg Phe Val Phe Ser Leu Asp Thr Ser Val Ser Thr Ala Tyr
65                  70                  75                  80
Leu Gln Ile Ser Ser Leu Lys Ala Glu Asp Thr Ala Val Tyr Tyr Cys
                85                  90                  95
Ala Arg Arg Gly Asn Tyr Gly Arg Trp Asp Phe Asp Val Trp Gly Gln
            100                 105                 110
Gly Thr Thr Val Thr Val Ser Ser Ala Ser Thr Lys Gly Pro Ser Val
        115                 120                 125
Phe Pro Leu Ala Pro Ser Ser Lys Ser Thr Ser Gly Gly Thr Ala Ala
    130                 135                 140
Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175
Leu Gln Ser Ser Gly Leu Tyr Ser Leu Ser Ser Val Val Thr Val Pro
            180                 185                 190
Ser Ser Ser Leu Gly Thr Gln Thr Tyr Ile Cys Asn Val Asn His Lys
        195                 200                 205
Pro Ser Asn Thr Lys Val Asp Lys Lys Val Glu Pro Lys Ser Cys Asp
    210                 215                 220
Lys Thr His Thr Cys Pro Pro Cys Pro Ala Pro Glu Leu Leu Gly Gly
225                 230                 235                 240
Pro Ser Val Phe Leu Phe Pro Pro Lys Pro Lys Asp Thr Leu Met Ile
                245                 250                 255
Ser Arg Thr Pro Glu Val Thr Cys Val Val Val Asp Val Ser His Glu
            260                 265                 270
Asp Pro Glu Val Lys Phe Asn Trp Tyr Val Asp Gly Val Glu Val His
        275                 280                 285
Asn Ala Lys Thr Lys Pro Arg Glu Glu Gln Tyr Asn Ser Thr Tyr Arg
    290                 295                 300
Val Val Ser Val Leu Thr Val Leu His Gln Asp Trp Leu Asn Gly Lys
305                 310                 315                 320
Glu Tyr Lys Cys Lys Val Ser Asn Lys Ala Leu Pro Ala Pro Ile Glu
                325                 330                 335
Lys Thr Ile Ser Lys Ala Lys Gly Gln Pro Arg Glu Pro Gln Val Tyr
            340                 345                 350
Thr Leu Pro Pro Ser Arg Asp Glu Leu Thr Lys Asn Gln Val Ser Leu
        355                 360                 365
Thr Cys Leu Val Lys Gly Phe Tyr Pro Ser Asp Ile Ala Val Glu Trp
    370                 375                 380
Glu Ser Asn Gly Gln Pro Glu Asn Asn Tyr Lys Thr Thr Pro Pro Val
385                 390                 395                 400
Leu Asp Ser Asp Gly Ser Phe Phe Leu Tyr Ser Lys Leu Thr Val Asp
                405                 410                 415
Lys Ser Arg Trp Gln Gln Gly Asn Val Phe Ser Cys Ser Val Met His
            420                 425                 430
Glu Ala Leu His Asn His Tyr Thr Gln Lys Ser Leu Ser Leu Ser Pro
        435                 440                 445
Gly Lys
    450

<210>  83
<211>  215
<212>  PRT
<213>  Artificial Sequence

<220>
<223>  Hu47-14 light chain sequence


<400>  83
Glu Ile Val Leu Thr Gln Ser Pro Asp Phe Gln Ser Val Thr Pro Lys
1               5                   10                  15
Glu Lys Val Thr Ile Thr Cys Ser Val Ser Ser Thr Ile Ser Ser Ser
            20                  25                  30
Asn Leu His Trp Tyr Gln Gln Lys Pro Asp Gln Ser Pro Lys Pro Leu
        35                  40                  45
Ile Tyr Gly Thr Ser Asn Leu Ala Ser Gly Val Pro Ser Arg Phe Ser
    50                  55                  60
Gly Ser Gly Ser Gly Thr Asp Tyr Thr Leu Thr Ile Asn Ser Leu Glu
65                  70                  75                  80
Ala Glu Asp Ala Ala Thr Tyr Tyr Cys Gln Gln Trp Ser Ile Tyr Pro
                85                  90                  95
Leu Thr Phe Gly Gln Gly Thr Lys Leu Glu Ile Lys Arg Thr Val Ala
            100                 105                 110

Ala Pro Ser Val Phe Ile Phe Pro Pro Ser Asp Glu Gln Leu Lys Ser
        115                 120                 125
Gly Thr Ala Ser Val Val Cys Leu Leu Asn Asn Phe Tyr Pro Arg Glu
    130                 135                 140
Ala Lys Val Gln Trp Lys Val Asp Asn Ala Leu Gln Ser Gly Asn Ser
145                 150                 155                 160
Gln Glu Ser Val Thr Glu Gln Asp Ser Lys Asp Ser Thr Tyr Ser Leu
                165                 170                 175
Ser Ser Thr Leu Thr Leu Ser Lys Ala Asp Tyr Glu Lys His Lys Val
            180                 185                 190
Tyr Ala Cys Glu Val Thr His Gln Gly Leu Ser Ser Pro Val Thr Lys
        195                 200                 205
Ser Phe Asn Arg Gly Glu Cys
    210                 215

<210> 84
<211> 447
<212> PRT
<213> Artificial Sequence

<220>
<223> Hu67-14 heavy chain sequence


<400> 84
Glu Val Gln Leu Val Gln Ser Gly Ala Glu Val Lys Lys Pro Gly Glu
1               5                   10                  15
Ser Leu Lys Ile Ser Cys Lys Gly Ser Gly Tyr Ser Phe Thr Asp Tyr
            20                  25                  30
His Met Asn Trp Val Lys Gln Met Pro Gly Lys Gly Leu Glu Trp Met
        35                  40                  45
Gly Asp Ile Asn Pro Asp Ile Gly Gly Thr Ser Tyr Asn Gln Asn Phe
    50                  55                  60
Lys Gly Lys Val Thr Ile Ser Val Asp Lys Ser Ile Ser Thr Ala Tyr
65                  70                  75                  80
Leu Gln Trp Ser Ser Leu Lys Ala Ser Asp Thr Ala Met Tyr Tyr Cys
                85                  90                  95
Ala Arg Trp Asp Phe Asp Ser Phe Ala Asn Trp Gly Gln Gly Thr Leu
            100                 105                 110
Val Thr Val Ser Ser Ala Ser Thr Lys Gly Pro Ser Val Phe Pro Leu
        115                 120                 125
Ala Pro Ser Ser Lys Ser Thr Ser Gly Gly Thr Ala Ala Leu Gly Cys
    130                 135                 140
Leu Val Lys Asp Tyr Phe Pro Glu Pro Val Thr Val Ser Trp Asn Ser
145                 150                 155                 160
Gly Ala Leu Thr Ser Gly Val His Thr Phe Pro Ala Val Leu Gln Ser
                165                 170                 175
Ser Gly Leu Tyr Ser Leu Ser Ser Val Val Thr Val Pro Ser Ser Ser
            180                 185                 190
Leu Gly Thr Gln Thr Tyr Ile Cys Asn Val Asn His Lys Pro Ser Asn
        195                 200                 205
Thr Lys Val Asp Lys Lys Val Glu Pro Lys Ser Cys Asp Lys Thr His
    210                 215                 220
Thr Cys Pro Pro Cys Pro Ala Pro Glu Leu Leu Gly Gly Pro Ser Val
225                 230                 235                 240
Phe Leu Phe Pro Pro Lys Pro Lys Asp Thr Leu Met Ile Ser Arg Thr
                245                 250                 255
Pro Glu Val Thr Cys Val Val Val Asp Val Ser His Glu Asp Pro Glu
            260                 265                 270
Val Lys Phe Asn Trp Tyr Val Asp Gly Val Glu Val His Asn Ala Lys
        275                 280                 285
Thr Lys Pro Arg Glu Glu Gln Tyr Asn Ser Thr Tyr Arg Val Val Ser
    290                 295                 300

Val Leu Thr Val Leu His Gln Asp Trp Leu Asn Gly Lys Glu Tyr Lys
305                 310                 315                 320
Cys Lys Val Ser Asn Lys Ala Leu Pro Ala Pro Ile Glu Lys Thr Ile
                325                 330                 335
Ser Lys Ala Lys Gly Gln Pro Arg Glu Pro Gln Val Tyr Thr Leu Pro
            340                 345                 350
Pro Ser Arg Asp Glu Leu Thr Lys Asn Gln Val Ser Leu Thr Cys Leu
        355                 360                 365
Val Lys Gly Phe Tyr Pro Ser Asp Ile Ala Val Glu Trp Glu Ser Asn
    370                 375                 380
Gly Gln Pro Glu Asn Asn Tyr Lys Thr Thr Pro Pro Val Leu Asp Ser
385                 390                 395                 400
Asp Gly Ser Phe Phe Leu Tyr Ser Lys Leu Thr Val Asp Lys Ser Arg
                405                 410                 415
Trp Gln Gln Gly Asn Val Phe Ser Cys Ser Val Met His Glu Ala Leu
            420                 425                 430
His Asn His Tyr Thr Gln Lys Ser Leu Ser Leu Ser Pro Gly Lys
        435                 440                 445

<210>  85
<211>  218
<212>  PRT
<213>  Artificial Sequence

<220>
<223>  Hu67-14 light chain sequence


<400>  85
Asp Ile Gln Met Thr Gln Ser Pro Ser Ser Leu Ser Ala Ser Val Gly
1               5                   10                  15
Asp Arg Val Thr Ile Thr Cys Arg Ala Ser Glu Ser Val Ser Ile Ile
            20                  25                  30
Gly Thr Asn Leu Ile His Trp Tyr Gln Gln Lys Pro Gly Lys Ala Pro
        35                  40                  45
Lys Leu Leu Ile Tyr His Ala Ser Asn Leu Glu Thr Gly Val Pro Ser
    50                  55                  60
Arg Phe Ser Gly Ser Gly Ser Gly Thr Asp Phe Thr Leu Thr Ile Ser
65                  70                  75                  80
Ser Leu Gln Pro Glu Asp Phe Ala Thr Tyr Tyr Cys Leu Gln Ser Arg
                85                  90                  95
Lys Ile Pro Tyr Thr Phe Gly Gly Gly Thr Lys Val Glu Ile Lys Arg
            100                 105                 110
Thr Val Ala Ala Pro Ser Val Phe Ile Phe Pro Pro Ser Asp Glu Gln
        115                 120                 125
Leu Lys Ser Gly Thr Ala Ser Val Val Cys Leu Leu Asn Asn Phe Tyr
    130                 135                 140
Pro Arg Glu Ala Lys Val Gln Trp Lys Val Asp Asn Ala Leu Gln Ser
145                 150                 155                 160
Gly Asn Ser Gln Glu Ser Val Thr Glu Gln Asp Ser Lys Asp Ser Thr
                165                 170                 175
Tyr Ser Leu Ser Ser Thr Leu Thr Leu Ser Lys Ala Asp Tyr Glu Lys
            180                 185                 190
His Lys Val Tyr Ala Cys Glu Val Thr His Gln Gly Leu Ser Ser Pro
        195                 200                 205
Val Thr Lys Ser Phe Asn Arg Gly Glu Cys
    210                 215

<210>  86
<211>  452
<212>  PRT
<213>  Artificial Sequence

<220>
<223>  Hu103-32 heavy chain sequence


<400>  86
Glu Val Gln Leu Val Gln Ser Gly Ser Glu Leu Lys Lys Pro Gly Ala
1               5                   10                  15
Ser Val Lys Val Ser Cys Lys Ala Ser Gly Tyr Thr Phe Thr Thr Tyr
            20                  25                  30
Gly Val Ile Trp Val Arg Gln Ala Pro Gly Gln Gly Leu Glu Trp Met
        35                  40                  45
Gly Trp Ile Asn Thr Tyr Ser Gly Val Pro Thr Tyr Ala Ser Asp Phe
    50                  55                  60
Lys Gly Arg Phe Val Phe Ser Leu Asp Thr Ser Val Ser Thr Ala Tyr
65                  70                  75                  80
Leu Gln Ile Ser Ser Leu Lys Ala Glu Asp Thr Ala Val Tyr Tyr Cys
                85                  90                  95
Ala Arg Lys Lys Thr Leu Thr Thr Val Thr Pro Trp Phe Ala Tyr Trp
            100                 105                 110
Gly Gln Gly Thr Leu Val Thr Val Ser Ser Ala Ser Thr Lys Gly Pro
        115                 120                 125
Ser Val Phe Pro Leu Ala Pro Ser Ser Lys Ser Thr Ser Gly Gly Thr
    130                 135                 140
Ala Ala Leu Gly Cys Leu Val Lys Asp Tyr Phe Pro Glu Pro Val Thr
145                 150                 155                 160
Val Ser Trp Asn Ser Gly Ala Leu Thr Ser Gly Val His Thr Phe Pro
                165                 170                 175
Ala Val Leu Gln Ser Ser Gly Leu Tyr Ser Leu Ser Ser Val Val Thr
            180                 185                 190
Val Pro Ser Ser Ser Leu Gly Thr Gln Thr Tyr Ile Cys Asn Val Asn
        195                 200                 205
His Lys Pro Ser Asn Thr Lys Val Asp Lys Lys Val Glu Pro Lys Ser
    210                 215                 220
Cys Asp Lys Thr His Thr Cys Pro Pro Cys Pro Ala Pro Glu Leu Leu
225                 230                 235                 240
Gly Gly Pro Ser Val Phe Leu Phe Pro Pro Lys Pro Lys Asp Thr Leu
                245                 250                 255
Met Ile Ser Arg Thr Pro Glu Val Thr Cys Val Val Val Asp Val Ser
            260                 265                 270
His Glu Asp Pro Glu Val Lys Phe Asn Trp Tyr Val Asp Gly Val Glu
        275                 280                 285
Val His Asn Ala Lys Thr Lys Pro Arg Glu Glu Gln Tyr Asn Ser Thr
    290                 295                 300
Tyr Arg Val Val Ser Val Leu Thr Val Leu His Gln Asp Trp Leu Asn
305                 310                 315                 320
Gly Lys Glu Tyr Lys Cys Lys Val Ser Asn Lys Ala Leu Pro Ala Pro
                325                 330                 335
Ile Glu Lys Thr Ile Ser Lys Ala Lys Gly Gln Pro Arg Glu Pro Gln
            340                 345                 350
Val Tyr Thr Leu Pro Pro Ser Arg Asp Glu Leu Thr Lys Asn Gln Val
        355                 360                 365
Ser Leu Thr Cys Leu Val Lys Gly Phe Tyr Pro Ser Asp Ile Ala Val
    370                 375                 380
Glu Trp Glu Ser Asn Gly Gln Pro Glu Asn Asn Tyr Lys Thr Thr Pro
385                 390                 395                 400
Pro Val Leu Asp Ser Asp Gly Ser Phe Phe Leu Tyr Ser Lys Leu Thr
                405                 410                 415
Val Asp Lys Ser Arg Trp Gln Gln Gly Asn Val Phe Ser Cys Ser Val
            420                 425                 430
Met His Glu Ala Leu His Asn His Tyr Thr Gln Lys Ser Leu Ser Leu
        435                 440                 445
Ser Pro Gly Lys
    450

<210> 87
<211> 221
<212> PRT
<213> Artificial Sequence

<220>
<223> Hu103-32 light chain sequence

<400> 87
Glu Leu Val Leu Thr Gln Ser Pro Ser Ala Ser Ala Ser Leu Gly Ala
1               5                   10                  15
Ser Val Lys Leu Thr Cys Thr Leu Ser Ser Gln His Ser Thr Tyr Thr
            20                  25                  30
Ile Glu Trp Tyr Gln Gln Gln Pro Glu Lys Gly Pro Arg Tyr Val Met
        35                  40                  45
Glu Leu Lys Lys Asp Gly Ser His Ser Thr Gly Glu Gly Ile Pro Asp
    50                  55                  60
Arg Phe Ser Gly Ser Ser Ser Gly Ala Glu Arg Tyr Leu Thr Ile Ser
65                  70                  75                  80
Ser Leu Gln Ser Glu Asp Glu Ala Asp Tyr Tyr Cys Gly Val Gly Asn
                85                  90                  95
Thr Ile Lys Glu Gln Phe Val Tyr Val Phe Gly Gly Gly Thr Lys Leu
            100                 105                 110
Thr Val Leu Gly Gln Pro Lys Ala Asn Pro Thr Val Thr Leu Phe Pro
        115                 120                 125
Pro Ser Ser Glu Glu Leu Gln Ala Asn Lys Ala Thr Leu Val Cys Leu
    130                 135                 140
Ile Ser Asp Phe Tyr Pro Gly Ala Val Thr Val Ala Trp Lys Ala Asp
145                 150                 155                 160
Gly Ser Pro Val Lys Ala Gly Val Glu Thr Thr Lys Pro Ser Lys Gln
                165                 170                 175
Ser Asn Asn Lys Tyr Ala Ala Ser Ser Tyr Leu Ser Leu Thr Pro Glu
            180                 185                 190
Gln Trp Lys Ser His Arg Ser Tyr Ser Cys Gln Val Thr His Glu Gly
        195                 200                 205
Ser Thr Val Glu Lys Thr Val Ala Pro Thr Glu Cys Ser
    210                 215                 220

<210> 88
<211> 450
<212> PRT
<213> Artificial Sequence

<220>
<223> Sanofi antibody heavy chain sequence

<400> 88
Glu Val Gln Leu Gln Glu Ser Gly Pro Gly Leu Val Lys Pro Gly Gly
1               5                   10                  15
Ser Leu Ser Leu Ser Cys Ala Ala Ser Gly Phe Val Phe Ser Ser Tyr
            20                  25                  30
Asp Met Ser Trp Val Arg Gln Thr Pro Glu Arg Gly Leu Glu Trp Val
        35                  40                  45
Ala Tyr Ile Ser Ser Gly Gly Gly Ile Thr Tyr Ala Pro Ser Thr Val
    50                  55                  60
Lys Gly Arg Phe Thr Val Ser Arg Asp Asn Ala Lys Asn Thr Leu Tyr
65                  70                  75                  80
Leu Gln Met Asn Ser Leu Thr Ser Glu Asp Thr Ala Val Tyr Tyr Cys
                85                  90                  95
Ala Ala His Tyr Phe Gly Ser Ser Gly Pro Phe Ala Tyr Trp Gly Gln

                100                 105                 110
Gly Thr Leu Val Thr Val Ser Ser Ala Ser Thr Lys Gly Pro Ser Val
        115                 120                 125
Phe Pro Leu Ala Pro Ser Ser Lys Ser Thr Ser Gly Gly Thr Ala Ala
    130                 135                 140
Leu Gly Cys Leu Val Lys Asp Tyr Phe Pro Glu Pro Val Thr Val Ser
145                 150                 155                 160
Trp Asn Ser Gly Ala Leu Thr Ser Gly Val His Thr Phe Pro Ala Val
                165                 170                 175
Leu Gln Ser Ser Gly Leu Tyr Ser Leu Ser Ser Val Val Thr Val Pro
            180                 185                 190
Ser Ser Ser Leu Gly Thr Gln Thr Tyr Ile Cys Asn Val Asn His Lys
        195                 200                 205
Pro Ser Asn Thr Lys Val Asp Lys Lys Val Glu Pro Lys Ser Cys Asp
    210                 215                 220
Lys Thr His Thr Cys Pro Pro Cys Pro Ala Pro Glu Leu Leu Gly Gly
225                 230                 235                 240
Pro Ser Val Phe Leu Phe Pro Pro Lys Pro Lys Asp Thr Leu Met Ile
                245                 250                 255
Ser Arg Thr Pro Glu Val Thr Cys Val Val Val Asp Val Ser His Glu
            260                 265                 270
Asp Pro Glu Val Lys Phe Asn Trp Tyr Val Asp Gly Val Glu Val His
        275                 280                 285
Asn Ala Lys Thr Lys Pro Arg Glu Glu Gln Tyr Asn Ser Thr Tyr Arg
    290                 295                 300
Val Val Ser Val Leu Thr Val Leu His Gln Asp Trp Leu Asn Gly Lys
305                 310                 315                 320
Glu Tyr Lys Cys Lys Val Ser Asn Lys Ala Leu Pro Ala Pro Ile Glu
                325                 330                 335
Lys Thr Ile Ser Lys Ala Lys Gly Gln Pro Arg Glu Pro Gln Val Tyr
            340                 345                 350
Thr Leu Pro Pro Ser Arg Asp Glu Leu Thr Lys Asn Gln Val Ser Leu
        355                 360                 365
Thr Cys Leu Val Lys Gly Phe Tyr Pro Ser Asp Ile Ala Val Glu Trp
    370                 375                 380
Glu Ser Asn Gly Gln Pro Glu Asn Asn Tyr Lys Thr Thr Pro Pro Val
385                 390                 395                 400
Leu Asp Ser Asp Gly Ser Phe Phe Leu Tyr Ser Lys Leu Thr Val Asp
                405                 410                 415
Lys Ser Arg Trp Gln Gln Gly Asn Val Phe Ser Cys Ser Val Met His
            420                 425                 430
Glu Ala Leu His Asn His Tyr Thr Gln Lys Ser Leu Ser Leu Ser Pro
        435                 440                 445
Gly Lys
    450

<210>  89
<211>  214
<212>  PRT
<213>  Artificial Sequence

<220>
<223>  Sanofi antibody light chain sequence


<400>  89
Asp Ile Gln Met Thr Gln Ser Pro Ala Ser Leu Ser Ala Ser Val Gly
1               5                   10                  15
Asp Arg Val Thr Ile Thr Cys Arg Ala Ser Glu Asn Ile Phe Ser Tyr
            20                  25                  30
Leu Ala Trp Tyr Gln Gln Lys Pro Gly Lys Ser Pro Lys Leu Leu Val
        35                  40                  45
Tyr Asn Thr Arg Thr Leu Ala Glu Gly Val Pro Ser Arg Phe Ser Gly

50                  55                  60
Ser Gly Ser Gly Thr Asp Phe Ser Leu Thr Ile Ser Ser Leu Gln Pro
65                  70                  75                  80
Glu Asp Phe Ala Thr Tyr Tyr Cys Gln His His Tyr Gly Thr Pro Phe
                85                  90                  95
Thr Phe Gly Ser Gly Thr Lys Leu Glu Ile Lys Arg Thr Val Ala Ala
            100                 105                 110
Pro Ser Val Phe Ile Phe Pro Pro Ser Asp Glu Gln Leu Lys Ser Gly
        115                 120                 125
Thr Ala Ser Val Val Cys Leu Leu Asn Asn Phe Tyr Pro Arg Glu Ala
    130                 135                 140
Lys Val Gln Trp Lys Val Asp Asn Ala Leu Gln Ser Gly Asn Ser Gln
145                 150                 155                 160
Glu Ser Val Thr Glu Gln Asp Ser Lys Asp Ser Thr Tyr Ser Leu Ser
                165                 170                 175
Ser Thr Leu Thr Leu Ser Lys Ala Asp Tyr Glu Lys His Lys Val Tyr
            180                 185                 190
Ala Cys Glu Val Thr His Gln Gly Leu Ser Ser Pro Val Thr Lys Ser
        195                 200                 205
Phe Asn Arg Gly Glu Cys
    210

<210>  90
<211>  449
<212>  PRT
<213>  Artificial Sequence

<220>
<223>  Lmab antibody heavy chain sequence


<400>  90
Glu Val Gln Leu Val Glu Ser Gly Gly Gly Val Val Gln Pro Gly Arg
1               5                   10                  15
Ser Leu Arg Leu Ser Cys Ser Ala Ser Gly Phe Asp Phe Thr Thr Tyr
            20                  25                  30
Trp Met Ser Trp Val Arg Gln Ala Pro Gly Lys Gly Leu Glu Trp Ile
        35                  40                  45
Gly Glu Ile His Pro Asp Ser Ser Thr Ile Asn Tyr Ala Pro Ser Leu
    50                  55                  60
Lys Asp Arg Phe Thr Ile Ser Arg Asp Asn Ala Lys Asn Thr Leu Phe
65                  70                  75                  80
Leu Gln Met Asp Ser Leu Arg Pro Glu Asp Thr Gly Val Tyr Phe Cys
                85                  90                  95
Ala Ser Leu Tyr Phe Gly Phe Pro Trp Phe Ala Tyr Trp Gly Gln Gly
            100                 105                 110
Thr Pro Val Thr Val Ser Ser Ala Ser Thr Lys Gly Pro Ser Val Phe
        115                 120                 125
Pro Leu Ala Pro Ser Ser Lys Ser Thr Ser Gly Gly Thr Ala Ala Leu
    130                 135                 140
Gly Cys Leu Val Lys Asp Tyr Phe Pro Glu Pro Val Thr Val Ser Trp
145                 150                 155                 160
Asn Ser Gly Ala Leu Thr Ser Gly Val His Thr Phe Pro Ala Val Leu
                165                 170                 175
Gln Ser Ser Gly Leu Tyr Ser Leu Ser Ser Val Val Thr Val Pro Ser
            180                 185                 190
Ser Ser Leu Gly Thr Gln Thr Tyr Ile Cys Asn Val Asn His Lys Pro
        195                 200                 205
Ser Asn Thr Lys Val Asp Lys Arg Val Glu Pro Lys Ser Cys Asp Lys
    210                 215                 220
Thr His Thr Cys Pro Pro Cys Pro Ala Pro Glu Leu Leu Gly Gly Pro
225                 230                 235                 240
Ser Val Phe Leu Phe Pro Pro Lys Pro Lys Asp Thr Leu Met Ile Ser

                245                 250                 255
Arg Thr Pro Glu Val Thr Cys Val Val Val Asp Val Ser His Glu Asp
            260                 265                 270
Pro Glu Val Lys Phe Asn Trp Tyr Val Asp Gly Val Glu Val His Asn
        275                 280                 285
Ala Lys Thr Lys Pro Arg Glu Glu Gln Tyr Asn Ser Thr Tyr Arg Val
    290                 295                 300
Val Ser Val Leu Thr Val Leu His Gln Asp Trp Leu Asn Gly Lys Glu
305                 310                 315                 320
Tyr Lys Cys Lys Val Ser Asn Lys Ala Leu Pro Ala Pro Ile Glu Lys
                325                 330                 335
Thr Ile Ser Lys Ala Lys Gly Gln Pro Arg Glu Pro Gln Val Tyr Thr
            340                 345                 350
Leu Pro Pro Ser Arg Glu Glu Met Thr Lys Asn Gln Val Ser Leu Thr
        355                 360                 365
Cys Leu Val Lys Gly Phe Tyr Pro Ser Asp Ile Ala Val Glu Trp Glu
    370                 375                 380
Ser Asn Gly Gln Pro Glu Asn Asn Tyr Lys Thr Thr Pro Pro Val Leu
385                 390                 395                 400
Asp Ser Asp Gly Ser Phe Phe Leu Tyr Ser Lys Leu Thr Val Asp Lys
                405                 410                 415
Ser Arg Trp Gln Gln Gly Asn Val Phe Ser Cys Ser Val Met His Glu
            420                 425                 430
Ala Leu His Asn His Tyr Thr Gln Lys Ser Leu Ser Leu Ser Pro Gly
        435                 440                 445
Lys

<210>  91
<211>  213
<212>  PRT
<213>  Artificial Sequence

<220>
<223>  Lmab antibody light chain sequence

<400>  91
Asp Ile Gln Leu Thr Gln Ser Pro Ser Ser Leu Ser Ala Ser Val Gly
1               5                   10                  15
Asp Arg Val Thr Ile Thr Cys Lys Ala Ser Gln Asp Val Gly Thr Ser
            20                  25                  30
Val Ala Trp Tyr Gln Gln Lys Pro Gly Lys Ala Pro Lys Leu Leu Ile
        35                  40                  45
Tyr Trp Thr Ser Thr Arg His Thr Gly Val Pro Ser Arg Phe Ser Gly
    50                  55                  60
Ser Gly Ser Gly Thr Asp Phe Thr Phe Thr Ile Ser Ser Leu Gln Pro
65                  70                  75                  80
Glu Asp Ile Ala Thr Tyr Tyr Cys Gln Gln Tyr Ser Leu Tyr Arg Ser
                85                  90                  95
Phe Gly Gln Gly Thr Lys Val Glu Ile Lys Arg Thr Val Ala Ala Pro
            100                 105                 110
Ser Val Phe Ile Phe Pro Pro Ser Asp Glu Gln Leu Lys Ser Gly Thr
        115                 120                 125
Ala Ser Val Val Cys Leu Leu Asn Asn Phe Tyr Pro Arg Glu Ala Lys
    130                 135                 140
Val Gln Trp Lys Val Asp Asn Ala Leu Gln Ser Gly Asn Ser Gln Glu
145                 150                 155                 160
Ser Val Thr Glu Gln Asp Ser Lys Asp Ser Thr Tyr Ser Leu Ser Ser
                165                 170                 175
Thr Leu Thr Leu Ser Lys Ala Asp Tyr Glu Lys His Lys Val Tyr Ala
            180                 185                 190
Cys Glu Val Thr His Gln Gly Leu Ser Ser Pro Val Thr Lys Ser Phe

        195                 200                 205
Asn Arg Gly Glu Cys
    210